# EUROPEAN PATENT SPECIFICATION

(11) **EP 0 632 803 B1**
(45) Date of publication and mention of the grant of the patent: **09.12.1998**
(21) Application number: 93907555.2
(22) Date of filing: 18.03.1993
(51) Int. Cl.: C07D 207/20, C07D 253/10, C07D 235/02, C07D 273/04, A01N 43/36, A01N 43/707, A01N 43/52, A01N 43/88

(54) **ARTHROPODICIDAL AMIDES**
ATHROPODIZIDE AMIDE
AMIDES ARTHROPODICIDES

(30) Priority: 26.03.1992 US 858205; 28.04.1992 US 875174
(43) Date of publication of application: 11.01.1995
(73) Proprietor: E.I. DU PONT DE NEMOURS AND COMPANY, Wilmington Delaware 19898 (US)
(72) Inventor: AMOO, Victor, Ekow, Newark, DE 19711 (US); ANNIS, Gary, David, Landenberg, PA 19350 (US); MARCH, Robert, William, Jr., Newark, DE 19711 (US)
(74) Representative: Woodman, Derek
(86) International application number: US9302434
(87) International publication number: WO9319045

(56) References cited:
- EP-A- 0 286 346
- WO-A-90/03369
- WO-A-91/17983
- WO-A-92/11249
- WO-A-92/20682

## Description

U.S. 4,070,365 discloses insecticidally active amides that do not, however, suggest the particular compounds of this invention.

### SUMMARY OF THE INVENTION

This invention pertains to amides of Formula I, including all geometric and stereoisomers, suitable salts thereof, compositions containing them and use of such compounds to control arthropods in both agronomic and nonagronomic environments. The term "compounds" will be understood to include all such isomers and salts thereof. The compounds are: 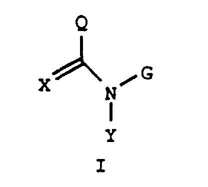 wherein
- Q: is selected from the group 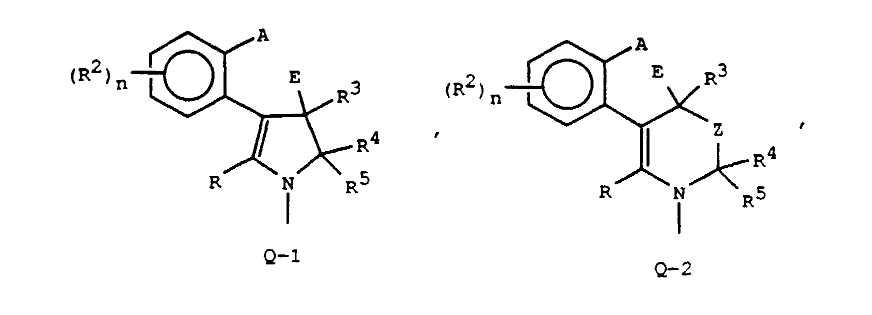 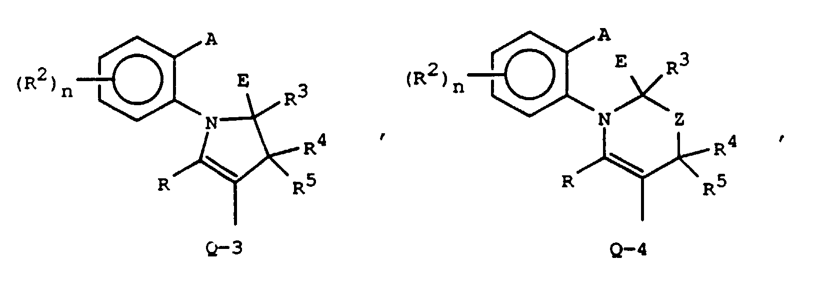 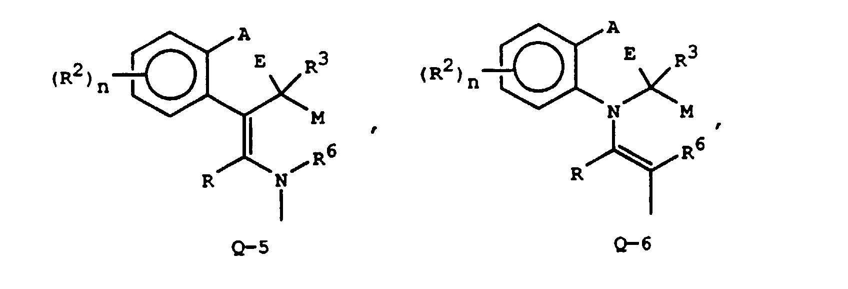 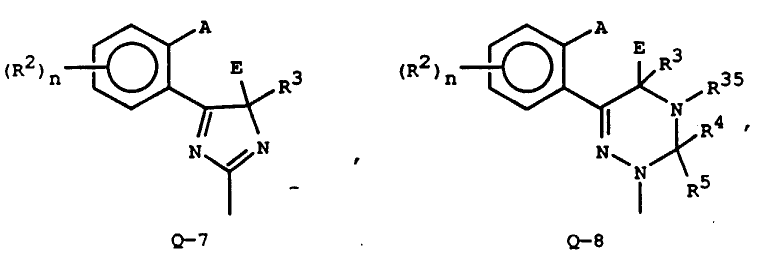 and 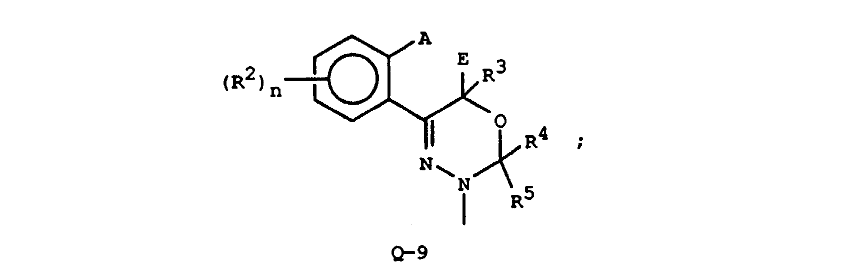
- A: is H;
- E: is selected from the group H and C₁-C₃ alkyl; E being other than H when Q is Q-7; or
- A and E: are taken together to form -CH₂-,-CH₂CH₂-, -O-, -S-, -S(O)-, -S(O)₂-, -NR⁷-, -OCH₂-, -SCH₂-, -N(R⁷)CH₂-, substituted -CH₂-, and substituted -CH₂CH₂-, the substituents independently selected from 1-2 halogen and 1-2 methyl;
- M: is selected from the group H and C₁-C₃ alkyl; or
- E and M: are taken together as -CH₂CH₂-, -CH₂CH₂CH₂- or -CH₂CH₂CH₂CH₂-, each group optionally substituted with one or more members independently selected from the group halogen, NO₂, CN, C₁-C₃ alkyl, C₁-C₃ haloalkyl, OH, OR⁶ and C(O)₂R¹⁹;
- G: is selected from the group 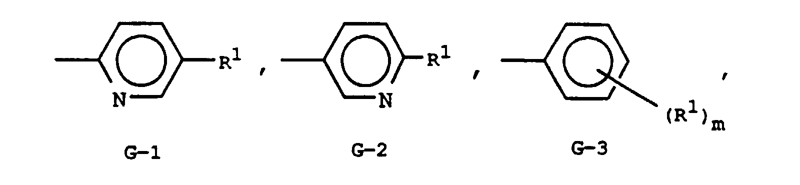 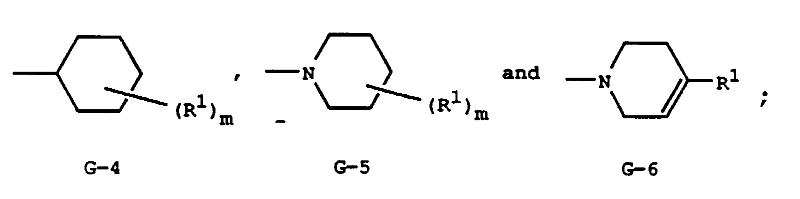
- X: is selected from the group O and S;
- Y: is selected from the group H; C₁-C₆ alkyl; benzyl; C₂-C₆ alkenyl; C₂-C₆ alkynyl; C₁-C₆ alkyl substituted by halogen, C₁-C₃ alkoxy, C₁-C₃ haloalkoxy, CN, NO₂, S(O)ᵣR³⁰, P(X)(OR²⁵)₂, C(O)R³⁰, C(O)₂R³⁰ and phenyl optionally substituted by halogen, CN, C₁-C₂ haloalkyl and C₁-C₂ haloalkoxy; C₃-C₆ cycloalkyl; C₃-C₆ halocycloalkyl; C₄-C₆ cycloalkylalkyl; CHO; C₂-C₆ alkylcarbonyl; C₂-C₆ alkoxycarbonyl; C₂-C₆ haloalkylcarbonyl; C(O)R³³; C(O)₂R³³; C(S)R²⁶; C(S)R³³; C(O)C(O)₂R²⁵; C(O)CH₂C(O)₂R²⁵; S(O)ᵣR³⁰; S(O)₂CH₂C(O)₂R²⁵; P(X) (OR²⁵)₂; phenylthio; R¹¹OC(O)N(R¹²)S-; R¹³(R¹⁴)NS-; N=CR⁹R¹⁰; OR⁸; NR⁸R⁹; and R³⁸; Y being R³⁸ when Q is Q-9; Y being other than N-CR⁹R¹⁰, OR⁸, and NR⁸R⁹ when Q is Q-8 and A and E are taken together as -CH₂-;
- Z: is selected from the group CH₂, O, S and NR²⁹;
- R: is selected from the group H, halogen, C₁-C₃ alkyl, C₁-C₃ alkoxy, CN, and NO₂;
- R¹ and R²: are independently selected from the group H, C₁-C₆ alkyl, C₁-C₆ haloalkyl, C₂-C₆ alkenyl, C₂-C₆ haloalkenyl, C₂-C₆ alkynyl, C₃-C₆ haloalkynyl, C₂-C₆ alkoxyalkyl, C₂-C₆ alkylthioalkyl, C₁-C₆ nitroalkyl, C₂-C₆ cyanoalkyl, C₃-C₈ alkoxycarbonylalkyl, C₃-C₆ cycloalkyl, C₃-C₆ halocycloalkyl, halogen, CN, N₃, SCN, NO₂, OR¹⁶, SR¹⁶, S(O)R¹⁶, S(O)₂R¹⁶, OC(O)R¹⁶, OS(O)₂R¹⁶, C(O)R¹⁶, C(O)₂R¹⁶, C(O)NR¹⁶R¹⁷, S(O)₂NR¹⁶R¹⁷, NR¹⁶R¹⁷, NR¹⁷C(O)R¹⁶, OC(O)NHR¹⁶, NR¹⁷C(O)NHR¹⁶, NR¹⁷S(O)₂R¹⁶, phenyl optionally substituted with 1 to 3 substituents independently selected from W, and benzyl optionally substituted with 1 to 3 substituents independently selected from W; or when m or n is 2, (R¹)₂ are taken together, or (R²)₂ are taken together as -OCH₂O-, -OCF₂O-, -OCH₂CH₂O-, -CH₂C(CH₃)₂O-, -CF₂CF₂O- or -OCF₂CF₂O- to form a cyclic bridge; provided that when R¹ or R² is S(O)R¹⁶, S(O)₂R¹⁶, OC(O)R¹⁶, NR¹⁷S(O)₂R¹⁶ or OS(O)₂R¹⁶ then R¹⁶ is other than H;
- R³: is selected from the group H, J, N₃, NO₂, halogen, N(R²¹)R²², C(R³¹)=N-O-R³², C₁-C₆ alkyl, C₁-C₆ haloalkyl, C₂-C₆ alkenyl, C₂-C₆ haloalkenyl, C₂-C₆ alkynyl, C₂-C₆ alkoxyalkyl, C₃-C₈ alkoxycarbonylalkyl, C(O)R¹⁶, C(O)₂R¹⁶, OR¹⁸, C(O)NR¹⁶R¹⁷, C(S)NR¹⁶R¹⁷, C(S)R¹⁶, C(S)SR¹⁶, CN, Si(R²⁵)(R²⁶)(R²⁷), SR²⁵, S(O)R²⁵, S(O)₂R²⁵, P(O)(OR²⁵)₂, phenyl optionally substituted with (R¹⁵)ₚ, and benzyl optionally substituted with 1 to 3 substituents independently selected from W; or R³ is C₂-C₆ epoxyalkyl optionally substituted with one or more members independently selected from the group C₁-C₃ alkyl, CN, C(O)R²³, C(O)₂R²³, and phenyl optionally substituted with W; or R³ is C₁-C₆ alkyl substituted with one or more members independently selected from the group C(O)N(R²⁴)R³⁴, C(O)R²⁴, SR²⁵, S(O)R²⁵, S(O)₂R²⁵, SCN, CN, C₁-C₂ haloalkoxy, Si(R²⁵)(R²⁶)(R²⁷), N(R²¹)R²², NO₂, OC(O)R²⁴, P(O)(OR²⁵)₂, and J; R³ being other than H when Q is Q-7;
- J: is selected from the group saturated, partially unsaturated or aromatic 5- or 6-membered heterocyclic ring, bonded through carbon or nitrogen, containing 1-4 heteroatoms independently selected from the group consisting of 0-2 oxygen, 0-2 sulfur and 0-4 nitrogen, this substituent optionally containing one carbonyl and optionally substituted with one or more members independently selected from W;
- R⁴ and R⁵: are independently selected from the group H, C₁-C₄ alkyl, C(O)R¹⁹ and C₂-C₄ alkoxycarbonyl;
- R⁶: is selected from the group H, C₁-C₄ alkyl, C(O)R¹⁹ and C(O)₂R¹⁹;
- R⁷: is selected from the group H, C₁-C₄ alkyl, C₁-C₄ haloalkyl, C₂-C₄ alkenyl, C₂-C₄ haloalkenyl, SR¹⁶, S(O)R¹⁶, S(O)₂R¹⁶, C(O)R¹⁶, C(O)₂R¹⁶, C(O)NR¹⁶R²⁰, C(S)NR¹⁶R²⁰, C(S)R¹⁶, C(S)OR¹⁶, P(O)(OR¹⁶)₂, P(S)(OR¹⁶)₂, P(O)(R¹⁶)OR¹⁶, P(O)(R¹⁶)SR²⁰, optionally substituted phenyl, and optionally substituted benzyl wherein the optional phenyl and benzyl substituents are independently selected from F, Cl, Br, CH₃, CF₃ and OCF₃; provided that when R⁷ is other than C(O)R¹⁶, C(O)NR¹⁶R²⁰ or C(S)NR¹⁶R²⁰ then R¹⁶ is other than H;
- R⁸: is selected from the group H, C₁-C₄ alkyl, C₁-C₄ haloalkyl, C₂-C₄ alkenyl, C₂-C₄ alkynyl, S(O)₂NR¹⁷R¹⁸, S(O)₂R¹⁶, C(O)R¹⁶, C(O)₂R¹⁶, C(O)NR¹⁶R²⁰, phenyl optionally substituted with halogen or C₁-C₄ alkoxy, and benzyl optionally substituted with halogen; provided that when R⁸ is S(O)₂R¹⁶, R¹⁶ is other than H;
- R⁹: is selected from the group H, C₁-C₄ alkyl and C(O)R¹⁶;
- R¹⁰: is selected from the group H, C₁-C₄ alkyl, C₁-C₄ haloalkyl and phenyl optionally substituted with one or more members independently selected from halogen, CN, NO₂, CF₃ and OCF₃; or
- R⁹ and R¹⁰: are taken together as -CH₂CH₂CH₂-, -CH₂CH₂CH₂CH₂- or -CH₂CH₂CH₂CH₂CH₂-;
- R¹¹: is C₁-C₁₈ alkyl;
- R¹²: is C₁-C₄ alkyl;
- R¹³ and R¹⁴: are independently C₁-C₄ alkyl; or
- R¹³ and R¹⁴: are taken together as -CH₂CH₂CH₂CH₂CH₂- or -CH₂CH₂OCH₂CH₂-;
- R¹⁵: is selected from the group C₁-C₆ alkyl, C₁-C₆ haloalkyl, C₂-C₆ alkenyl, C₂-C₆ haloalkenyl, C₂-C₆ alkynyl, C₃-C₆ haloalkynyl, C₂-C₆ alkoxyalkyl, C₂-C₆ alkylthioalkyl, C₁-C₆ nitroalkyl, C₂-C₆ cyanoalkyl, C₃-C₈ alkoxycarbonylalkyl, C₃-C₆ cycloalkyl, C₃-C₆ halocycloalkyl, halogen, CN, N₃, SCN, NO₂, OR¹⁶, SR¹⁶, S(O)R¹⁶, S(O)₂R¹⁶, OC(O)R¹⁶, OS(O)₂R¹⁶, C(O)R¹⁶, C(O)₂R¹⁶, C(O)NR¹⁶R¹⁷, S(O)₂NR¹⁶R¹⁷, NR¹⁶R¹⁷, NR¹⁷C(O)R¹⁶, OC(O)NHR¹⁶, NR¹⁷C(O)NHR¹⁶, NR¹⁷S(O)₂R¹⁶, phenyl optionally substituted with 1 to 3 substituents independently selected from W, and benzyl optionally substituted with 1 to 3 substituents independently selected from W; or when p is 2, (R¹⁵)₂ are taken together as -OCH₂O-, -OCF₂O-, -OCH₂CH₂O-, -CH₂C(CH₃)₂O-, -CF₂CF₂O- or -OCF₂CF₂O- to form a cyclic bridge; provided that when R¹⁵ is S(O)R¹⁶, S(O)₂R¹⁶, OC(O)R¹⁶, NR¹⁷S(O)₂R¹⁶ or OS(O)₂R¹⁶ then R¹⁶ is other than H;
- R¹⁶: is selected from the group H, C₁-C₆ alkyl, C₁-C₆ haloalkyl, C₂-C₆ alkenyl, C₂-C₆ haloalkenyl, C₂-C₆ alkynyl, C₃-C₆ haloalkynyl, C₂-C₆ alkoxyalkyl, C₂-C₆ alkylthioalkyl, C₁-C₆ nitroalkyl, C₂-C₆ cyanoalkyl, C₃-C₈ alkoxycarbonylalkyl, C₃-C₆ cycloalkyl, C₃-C₆ halocycloalkyl, optionally substituted phenyl and optionally substituted benzyl wherein the optional phenyl and benzyl substituents are 1 to 3 substituents independently selected from W;
- R¹⁷: is selected from the group H and C₁-C₄ alkyl; or
- R¹⁶ and R¹⁷,: when attached to the same atom, are taken together as -(CH₂)₄-, -(CH₂)₅-, or -CH₂CH₂OCH₂CH₂-;
- R¹⁸: is selected from the group H, C₁-C₄ alkyl, C₂-C₄ alkenyl, C₂-C₄ alkynyl, C₂-C₄ alkylcarbonyl, C₂-C₄ alkoxycarbonyl and C₁-C₄ alkylsulfonyl;
- R¹⁹: is C₁-C₃ alkyl;
- R²⁰: is selected from the group H, C₁-C₄ alkyl, C₂-C₄ alkenyl and C₂-C₄ alkynyl;
- R²¹: is selected from the group H, C₂-C₇ alkylcarbonyl, C₂-C₇ alkoxycarbonyl, optionally substituted C₁-C₄ alkyl, optionally substituted C₂-C₄ alkenyl, and optionally substituted C₂-C₄ alkynyl, all of these optional substituents being independently selected from C₁-C₂ alkoxy, CN, C(O)R²⁸ and C(O)₂R²⁵;
- R²²: is selected from the group H, C₁-C₃ alkyl, phenyl optionally substituted with at least one member independently selected from W, and benzyl optionally substituted with at least one member independently selected from W;
- R²³: is selected from the group H, C₁-C₄ alkyl, C₂-C₄ alkenyl and C₂-C₄ alkynyl;
- R²⁴: is selected from the group H and C₁-C₃ alkyl;
- R²⁵: is selected from the group C₁-C₃ alkyl and phenyl optionally substituted with at least one member independently selected from W;
- R²⁶: is selected from the group C₁-C₄ alkyl, C₁-C₄ haloalkyl, C₁-C₄ alkoxy, and C₁-C₄ haloalkoxy;
- R²⁷: is C₁-C₃ alkyl;
- R²⁸: is selected from the group H, C₁-C₃ alkyl and phenyl optionally substituted with at least one member independently selected from W;
- R²⁹: is selected from the group H, CHO, C₁-C₄ alkyl, C₂-C₄ alkylcarbonyl and C₂-C₄ alkoxycarbonyl;
- R³⁰: is selected from the group C₁-C₃ alkyl and C₁-C₃ haloalkyl;
- R³¹: is selected from the group H, Cl, C₁-C₄ alkyl, C₁-C₄ alkoxy, C₁-C₂ alkylthio and CN;
- R³²: is selected from the group H, C₁-C₄ alkyl, C₂-C₃ alkylcarbonyl and C₂-C₃ alkoxycarbonyl;
- R³³: is selected from the group C₁-C₄ alkylthio, C₁-C₄ haloalkylthio, and phenyl optionally substituted with at least one member independently selected from W;
- R³⁴: is selected from the group H and C₁-C₂ alkyl;
- R³⁵: is selected from the group CHO; C₁-C₄ alkyl substituted with substituents independently selected from halogen, C₁-C₂ alkoxy, C₁-C₂ haloalkoxy, CN, NO₂, C₂-C₄ alkylcarbonyl, C₂-C₄ alkoxycarbonyl and N(R³⁶)(R³⁷); or R³⁵ is C₂-C₆ haloalkylcarbonyl; C₃-C₆ alkenyl; C₃-C₆ haloalkenyl; C₃-C₆ alkynyl; C₃-C₆ haloalkynyl; C₃-C₆ cycloalkyl; C(S)R²⁶; C(S)R³³; C(O)C(O)₂R²⁵; C(O)CH₂C(O)₂R²⁵; S(O)ᵣR³⁰; S(O)₂CH₂C(O)₂R²⁵; P(X)(OR²⁵)₂; C(O)N(R³⁶)(R³⁷); S(O)ᵣN(R¹³)R¹⁴; S(O)ᵣN(R¹²)C(O)OR¹¹; S(O)ᵣN(R¹²)CHO; J; CH₂J; C(O)J; C(O)Ph where the phenyl group is optionally substituted by a group independently selected from W; and benzyl optionally substituted by a group independently selected from W;
- R³⁶: is selected from the group H, C₁-C₄ alkyl, C₁-C₄ alkenyl, phenyl optionally substituted by a group independently selected from W, and benzyl optionally substituted by a group independently selected from W;
- R³⁷: is selected from the group C₁-C₄ alkyl and C₁-C₄ alkenyl;
- R³⁸: is selected from the group C(S)R²⁶, C(S)R³³, C(O)C(O)₂R²⁵, C(O)CH₂C(O)₂R²⁵, S(O)R³⁰, S(O)₂R³⁰, S(O)₂CH₂C(O)₂R²⁵, P(X)(OR²⁵)₂, C₃-C₆ haloalkynyl, and C₁-C₆ alkyl substituted by P(X)(OR²⁵)₂;
- W: is selected from the group halogen, CN, NO₂, C₁-C₂ alkyl, C₁-C₂ haloalkyl, C₁-C₂ alkoxy, C₁-C₂ haloalkoxy, C₁-C₂ alkylthio, C₁-C₂ haloalkylthio, C₁-C₂ alkylsulfonyl, and C₁-C₂ haloalkylsulfonyl;
- m: is 1 to 3;
- n: is 1 to 3;
- p: is 1 to 3; and
- r: is 0, 1 or 2.
Exemplary values of J include:

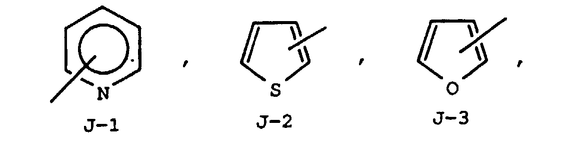

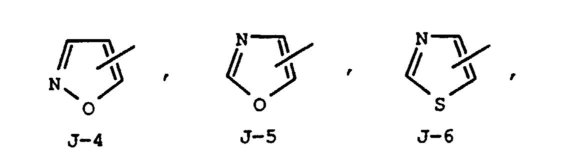

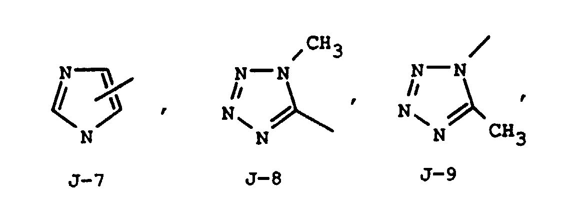

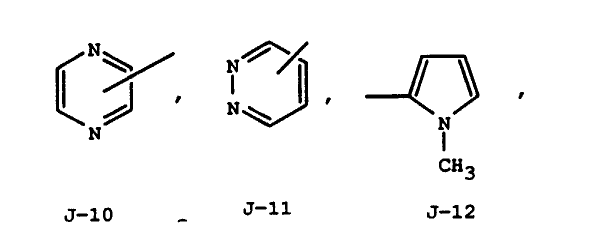

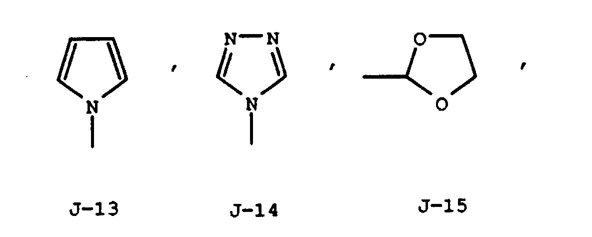

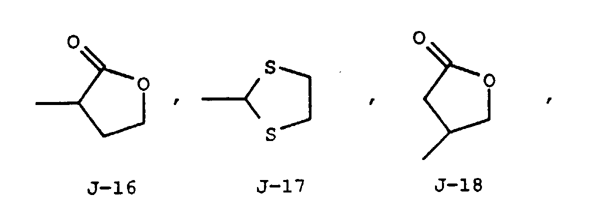

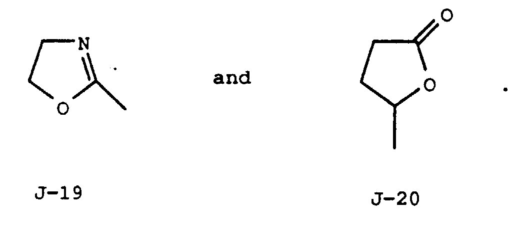

Preferred for reasons including ease of synthesis and/or greater arthropodicidal efficacy are the following compounds, A through L:
Compounds A are those wherein:
- R¹: is selected from the group H, C₁-C₆ alkyl, C₁-C₆ haloalkyl, C₂-C₆ alkenyl, C₂-C₆ haloalkenyl, C₂-C₆ alkynyl, C₃-C₆ haloalkynyl, C₂-C₆ alkoxyalkyl, C₂-C₆ alkylthioalkyl, C₁-C₆ nitroalkyl, C₂-C₆ cyanoalkyl, C₃-C₈ alkoxycarbonylalkyl, C₃-C₆ cycloalkyl, C₃-C₆ halocycloalkyl, halogen, CN, SCN, NO₂, OR¹⁶, SR¹⁶, S(O)₂R¹⁶, O(O)R¹⁶, C(O)₂R¹⁶, phenyl optionally substituted with 1 to 3 substituents independently selected from W, and benzyl optionally substituted with 1 to 3 substituents independently selected from W; with one R¹ substituent in the 4-position, or when m is 2 then (R¹)₂ are taken together as -CH₂C(CH₃)₂O-, -OCH₂CH₂O-, -OCF₂CF₂O-, or -CF₂CF₂O- to form a 5- or 6-membered fused ring;
- R²: is selected from the group H, C₁-C₆ alkyl, C₁-C₆ haloalkyl, C₂-C₆ alkenyl, C₂-C₆ haloalkenyl, halogen, CN, SCN, NO₂, OR¹⁶, SR¹⁶, S(O)R¹⁶, OC(O)₂R¹⁶, OS(O)₂R¹⁶, C(O)R¹⁶, C(O)₂R¹⁶, C(O)NR¹⁶R¹⁷, S(O)₂NR¹⁶R¹⁷, NR¹⁶R¹⁷;
- R³: is selected from the group H, C₁-C₄ alkyl, C(O)R¹⁶, C(O)₂R¹⁶, and phenyl independently substituted by one or more substituents selected from (R¹⁵)ₚ;
- R¹⁵: is selected from the group C₁-C₆ alkyl, C₁-C₆ haloalkyl, C₂-C₆ alkenyl, C₂-C₆ haloalkenyl, halogen, CN, SCN, NO₂, OR¹⁶, SR¹⁶, S(O)₂R¹⁶, OC(O)₂R¹⁶, OS(O)R¹⁶, C(O)R¹⁶, C(O)₂R¹⁶, C(O)NR¹⁶R¹⁷, S(O)₂NR¹⁶R¹⁷, NR¹⁶R¹⁷, phenyl optionally substituted with 1 to 3 substituents independently selected from W, and benzyl optionally substituted with 1 to 3 substituents independently selected from W;
- R¹⁶: is selected from the group C₁-C₄ alkyl, C₁-C₂ haloalkyl, C₃-C₄ alkenyl and propargyl;
- R¹⁷: is selected from the group H and CH₃;
- R³⁵: is selected from CHO, C₁-C₄ alkyl substituted with substituents independently selected from the group halogen, C₁-C₂ alkoxy, C₁-C₂ haloalkoxy, CN, NO₂, C₂-C₄ alkylcarbonyl, C₂-C₄ alkoxycarbonyl and N(R³⁵)(R³⁶); or R³⁵ is C₂-C₆ haloalkylcarbonyl; C₃-C₆ alkenyl; C₃-C₆ haloalkenyl; C₃-C₆ alkynyl; C(O)N(R³⁵)(R³⁶); R¹¹OC(O)N(R¹²)S-; R¹³(R¹⁴)NS-; C(O)Ph where the phenyl group is optionally substituted by a group independently selected from W; and benzyl optionally substituted by a group independently selected from W;
- R³⁸: is selected from the group C(S)R²⁶, C(S)R³³, C(O)C(O)₂R²⁵, C(O)CH₂C(O)₂R²⁵, S(O)R³⁰, S(O)₂R³⁰, S(O)₂CH₂C(O)₂R²⁵, P(X)(OR²⁵)₂, C₃-C₆ haloalkynyl, and C₁-C₆ alkyl substituted by P(X) (OR²⁵)₂; and
- m: is 1 or 2.

Compounds B are those of Preferred A wherein G is G-3, R⁴ is H and R⁵ is H.

Compounds C are of Preferred B wherein Q is Q-1.

Compounds D are of Preferred B wherein Q is Q-2.

Compounds E are of Preferred B wherein Q is Q-7.

Compounds F are of Preferred B wherein Q is Q-8.

Compounds G are of Preferred B wherein Q is Q-9.

Specifically preferred are those compounds of Preferred B:
- (H): 3,4-bis(4-chlorophenyl)-4,5-dihydro-N-[4-(trifluoromethyl)phenyl]-1H-pyrrole-1-carboxamide,
- (I): methyl 2,3-dihydro-7-(trifluoromethyl)-2-[[[4-(trifluoromethyl)phenylamino]carbonyl]-[1]benzopyrano[3,4-c]pyrrole-3a(4H)-carboxylate,
- (J): methyl 7-chloro-3,9-dihydro-3-[[[4-(trifluoromethoxy)phenylamino]carbonyl]indeno[1,2-e]-1,3-oxazine-9a(2H)-carboxylate,
- (K): methyl 7-chloro-4-formyl-2,3,4,5-tetrahydro-2-[[4-(trifluoromethoxy)phenylamino]carbonyl]-4aH-indeno[2,1-e]-1,2,4-triazine-4a-carboxylate, and
- (L): methyl 4-formyl-2,3,4,5-tetrahydro-7-(2,2,2-trifluoroethoxy)-2-[[4-(trifluoromethoxy)-phenylamino]carbonyl]-4aH-indeno[2,1-e]-1,2,4-triazine-4a-carboxylate.

In the above definitions, the term "alkyl", used either alone or in compound words such as "alkylthio" or "haloalkyl", denotes straight chain or branched alkyl, such as methyl, ethyl, n-propyl, isopropyl and the different butyl, pentyl, and hexyl isomers. "Alkoxy" denotes methoxy, ethoxy, n-propoxy, isopropoxy and the different butoxy and pentoxy isomers. "Alkenyl" denotes straight chain or branched alkenes, such as vinyl, 1-propenyl, 2-propenyl, 3-propenyl and the different butenyl, pentenyl, and hexenyl isomers. "Alkynyl" denotes straight chain or branched alkynes, such as ethynyl, 1-propynyl, 3-propynyl and the different butynyl, pentynyl, and hexynyl isomers. "Alkylthio" denotes methylthio, ethylthio and the different propylthio, butylthio, pentylthio, and hexylthio isomers. "Alkylsulfinyl", "alkylsulfonyl", "alkylamino", and.the like, are defined analogously to the above examples. "Cycloalkyl" denotes cyclopropyl, cyclobutyl, cyclopentyl, and cyclohexyl.

The term "halogen", either alone or in compound words such as "haloalkyl", denotes fluorine, chlorine, bromine or iodine. Further, when used in compound words such as "haloalkyl" said alkyl can be partially or fully substituted with halogen atoms, which can be the same or different. Examples of haloalkyl include CH₂CH₂F, CF₂CF₂ and CH₂CHFCl. The terms "halocycloalkyl", "haloalkenyl" and "haloalkynyl" are defined analogously to the term "haloalkyl".

The total number of carbon atoms in a substituent group is indicated by the "Cᵢ-Cⱼ" prefix where i and j are numbers from 1 to 8. For example, C₁-C₃ alkylsulfonyl designates methylsulfonyl through propylsulfonyl; C₂ alkylcarbonyl includes C(O)CH₃, and C₄ alkylcarbonyl includes C(O)CH₂CH₂CH₃ and C(O)OH(OH₃)₂; C₂ alkoxycarbonyl designates C(O)OCH₃ and C₄ alkoxycarbonyl designates C(O)OCH₂CH₂CH₃ and C(O)OCH(CH₃)₂; and as a final example, C₃ alkoxycarbonylalkyl includes CH₂C(O)₂CH₃ and C₄ alkoxycarbonylalkyl includes CH₂CH₂C(O)₂CH₃, CH₂C(O)₂CH₂CH₃ and CH(CH₃)C(O)₂CH₃.

Compounds of Formula I can exist as one or more stereoisomers. The various stereoisomers include enantiomers, diastereomers and geometric isomers. One skilled in the art will appreciate that one stereoisomer may be the more active. One skilled in the art knows how to separate said enantiomers, diastereomers and geometric isomers. Accordingly, the present invention comprises racemic mixtures, individual stereoisomers,and optically active mixtures.

### DETAILS OF THE INVENTION

Compounds of Formula I are prepared as described in Schemes 1 through 28 with substituents as previously defined, unless otherwise noted. Compounds of Formula I (Q-1) where R is H and X is O or S can be prepared by the reaction of Formula II compounds in the presence of a conventional organic solvent such as benzene, toluene, xylene, acetonitrile and the like. This transformation can be performed optionally in the presence of an acid catalyst (0 to 1 equivalents). Typical acid catalysts include p-toluenesulfonic acid, pyridine p-toluenesulfonate, hydrochloric acid or sulfuric acid. The reaction temperature can range from about -10 to 200°C with 35 to 120°C being preferred. The reaction is generally complete after 24 hours. Scheme 1 illustrates this transformation.

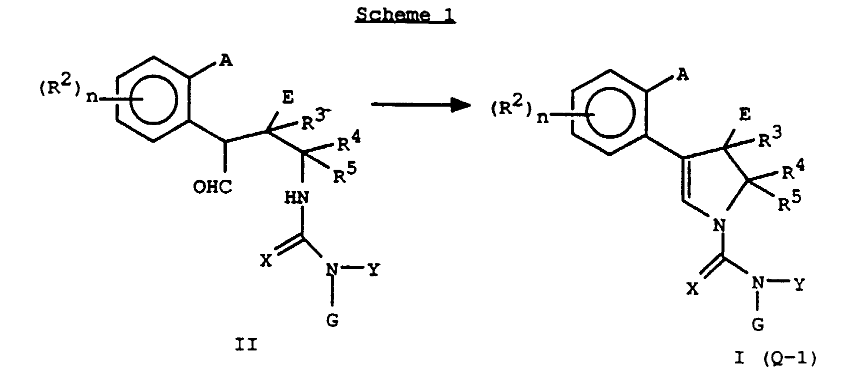

Compounds of Formula II can be prepared by the reaction of Formula III compounds with an oxidizing agent such as pyridine-sulfur trioxide complex/dimethyl sulfoxide, oxalyl chloride/dimethyl sulfoxide or pyridinium chlorochromate. These oxidation procedures are well documented in the literature. A typical reaction involves combination of Formula III compounds with the oxidizing agent in a solvent such as methylene chloride, chloroform, benzene or toluene. The reaction can be run in the.presence of a base such as triethylamine. The reaction temperatures can range from about -78 to 200°C. The reaction time can range from 30 minutes to 24 hours. Scheme 2 illustrates this transformation.

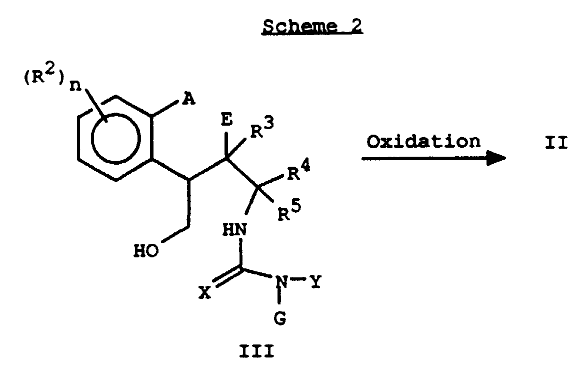

Compounds of Formula III where X is O or S can be prepared by the reaction of Formula IV compounds with isocyanates of Formula V. Typical reactions involve the combination of equimolar amounts of IV and V in a conventional organic solvent such as ethyl acetate, methylene chloride, chloroform, benzene and toluene. A base such as an alkali metal, alkali metal alkoxide or metal hydride can be used. The reaction temperatures can vary from 0°C to the reflux temperature of the particular solvent being used. The reaction is usually complete in less than 24 hours. Scheme 3 illustrates this transformation.

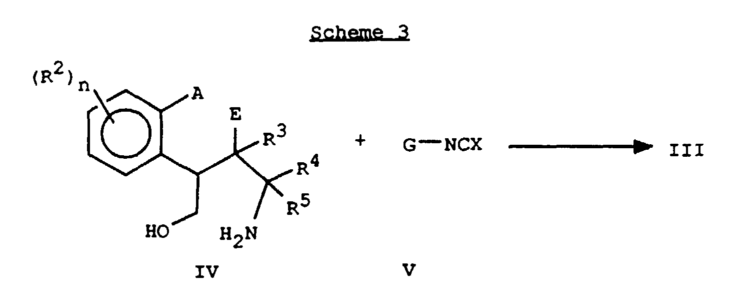

Compounds of Formula IV can be prepared by the reduction of Formula VI compounds. Typical reactions involve combinations of an excess in molar amounts of a reducing agent (1 to 10 equivalents) such as lithium aluminum hydride, with one equivalent of a Formula VI compound in a solvent such as tetrahydrofuran or ether. The reaction temperature can vary from 0°C to the reflux temperature of the particular solvent being used and the reaction is usually complete in 24 hours. This transformation is illustrated in Scheme 4.

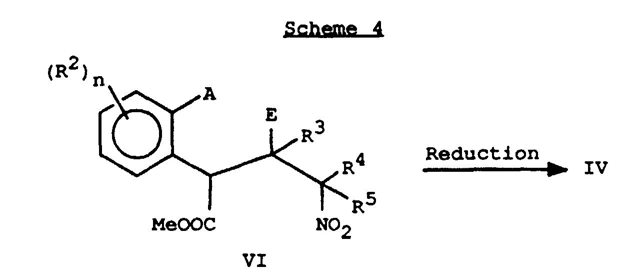

Compounds of Formula VI can be prepared using procedures known to the art (*J. Med. Chem.* 1991, 34, 2557; *Org. Syn.* 1952, *32,* 86).

Compounds of Formula I (Q-2) where R is H, X is O or S and Z is CH₂ can be prepared from Formula VII compounds utilizing analogous procedures described for Schemes 1 through 4 (see Scheme 5).

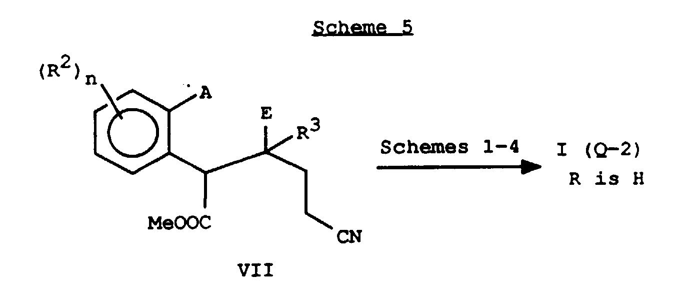

Compounds of Formula VII are known in the art or can be obtained by methods analogous to known procedures *(Synth. Commun. 1985, 15,* 899).

Compounds of Formula I (Q-3, where R is H and X is O; and Q-4, where R is H, X is O and Z is CH₂) can be prepared by reaction of acid chloride VIII with a substituted amine of Formula IX in equimolar proportions in the presence of an excess of an acid scavenger, such as tertiary alkylamines or pyridines in an aprotic organic solvent such as ether, tetrahydrofuran, chloroform, methylene chloride, benzene or toluene. The reaction temperature can vary between 0 to 50°C and the reaction is usually complete in less than 24 hours. Scheme 6 illustrates this transformation.

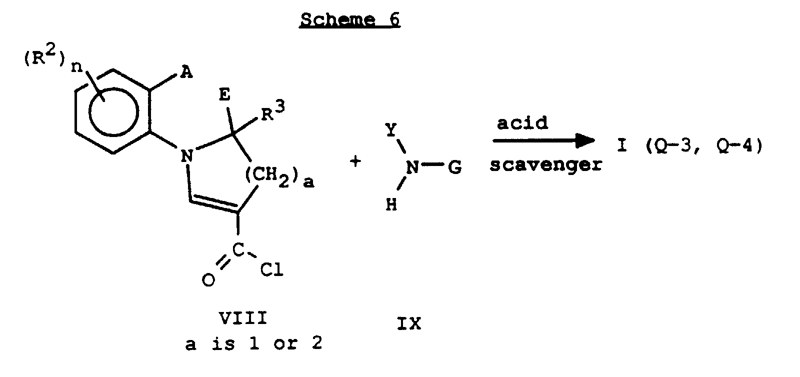

Compounds of Formula VIII can be prepared from compounds of Formula X through conventional methodology generally used for the conversion of esters to their corresponding acid chlorides as illustrated in Scheme 7.

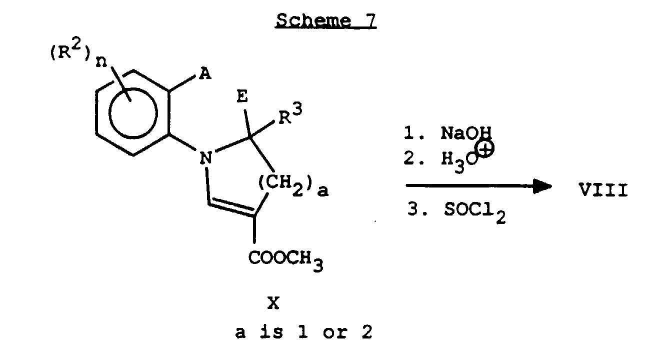

Compounds of Formula X can be readily prepared by the dehydration of alcohols of Formula XI (see Scheme 8). For a general review of dehydration reactions, see March, "Advanced Organic Chemistry", 3rd ed, pp. 901 to 903.

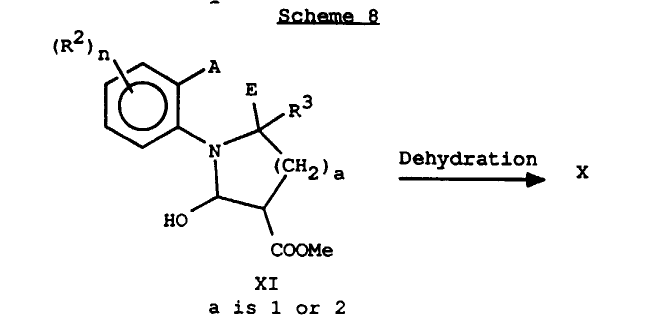

Compounds of Formula XI can be prepared by the reduction of Formula XII compounds. A typical reaction involves combination of an excess in molar amounts of a reducing agent such as sodium borohydride (1.1 equivalent to 3 equivalents) with a Formula XII compound. Conventional organic solvents such as methanol, ethanol, dimethoxyethane, ether or tetrahydrofuran can be used. Typical reaction time varies from 2 to 24 hours. This transformation is illustrated in Scheme 9.

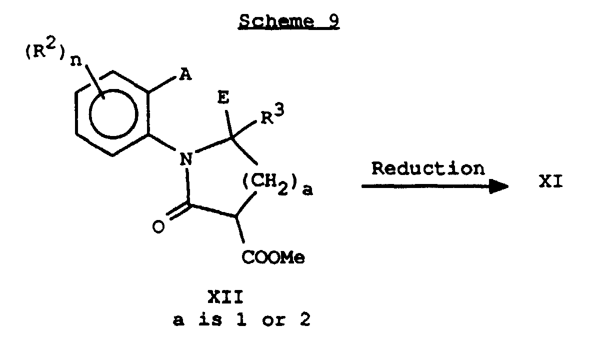

Compounds of Formula XII can be prepared from Formula XIII compounds (see Scheme 10). This is typically done by treatment of a Formula XIII compound with a strong base such as lithium diisopropylamide at low temperatures in the range of 0 to -78°C in a solvent such as tetrahydrofuran. An electrophile such as XIV is then added to the anion affording, after workup with acid, compounds of Formula XII. The reaction is usually complete in less than 24 hours. Literature precedence for Schemes 8 through 10 can be found in Tetrahedron Lett. 1991, 32, 6481.

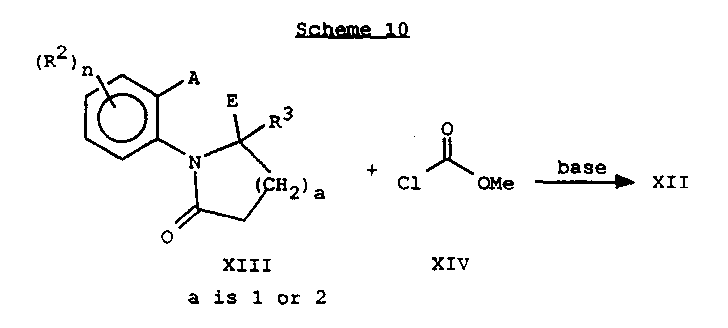

Compounds of Formula XIII can be prepared from compounds of Formula XV. Typically, Formula XV compounds are prepared by refluxing in a suitable solvent such as toluene, benzene, methanol or ethanol in the presence of a catalytic amount (0.1 to 0.5 equivalents) of a metal alkoxide such as sodium methoxide. Typical reaction times vary from 30 minutes to 4 hours. Scheme 11 illustrates this transformation.

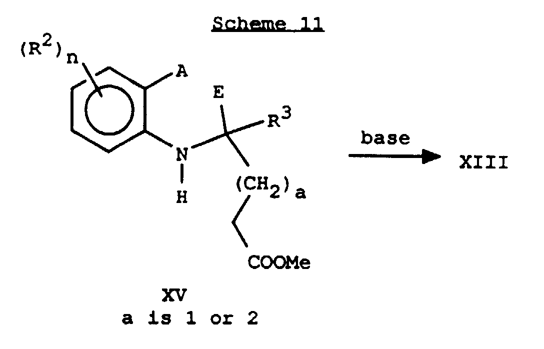

Compounds of Formula XV where E is H can be prepared by reductive amination of the ketoester XVII with an appropriately substituted aniline XVI (see March, "Advanced Organic Chemistry", 3rd ed. pp. 798-800 and *Tetrahedron Lett. 1990, 31,* 5547). This transformation is illustrated in Scheme 12.

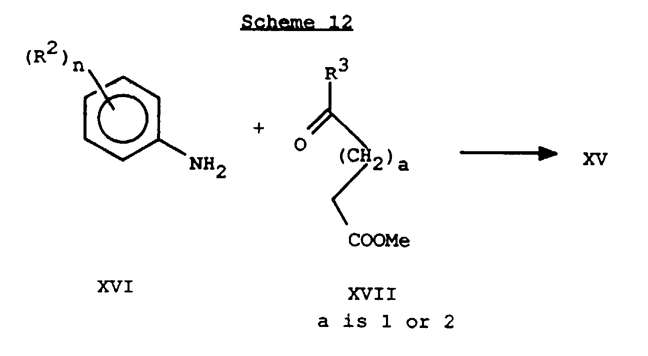

Compounds of Formula I (Q-5) can be prepared by reaction of compounds of Formula XVIII with compounds of Formula IX. Typical reactions involve the combination of equimolar amounts of XVIII and IX in a conventional organic solvent such as ethyl acetate, methylene chloride, chloroform, benzene or toluene. A base such as an alkali metal, tertiary amine, alkali metal alkoxide or metal hydride can be used. Scheme 13 illustrates this transformation.

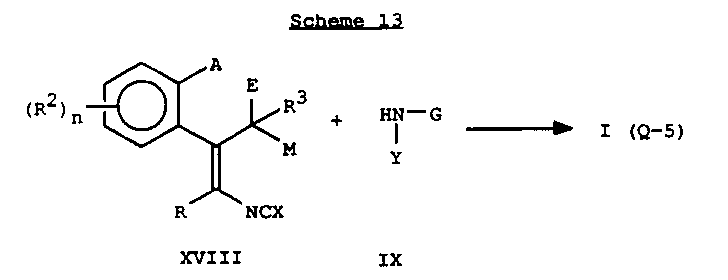

Compounds of the Formula XVIII (X = O) can be prepared by the Curtius rearrangement of compounds of the Formula XIX by procedures well known to the art (J. *Am. Chem. Soc.* 1927, 49, 2528). The reaction is usually performed in_an organic solvent such as benzene at temperatures of about 80°C. Scheme 14 illustrates this transformation.

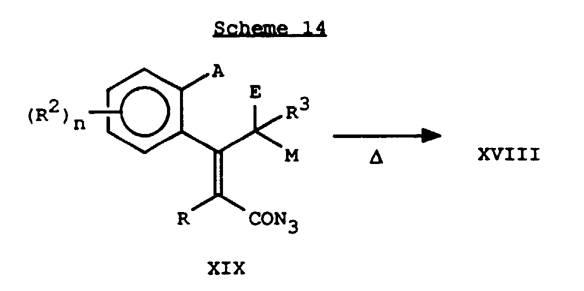

Compounds of Formula XVIII (X = S) can be prepared from compounds of Formula XX *(Chem. Zuesti* 1974, 28, 848) by reaction with thiophosgene in a conventional organic solvent such as dichloroethane, followed by treatment with triethyl amine, by procedures known to the art; (*J. Org. Chem. 1965, 30,* 1926). Scheme 15 illustrates this transformation.

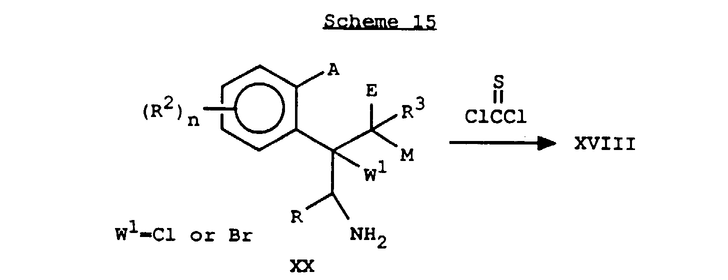

Compounds of Formula XIX can be prepared from compounds of Formula XXI by known procedures. Two carbon homologations are known (Wittig reaction, *Synthesis* 1979, 633). Suitable functionality can be introduced (e.g., an ester) in this way, such that azides of Formula XIX can be prepared by procedures that are known (Fieser & Fieser, "Reagents for Organic Synthesis," Vol. VI, 1967). Scheme 16 illustrates this transformation.

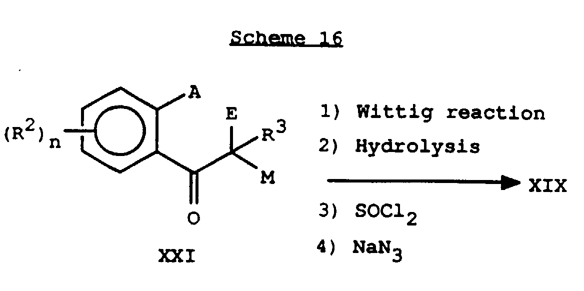

The starting ketones of Formula XXI are known in the art or can be obtained by methods analogous to known procedures. Those skilled in the art will recognize Formula XXI compounds to include deoxybenzoins, tetralones, chromanones, thiochromanones, benzofuran-3-ones, isochromanones, and others.

Compounds of Formula I (Q-6) can be prepared by the reaction of compounds of Formula XXII with a 1,3-dicarbonyl compound (or suitable equivalent) by known procedures (J. Het. *Chem.* 1969, 77, and references cited therein). Scheme 17 illustrates the transformation. 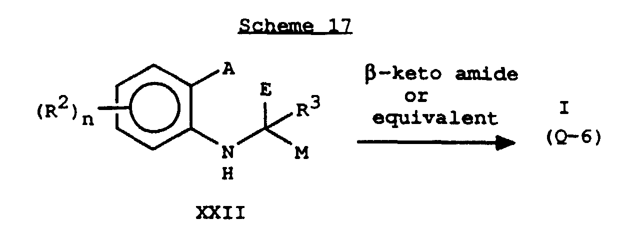 Compounds of Formula XXII are known and can be prepared by known procedures.

Compounds of Formula I (Q-7) can be prepared by the reaction of compounds of Formula XXIII with compounds of Formula IX. Typical reactions involve the combination of equimolar amounts of XXIII and IX in a conventional organic solvent such as ethyl acetate, dichloromethane or ether. A base such as triethylamine, pyridine, sodium amide, sodium methoxide or 2-hydroxypyridine can be added to the reaction.

The compound IX can optionally be pretreated with an organometallic reagent such as trimethylaluminium or a Grignard reagent, prior to reaction with XXIII. The compound XXIII (L = OH) can be optionally pretreated with a reagent such as 1,1'-carbonyldiimidazole. Compounds of Formula XXIII (L = OH, Cl, imidazole) are readily prepared from compounds of Formula XXIII (L = OMe or OEt) by methods known to those skilled in the art. Scheme 18 illustrates this transformation.

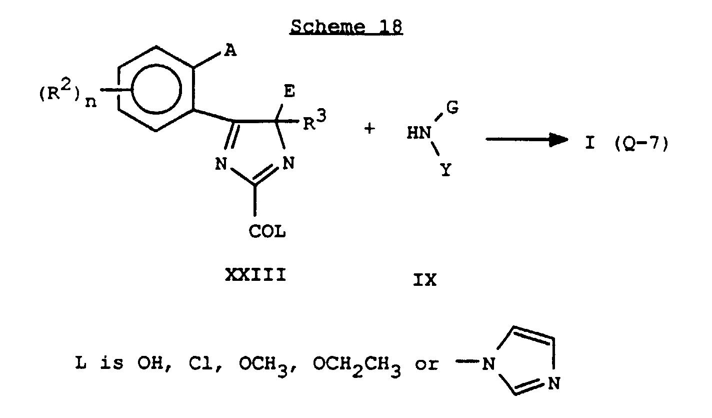

Compounds of Formula XXIII can be prepared from compounds of Formula XXIV by a two-step sequence. Reaction of ammonia with iminoyl chlorides is known to those skilled in the art (Ukr. *Khim. Zh.* (Russ. Ed.) 1979, 45(7), 624). Accordingly, treatment of compounds of Formula XXIV with ammonia in benzene at 80°C affords an intermediate aminal. This intermediate can be dehydrated directly By treatment with a suitable reagent such as phosphorus pentoxide in a conventional organic solvent such as dichloromethane. The reaction is usually complete in about 20 h at ambient temperature, although elevated temperatures in the range of 40-150°C can be used. Scheme 19 illustrates this transformation.

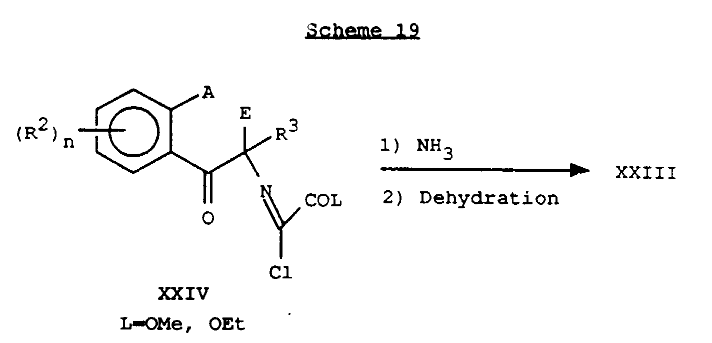

Compounds of Formula XXIV can be prepared from compounds of Formula XXV by a two-step sequence known to those skilled in the art. These are:
A: Preparation of an amide by addition of an oxalyl chloride derivative in a conventional organic solvent such as dichloromethane, in the presence of a suitable base such as triethylamine.
B: Preparation of an imidoyl chloride from an amide by use of a reagent such as ⌀'₃P/CCl₄, PCl₅ and the like. A conventional organic solvent such as dichloromethane or benzene can be used, and elevated temperatures up to 130°C can be used. Typically, the reaction is complete after a few hours at 40°C. Scheme 20 illustrates this transformation.

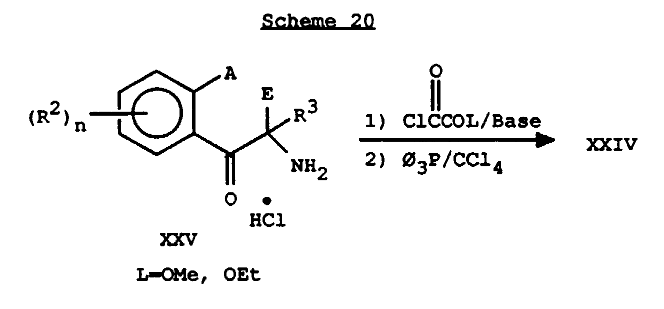

Compounds of Formula XXV can be prepared by the reaction of compounds of Formula XXVI with compounds of Formula XXVII by procedures similar to those described in the art *(Synthesis* 1991, 327). The conditions for this reaction are the combination of compounds of Formula XXVI with a small excess (1.1-1.5 eq.) of Formula XXVII derivatives in the presence of a base as a catalyst such as 1,4-diazabicyclooctane[2.2.2]octane (DABCO), 1,5-diazabicyclo[5,4,0]-undec-5-ene (DBU), lithium diisopropylamide, sodium hydroxide and the like. Suitable solvents include toluene, tetrahydrofuran, dioxane and water. Scheme 21 illustrates this transformation.

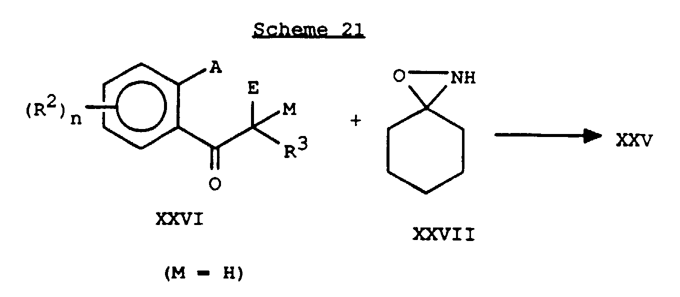

Compounds of Formula I (Q-8, R³⁵=CHO), where A and E are taken together, can be prepared by reaction of compounds of Formula I (Q-8, R³⁵=H) with acetic-formic anhydride; (see Reagents for Organic Synthesis, Fieser & Fieser, Vol 1, page 4). The compounds are combined in the absence of solvent, usually employing a large excess of acetic-formic anhydride, and the reaction is usually complete after 6 h. Scheme 22 illustrates the transformation. 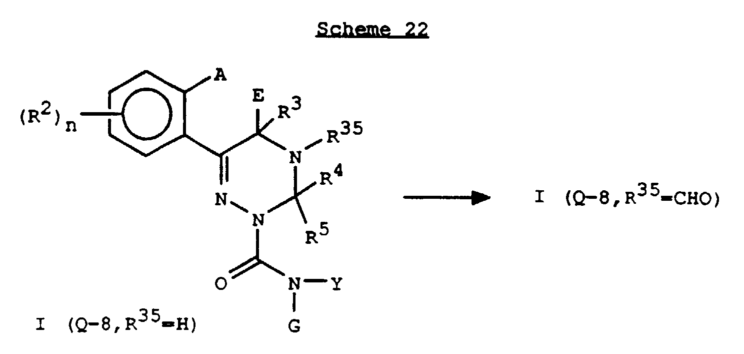 Compounds of Formula I (Q-8) where R³⁵ is other than CHO, can be prepared by standard alkylation, acylation, and sulfenylation reactions.

Alternatively, compounds of Formula I (Q-8) can be prepared by treatment of Formula XXVIII compounds with a slight excess of a reagent such as Eschermoser's salt, or a suitably substituted equivalent such as formaldehyde in a conventional organic solvent such as tetrahydrofuran. Reactions are usually complete within 24 h. Scheme 23 illustrates this tranformation.

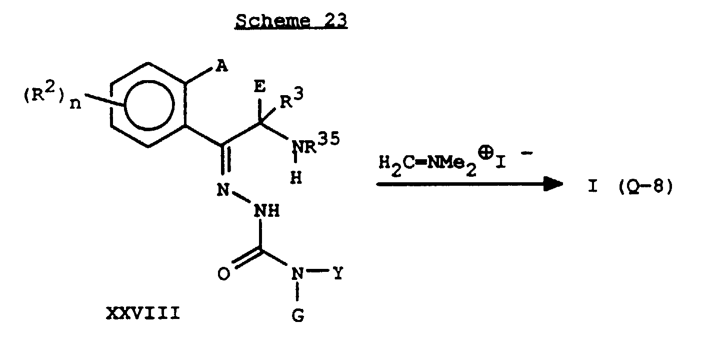

Compounds of Formula XXVIII can be prepared by the reaction of compounds of Formula XXV with semicarbazides of Formula XXIX. Conditions for this reaction require an acid catalyst such as hydrochloric, sulfuric or p-toluene sulfonic acid. Reaction temperatures can range from 0° to 150°C with the reflux temperature of the solvent used, generally preferred. Suitable solvents include methanol, ethanol, isopropanol, tetrahydrofuran and dioxane. Scheme 24 illustrates this transformation.

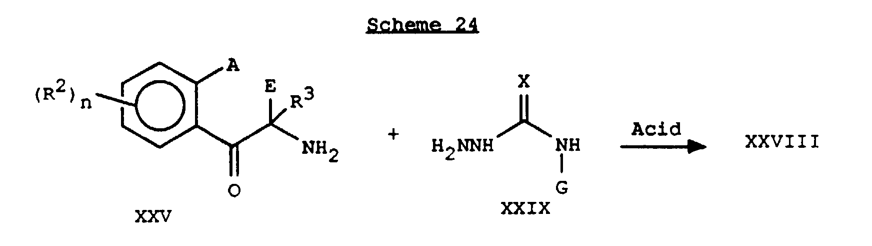

Compounds of Formula I (Q-9) (Y=R³⁸ and C₂-C₄ alkoxycarbonyl) can be prepared by treatment of compounds of Formula XXX with phosphorus pentoxide and dimethoxymethane in a conventional organic solvent such as dichloromethane, chloroform, 1,2-dichloroethane, or tetrahydrofuran. Typically, the reaction is complete in a few hours at 50°C, but can be conducted at temperatures between 25°-80°C with longer reaction times being experienced for lower temperatures. Scheme 25 illustrates this transformation.

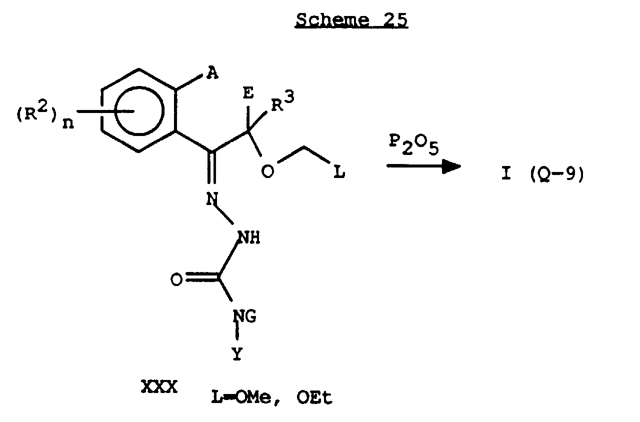

Compounds of Formula XXX can be prepared from compounds of Formula XXXI by methods known to those skilled in the art; see Fieser & Fieser, "Reagents for Organic Synthesis" (e.g., by use of phosphorus pentoxide/dimethoxymethane, or sodium hydride/chloromethyl methyl ether). Scheme 26 illustrates this transformation.

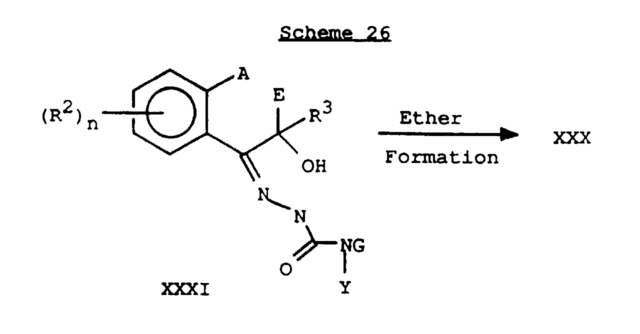

Compounds of Formula XXXI can be prepared by reacting compounds of Formula XXXII with compounds of Formula XXXIII in the presence of a suitable base such as triethylamine or sodium carbonate. The reaction is conducted in a conventional organic solvent such as dichloromethane, benzene, toluene, ether, or glyme and is usually complete in a few hours at ambient temperature. Scheme 27 illustrates this transformation.

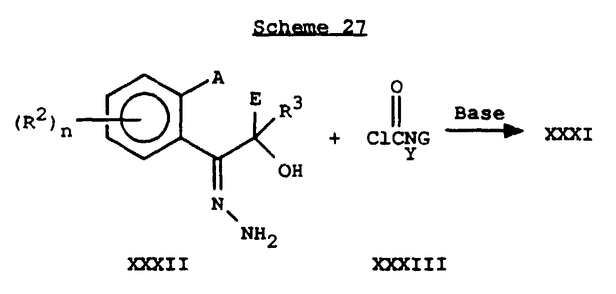

Compounds of Formula XXXII are known; see WO 92/11249. Compounds of Formula XXXIII can be prepared by treatment of compounds of Formula XXXIV with phosgene or phosgene equivalents in a conventional organic solvent such as benzene. Compounds of Formula XXXIV can optionally be treated with a base such as sodium hydride, sodium ethoxide or N,N-diethylaniline to facilitate the reaction with phosgene. The reaction is usually complete in a few hours at ambient temperature, but can be conducted at elevated temperatures if shorter reaction times are desirable. Scheme 28 illustrates this transformation.

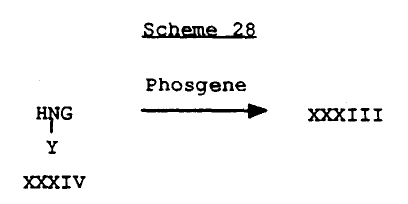

Alternatively., compounds of Formula I (Q-9) where Y is other than H, can be prepared by standard alkylation, acylation, or sulfenylation reactions by known methods.

It is recognized that in many of the transformations described it will be necessary to utilize appropriate protecting groups to prevent unwanted side reactions or use reagents that do not affect functionality other than that desired. One skilled in the art will be able to select appropriate protecting groups and reagents to this end.

### EXAMPLE 1

### Step A: 4-chloro-α-[(4-chlorophenyl)methylene]-acetic acid

A mixture of 17.0 g (0.1 mol) of 4-chlorophenylacetic acid, 16.9 g (0.12 mol) of 4-chlorobenzaldehyde, 25 mL of triethylamine and 25 mL of acetic anhydride was heated at reflux for 1 1/2 hours. Ice was added to the mixture after cooling and the pH adjusted to 6 with concentrated hydrochloric acid. The resulting mixture was extracted twice with ether, and the ether solution concentrated. The residue was dissolved in 1N sodium hydroxide and washed with ether:hexanes (1:1). The pH of the aqueous layer was adjusted to 3 with concentrated hydrochloric acid and extracted twice with ether. The combined ether extracts was dried over anhydrous magnesium sulfate, filtered and concentrated to give an oily solid. Recrystallization from ethanol:water (1:1) afforded 23.5 g of a light yellow solid, 178-180°C, ¹H NMR (CDCl₃) δ 7.88 (s, lH), 7.35-6.98, (2dd, 8H).

### Step B: methyl 4-chloro-α-[(4-chlorophenyl)methylene]-acetate

To a suspension of 10.0 g (0.034 mol) of the product of Step A in 70 mL methanol was added 5 mL of thionyl chloride. The reaction was heated at reflux for 45 min. After cooling, it was carefully poured into saturated aqueous sodium bicarbonate solution. The mixture was extracted twice with ether and the combined ether extracts washed with brine, dried over anhydrous magnesium sulfate, filtered and concentrated to give 10.06 g of a white solid, 87-88°C, ¹H NMR (CDCl₃) δ 7.80 (s, 1H) 7.36-6.96 (2dd, 8H), 3.80 (s, 3H).

### Step C: methyl 4-chloro-α-[(4-chlorophenyl)-β-(nitromethyl)benzene propanoate

A mixture of 8.52 g (0.028 mol) of the product of Step B and 2.2 mL of triton B (40% in methanol) in 55 mL of nitromethane was heated at reflux for 4 hours. After cooling, the mixture was made acidic with 1N hydrochloric acid. Water was added and the mixture extracted three times with methylene chloride. The combined methylene chloride extracts was washed with 1N hydrochloric acid, brine, dried over anhydrous magnesium sulfate, filtered, and concentrated to give an oil which was purified by chromatography on silica gel (ethyl acetate:hexanes 1:8) to give 2.6 g of a brown solid, 132-135°C, ¹H NMR (CDCl₃) δ 7.40-7.23 (m, 8H), 4.40 (dd, 1H), 4.25 (dd, 1H), 4.18 (m, 1H), 3.94 (d, 1H), 3.45 (s, 3H).

### Step D: methyl 4-chloro-α-[(4-chlorophenyl)-β-(aminomethyl)benzene propanol

To a suspension of 2.97 g (0.078 mol) of lithium aluminum hydride in 30 mL of tetrahydrofuran at 0°C was added dropwise a solution of 2.88 g (0.0078 mol) of the product of Step C in 30 mL of tetrahydrofuran. The mixture was heated at reflux for 2 hours, then stirred at ambient temperature for 2 days. Excess lithium aluminum hydride was carefully quenched with water. 50% aqueous sodium hydroxide was added to dissolve aluminum salts, and the mixture extracted three times with ethyl acetate. The combined ethyl acetate extracts was washed with brine, dried over anhydrous magnesium sulfate, filtered, and concentrated to give an oil which solidified on standing. The solid was triturated with ether:hexanes (1:1) to give 2.37 g of an off-white solid, 139-145°C, ¹H NMR (CDCl₃) δ 7.35-7.30 (m, 8H), 4.05 (m, 2H), 3.75 (m, 2H), 3.55 (m, 2H), 2.14 (bs, 3H).

### Step E: N-[2,3-bis(4-chlorophenyl)-4-hydroxybutyl]-N'-[4-(trifluoromethyl)phenyl]urea

To a suspension of 1.0 g (0.0032 mol) of the product of Step D in 5.5 mL tetrahydrofuran was added 0.603 g (0.0032 mol) of 4-trifluoromethylphenylisocyanate. The reaction was stirred at ambient temperature for 30 min., concentrated and the residue triturated with methylene chloride to give 1.18 g of a white solid. ¹H NMR (DMSO-d₆) δ 8.70 (s, 1H), 7.49-7.20 (m, 12H), 5.70 (t, 1H), 3.60-2.80 (m, 6H).

### Step F: 3,4-bis(4-chlorophenyl)-4,5-dihydro-N-[4-trifluoromethyl)phenyl]-1H-pyrrole-1-carboxamide

To a solution of 0.836 g (0.0017 mol) of the product of Step E and 1.7 mL of triethylamine in 2.5 mL of dimethyl sulfoxide was added a solution of 1.68 g (0.011 mol) of sulfur trioxide-pyridine complex in 5 mL of dimethyl sulfoxide. The reaction mixture was stirred at ambient temperature for 4 hours, poured into ice and the resulting solid filtered. The solid was washed several times with water and transferred into a flask. 50 mL of toluene was added and the mixture refluxed in a Dean-Starke trap to remove water. 17 mg of pyridine p-toluene sulfonate was added and refluxing continued for another hour. The reaction was concentrated and the residue purified by column chromatography on silica gel (methylene chloride: hexanes 4:1) to give 0.230 g of a white solid. Trituration with hexanes afforded 0.213 g of a white solid, 206-208°C. ¹H NMR (DMSO-d₆) δ 9.10 (s, lH), 7.87-7.30 (m, 13H), 4.74 (dd, lH), 4.38 (t, lH), 3.84 (dd, 1H).

### EXAMPLE 2

### Step A: methyl 2-amino-5-chloro-2,3-dihydro-1-oxo-1H-indene-2-carboxylate hydrochloride

An ice cold solution of hydroxylamine-o-sulfonic acid (11.4 g, 100.8 mmol) in water (102 mL) and sodium hydroxide solution (50 mL, 2N) was added in one portion to a mixture of cyclohexanone (10 g, 102 mmol), toluene (180 mL), and sodium hydroxide solution (50 mL, 2N) at 0°C. The mixture was stirred for 10 minutes. The organic layer was removed and dried over magnesium sulfate. Methyl 5-chloro-2,3-dihydro-1-oxo-2-1H-indene-2-carboxylate (7 g, 31.3 mmol) was added in one portion to a portion (155 mL) of the toluene solution. DABCO (0.1 g, 0.89 mmol) was added in one portion and the mixture was stirred for 1 h at 5°C. The mixture was washed with hydrochloric acid (2x50 mL, 1N). The acid washings were evaporated under reduced pressure to give the product (4.37 g), as a solid; m.p. 145-148°C (dec); ¹H NMR (free base) (CDCl₃) δ 7.73 (d, 1H), 7.48 (s, lH), 7.41 (d, 1H), 3.69 (s, 3H), 3.68 (1/2 ABq, 1H), and 3.05 (1/2 ABq, 1H).

### Step B: methyl 2-amino-5-chloro-2,3-dihydro-1- [[[-4-(trifluoromethoxy)phenylamino]carbonyl]-hydrazonol-1H-indene-2-carboxylate

A mixture of the product (2 g, 7.24 mmol) from Step A and 4-(4-trifluoromethoxy)phenylsemicarbazide (1.83 g, 7.78 mmol) in ethanol (18 mL) was boiled for 2 h. The mixture was allowed to cool and was stirred at room temperature overnight. The mixture was poured into sodium bicarbonate solution (200 mL, saturated) and then extracted with ethyl acetate (3x100 mL). The combined extracts were dried and evaporated and the material washed with ether to give 1.0 g of an off-white solid, of which a small portion was further purified by chromatography on silica gel (ethyl acetate/ethanol 5:1), m.p. 156.5 to 158°C (dec). ¹H NMR (CDCl₃) δ 8.4 (s, 1H), 8.20 (s, 1H), 7.83 (d, 1H), 7.56 (d, 2H), 7.42-7.18 (m, 4H), 3.73-3.65 (m, 4H), 3.05 (1/2 ABq, 1H).

### Step C: methyl 7-chloro-2,3,4,5-tetrahydro-2-[[4-(trifluoromethoxy)phenylamino]carbonyl]-4aH-indeno[2,1-e]-1,2,4-triazine-4a-carboxylate

Esthermoser's salt (0.8 g, 4.32 mmol) and the product (1.97 g, 4.31 mmol) from Step B were added to tetrahydrofuran (35 mL) and the mixture was stirred for 20 h at room temperature. The mixture was poured into water and was extracted with ethyl acetate (2x100 mL). The combined organic extracts were dried and evaporated. The product was purified by chromatography on silica gel (eluted with ethyl acetate/hexanes (3:2)) and afforded 0.87 g (43%) as a white solid; m.p. 183-187°C; IR (Mineral Oil) 3368, 3291, 1752, 1665, 1639, 1603, 1563, 1535, 1416, 1316, 1275, 1226, 1200, 1174, 1068, 1009, 945, 914, 890, 828 cm⁻¹; ¹H NMR (CDCl₃) δ 8.42 (s, 1H), 7.63-7.14 (m, 7H), 5.38 (1/2 ABq, 1H), 4.42-4.32 (m, 1H), 3.70 (s, 3H), 3.52 (1/2 ABq, 1H), 3.07 (1/2, 1H ABq), 2.43 (br s, 1H).

### Step D: methyl 7-chloro-4-formyl-2,3,4,5-tetrahydro-2[[4-(trifluoromethoxy)phenylamino]carbonyl]-4aH-indeno[2,1-e]-1,2,4-triazine-4a-carboxylate

Acetic anhydride (0.6 mL) was added in one portion to formic acid (0.5 g). When the mixture had cooled to room temperature, the product from Step C (0.12 g, 2.56 mmol) was added in one portion. After 2 h, the mixture was poured into sodium bicarbonate solution (100 mL, saturated), and the solution was extracted with ethyl acetate (3x50 mL). The combined extracts were dried and evaporated to give the product (0.12 g, 94%) as a solid; m.p. 204-206°C; IR (Mineral oil) 3301, 1741, 1691, 1664, 1604, 1562, 1533, 1416, 1319, 1262, 1240, 1219, 1193,. 1163, 1091, 1012, 883, 831, 805 cm⁻¹; ¹H NMR (CDCl₃) 9.63 (s, ∼0.6H), 9.55 (s, ∼0.4H), 8.49 is, ∼0.6H), 8.45 (s, ∼0.4H), 7.98-7.33 (m, 7H), 5.82 (1/2 ABq, ∼0.7H), 5.76 (1/2 ABq, ∼0.3H), 4.69 (1/2 ABq, 1H), 4.0 (1/2 ABq, ∼0.7H), 3.83 (1/2 ABq, ∼0.3H), 3.77 (1/2 ABq, ∼0.3H), 3.61 (s, ∼1H), 3.53 (s, ∼2H), 3.36 (1/2 ABq, ∼0.7H). (Two conformations are apparent by NMR, and by integration the ratio appears to be 2.3-1.5:1).

### EXAMPLE 3

### Step A: 2-amino-6-chloro-3,4-dihydro-2-methyl-1(2H)-napthalenone hydrochloride

Ice cold sodium hydroxide solution (75 mL, 2N) was added to hydroxylamine-O-sulfonic acid (17.1 g, 0.15 mol) in water (150 mL) at 0°C. When the solution had cooled to about 5°C it was added in one portion to a mixture of sodium hydroxide solution (75 mL, 2N), cyclohexanone (15 g, 0.15 mol) and toluene (270 mL) at 0°C. The mixture was stirred for 10 min. The organic layer was separated, dried and decanted from the drying agent. This afforded a solution of the cyclohexane-spiro-3'-oxaziridine which was used directly. The oxyaziridine solution prepared as above (540 mL) was added in one portion to an enolate (previously prepared by addition of 2-methyl-6-chlorotetralone (18.07 g, 92.9 mmol) in THF (40 mL) to lithium diisopropylamide (10.4 g, 97.1 mmol) in THF/hexanes (60 mL/40 mL) at -50°C. When the addition was complete, the mixture was stirred for 1 h at -50°C). The reaction was stirred for 1 h, and was allowed to warm to 0°C. The mixture was poured into water (500 mL), and the organic layer was separated. The aqueous layer was further extracted with dichloromethane (3x250 mL). The combined extracts were dried, hydrochloric acid (about 10 mL, conc.) was added, and the mixture was evaporated. Ether (250 mL) was added and hydrochloric acid (about 10 mL, conc.) was added. The mixture was stirred overnight and was filtered to give the product as a solid (19.18 g, 83%). ¹H NMR (free base) (CDCl₃) 8.00 (d, 1H), 7.32-7.25 (m, 2H), 3.19-2.89 (m, 2H), 2.18-1.98 (m, 2H), 1.29 (s, 3H).

### Step B: methyl [(6-chloro-3,4-dihydro-2-methyl-1-oxo-2(1H)-naphthalenyl)amino]oxoacetate

Methyl oxalyl chloride (2.55 g, 20.8 mmol) was added to the product of Step A (5.15 g, 20.8 mmol) and triethylamine (4.2 g, 41.5 mmol) in dichloromethane (20 mL). After 20 min, the mixture was poured into water (100 mL), and the organic layer was removed. The aqueous layer was further extracted with ethyl acetate (2x20 mL). The combinated extracts were dried and evaporated. Crystallization from hexanes/ether afforded 4 g (65%) of a solid: m.p. 128-129.5°C. IR (Mineral oil): 3310, 1743, 1710, 1681, 1592, 1565, 1525, 1411, 1348, 1310, 1286, 1244, 1227, 1114, 1006, 977, 961, 937, 895, 865 and 849 cm⁻¹, ¹H NMR (CDCl₃) δ 8.32 (s, lH), 8.0 (d, 1H), 7.34 (d, 1H), 7.27 (s, lH), 3.91 (s, 3H), 3.19-2.93 (m, 3H), 2.30-2.22 (m, 1H), 1.61 (s, 3H).

### Step C: methyl chloro[(6-chloro-3,4-dihydro-2-methyl-1-oxo-2(1H)-naphthalenyl)imino]acetate

Triphenylphosphine (1.95 g, 7.44 mmol) was added to the product from Step B (2 g, 6.77 mmol) and carbon tetrachloride (1.15 g, 7.46 mmol) in dichloromethane (14 mL). The mixture was boiled for about 3 h. A further portion of carbon tetrachloride (0.12 g, 0.78 mmol) and triphenylphosphine (0.2 g, 0.67 mmol) was added. The mixture was boiled for about 1 h. The mixture was allowed to cool and stirred at room temperature overnight. The mixture was diluted with ether (100 ml) and filtered. The filtrate was evaporated to dryness to give 3.84 g of a solid. A portion (0.2 g) of the solid was purified by chromatography on silica gel (eluted with ethyl acetate/hexanes 1:1) to give 0.05 g of a yellow solid. IR (mineral oil): 1744, 1678, 1593, 1566, 1548, 1512, 1442, 1372, 1333, 1295, 1271, 1226, 1082, 1031, 969, 910, 897, 868, 831 cm⁻¹. ¹H NMR (CDCl₃) δ 8.01 (d, 1H), 7.35-7.26 (m, 2H), 3.90 (s, 3H), 3.06 (t, 2H), 2.75-2.60 (m, 1H), 2.23-2.10 (m, 1H), 1.68 (s, 3H).

### Step D: methyl 7-chloro-3a,5-dihydro-3a-methyl-4H-napth[1,2-d]imidazole-2-carboxylate

Ammonia was passed through the product from Step C (2.94 g, 9.36 mmol) in boiling benzene (100 mL). After about 5 h, the mixture was allowed to cool and was diluted with ethyl acetate (100 mL). The mixture was extracted with hydrochloric acid (1N, 2x60 mL). The combined acidic extracts were made basic by addition of sodium carbonate solution. The mixture was extracted with ethyl acetate (3x60 mL), and the combined extracts were dried and evaporated. Crystallization from ether/hexane gave 0.9 g of a solid product. Phosphorus pentoxide was added to a portion (0.07 g, 0.23 mmol) of this product in dichloromethane (5 mL). The mixture was stirred at room temperature overnight, and was poured into a mixture of sodium carbonate solution (100 mL) and ice. The mixture was extracted with ethyl acetate (3x20 mL) and the combined extracts were dried and evaporated. Chromatography on silica gel (eluted with ethyl acetate) afforded 20 mg (30%) of a white crystalline solid. IR (mineral oil): 1736, 1592, 1539, 1185, 1132, 889, 838, 809 cm⁻¹, ¹H NMR (CDCl₃) δ 8.06 (d, 1H), 7.37-7.33 (m, 2H), 7.04 (s, 3H), 3.25 (1/2 AB of ABX, 1H), 3.06 (1/2 AB of ABX, 1H), 2.65 (1/2 AB of ABX, 1H), 1.84-1.66 (m, 1H), 1.36 (s, 3H). MS m/e 276(M).

### Step E: 7-chloro-3a,5-dihydro-3a-methyl-N-((4-(trifluoromethoxy)phenyl))-4H-napth((1,2-D))imidazole-2-carboxamide

The compound, 4-trifluoromethoxy aniline (0.51 g, 2.88 mmol), in THF (3 mL) was added dropwise to methyl magnesium chloride (0.96 mL, 3M) in THF (1 mL). When all effervescence had ceased, a portion (about 2 mL, about 1.16 mmol) of the solution was added dropwise to a portion (0.16 g, 0.57 mmol) of the product from Step D in THF (6 mL). The mixture was poured into hydrochloric acid (50 mL, 1N), and the aqueous mixture was extracted with ethyl acetate (1x50 mL). The organic extracts were washed with sodium bicarbonate solution (1x50 mL), dried and evaporated. Crystallization from ether/hexanes gave 0.12 g (50%) of a white solid: m.p. 109-110.5°C. IR (mineral oil): 3321, 1669, 1598, 1579, 1549, 1411, 1267, 1221, 1178, 1095, 1018, 993, 952, 901, 876, 824 cm⁻¹, ¹H NMR (CDCl₃) δ 9.33 (s, 1H), 8.05 (s, 1H), 7.80 (d, 1H), 7.38-7.36 (m, 2H), 7.25 (d, 1H), 3.36-3.27 (m, 1H), 3.14-3.05 (m, 1H), 2.68-2.63 (m, 1H), 1.82-1.73 (m, 1H), 1.39 (s, 1H). MS m/e 421(M).

### Key for Tables

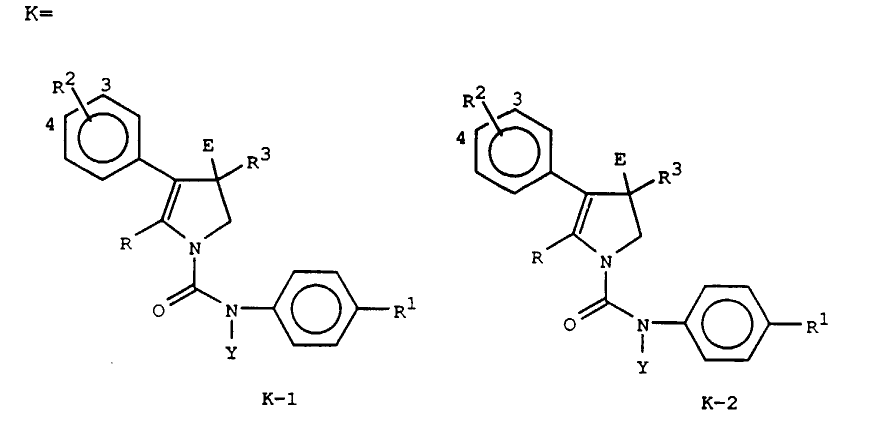

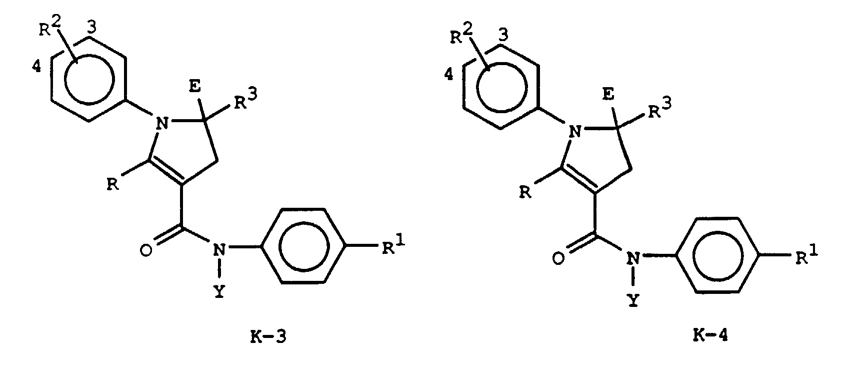

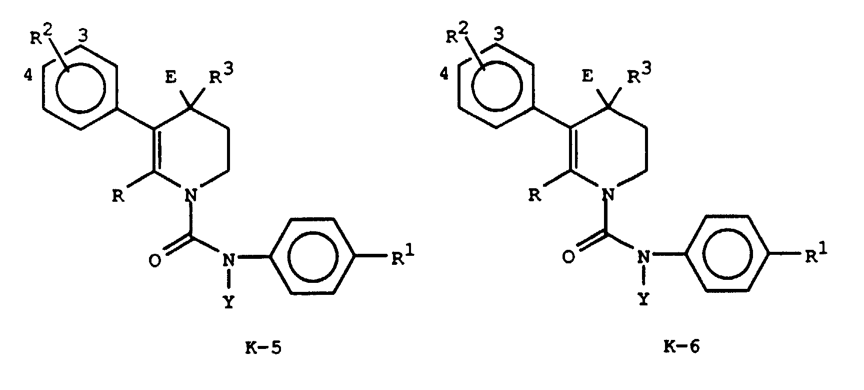

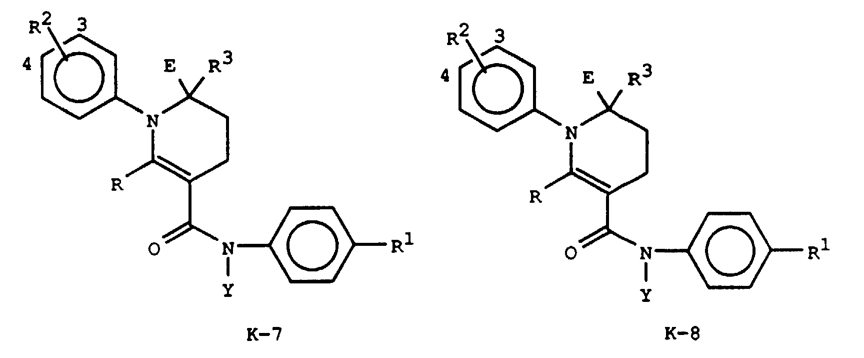

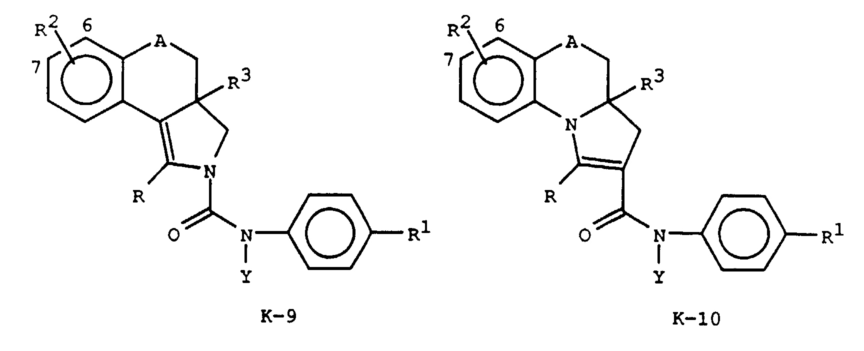

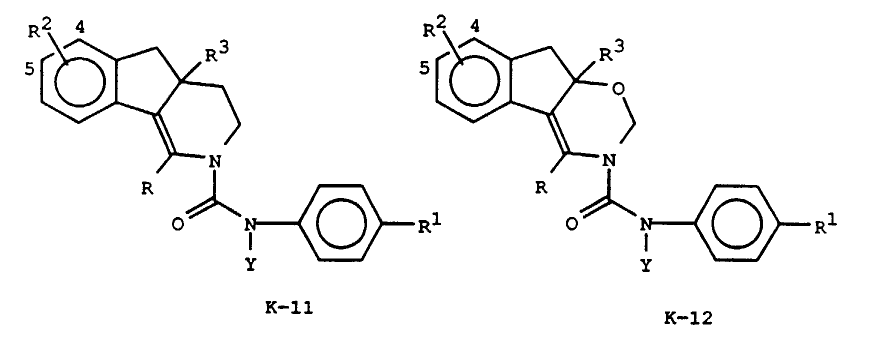

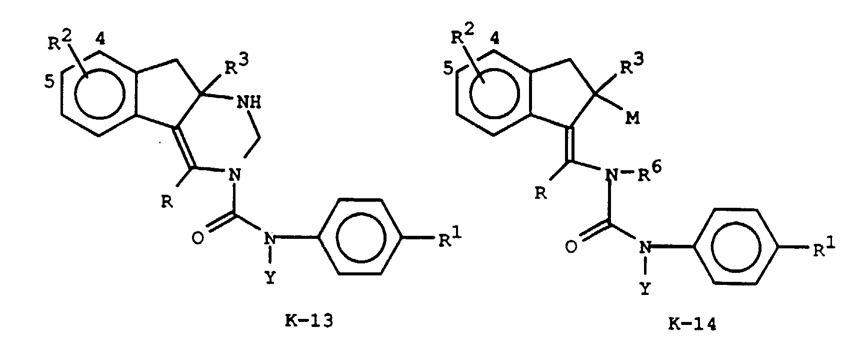

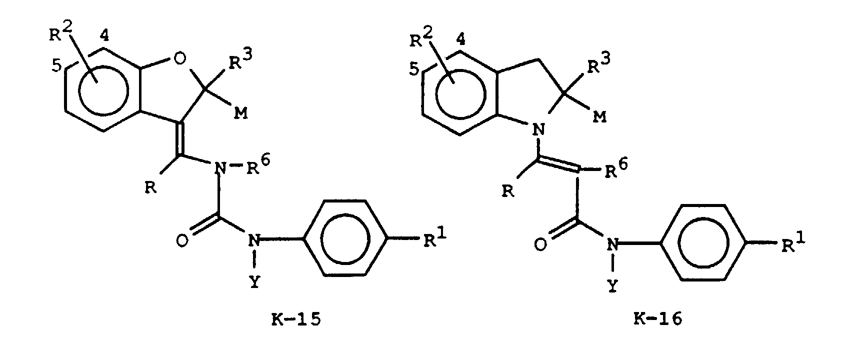

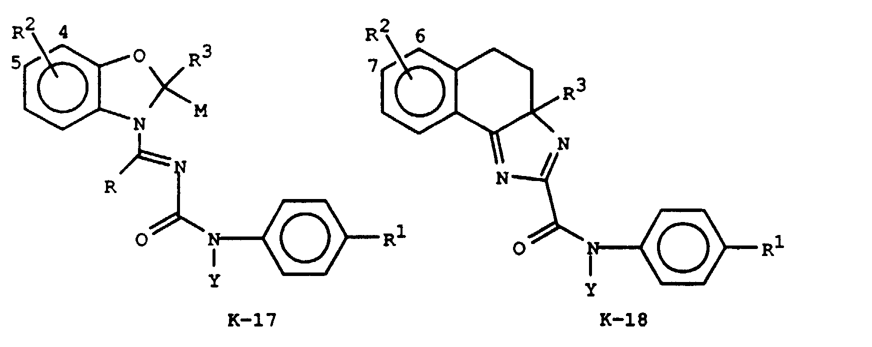

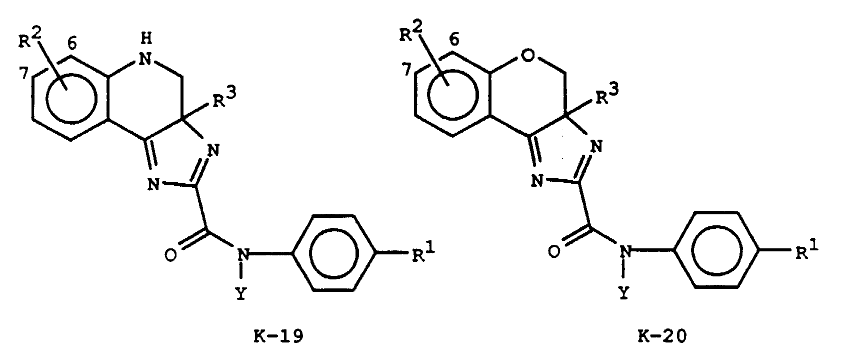

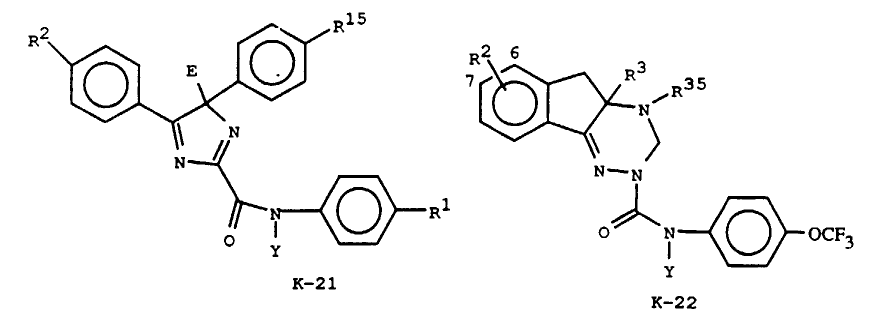

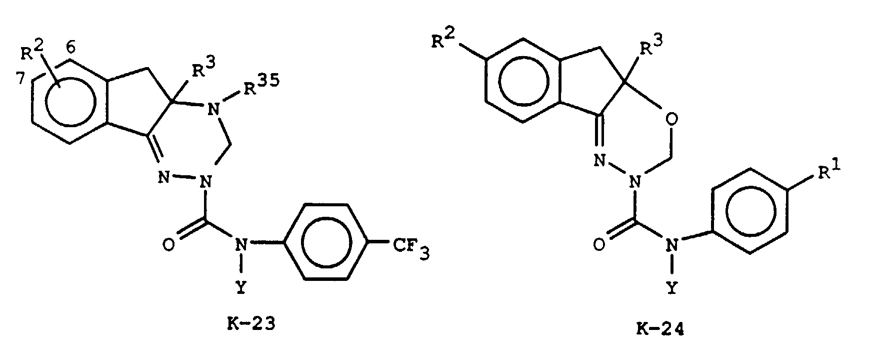

By the procedures described herein, the following compounds of Tables 1-24 can be prepared. The compounds in Table 1, line 1 can be referred to as 1-1 and 1-2 (as designated by line and column). The four separate compounds in Table 22, line 1 are compounds wherein K=K-22, R²=6-Cl, R³⁵=CHO, Y=H, and R³ is one of CO₂Me, CO₂Et, 4-F-Ph, or 4-Cl-Ph. Thus, the four compounds of Table 22, line 1 are 1-1 (R³-CO₂Me), 1-2 (R³=CO₂Et), 1-3 (R³=4-F-Ph), and 1-4 (R³=4-Cl-Ph). The other lines 2-510 describe four separate compounds per line similarly to line 1.

The abbreviations, Me, Et, i-Pr, Ac, and Ph have the following meaning:
Me = -CH₃;
Et = -CH₂CH₃;
n-Pr = CH₂CH₂CH₃;
i-Pr = -CH(CH₃)₂;
Ac = -C(O)CH₃; and

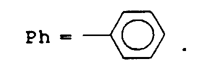

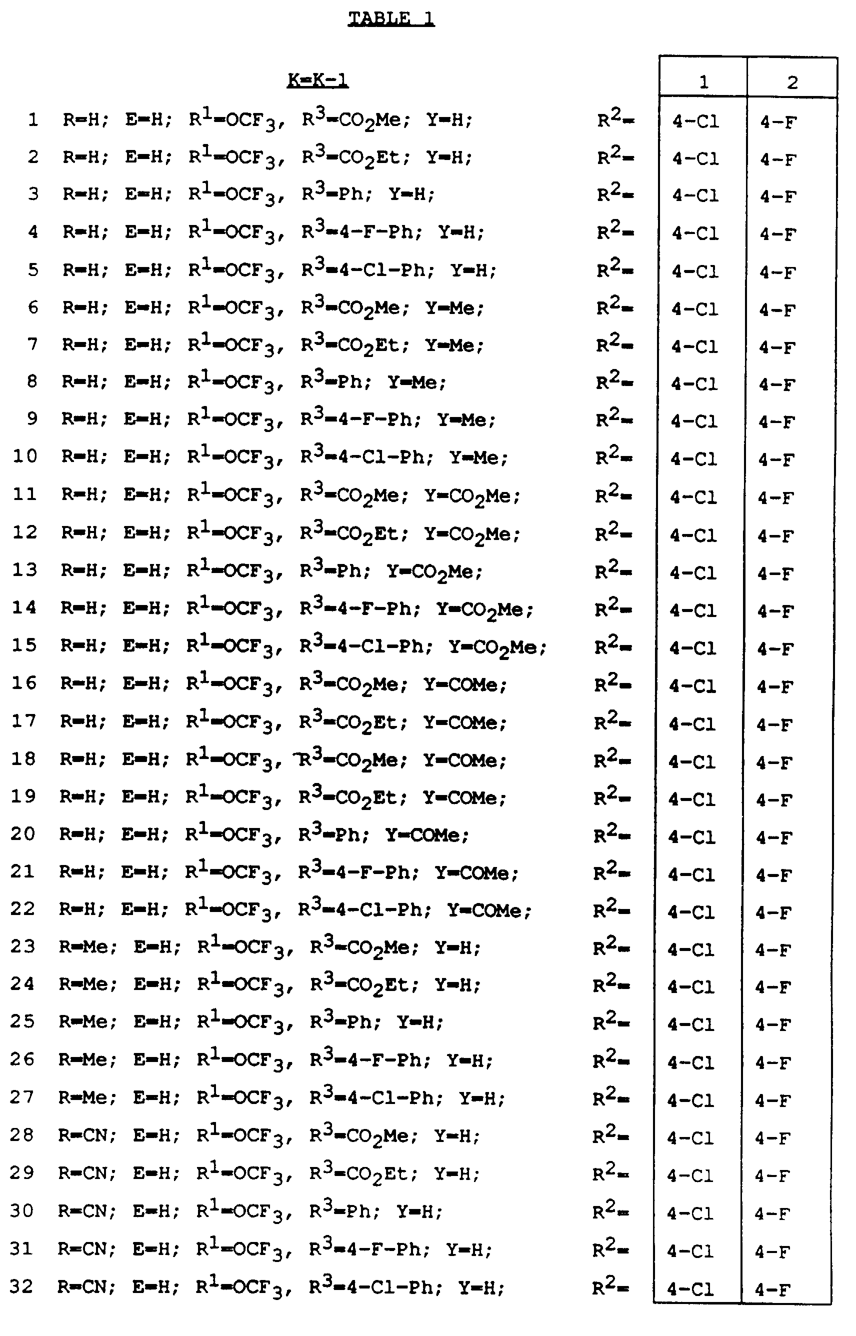

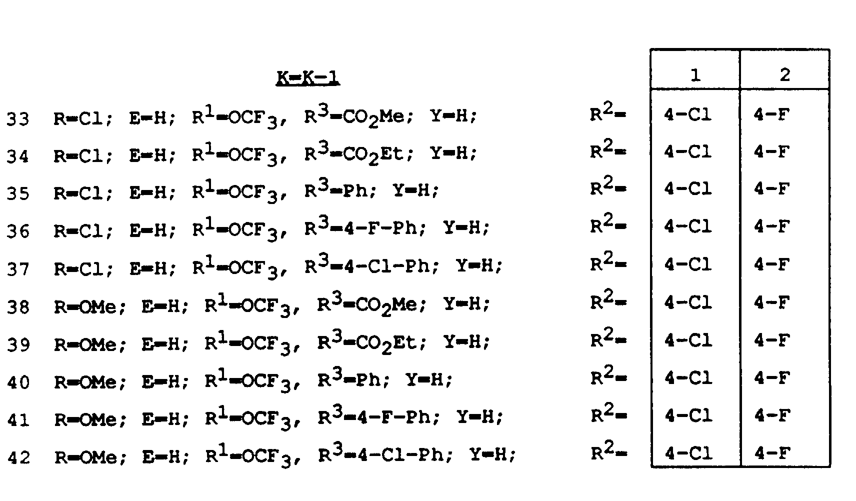

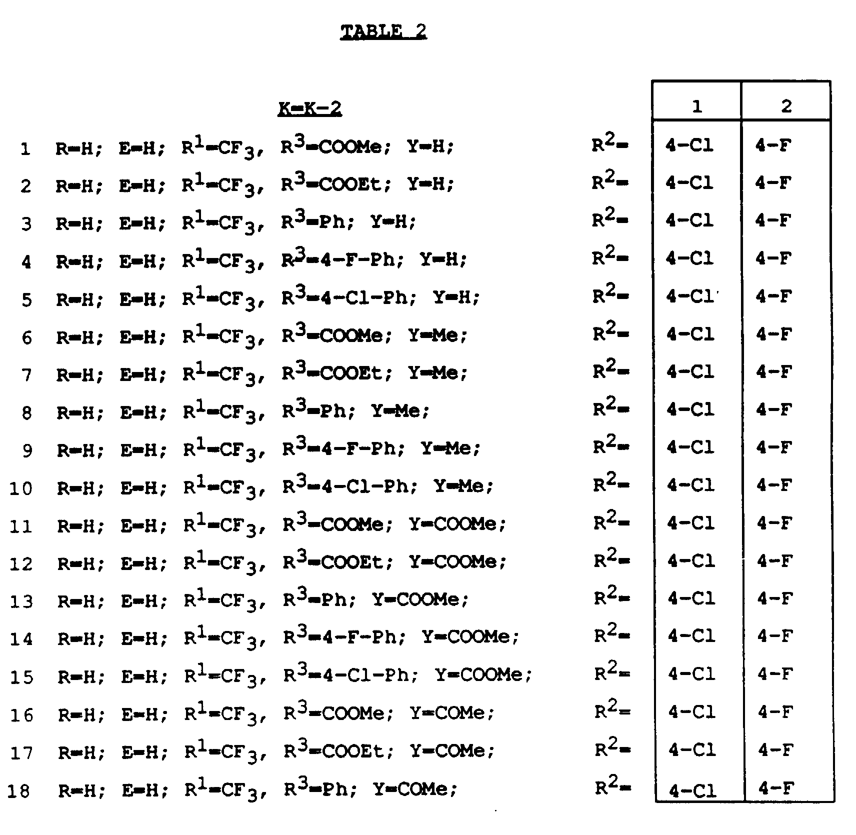

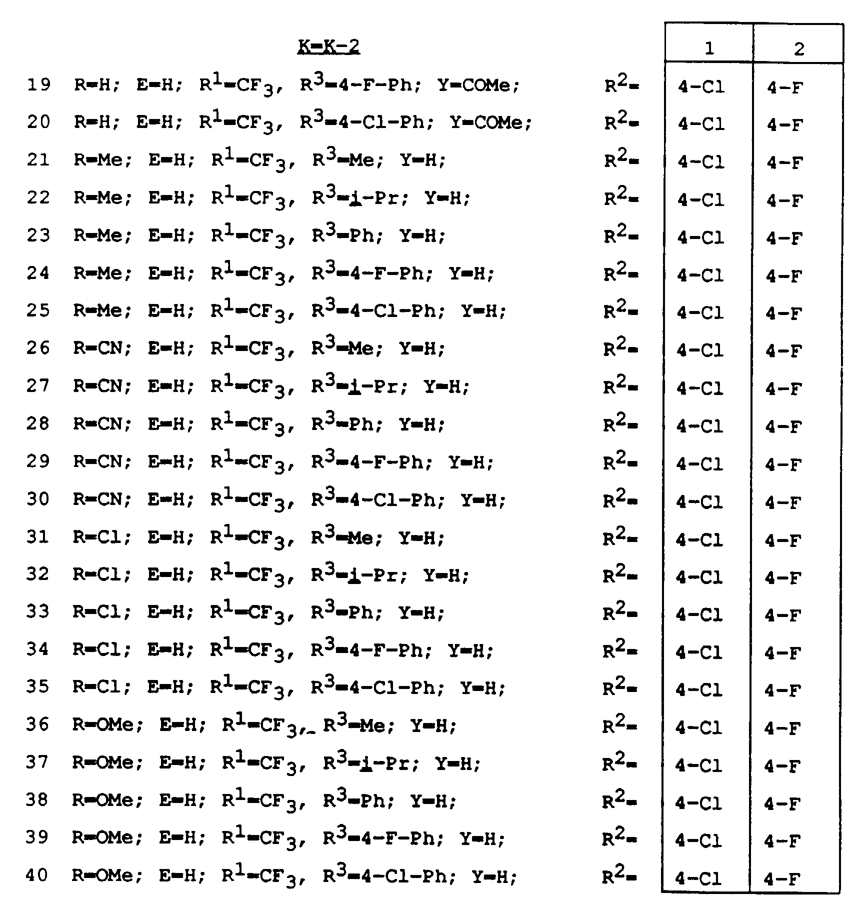

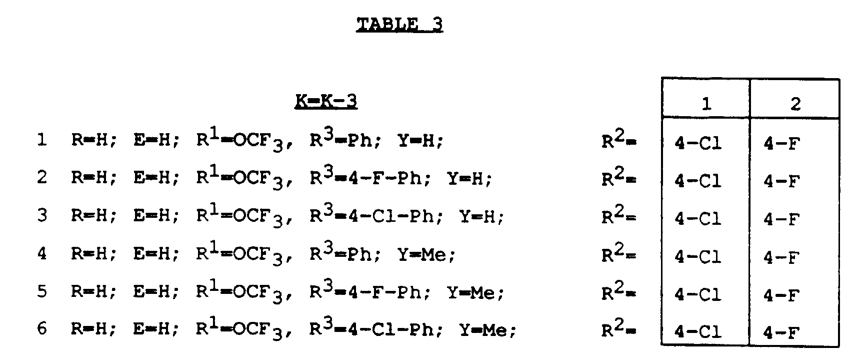

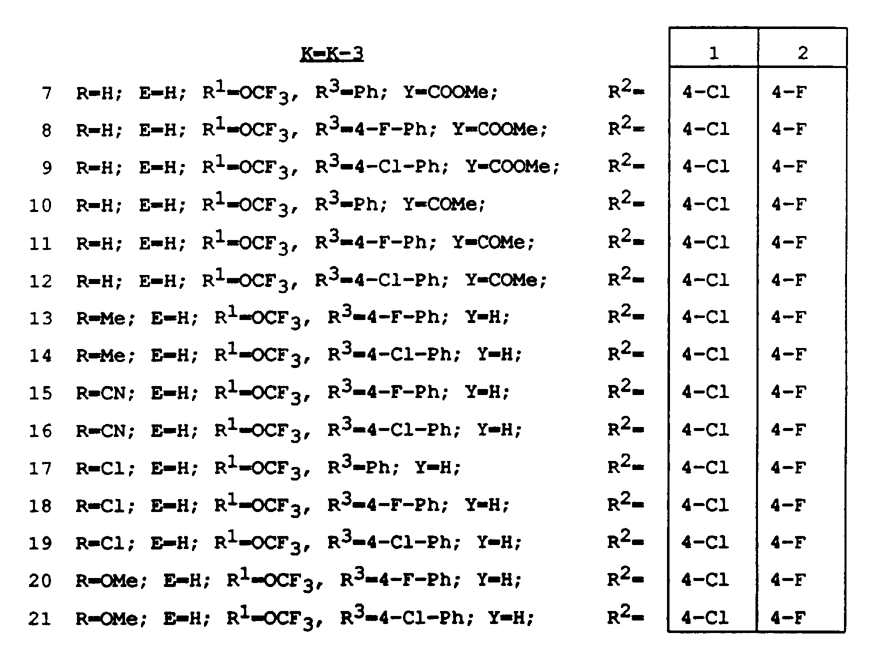

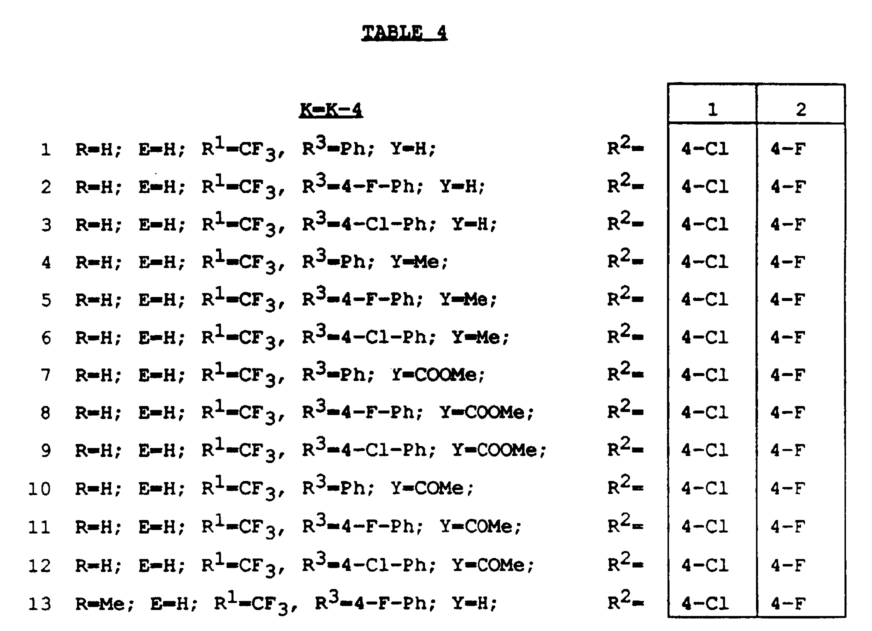

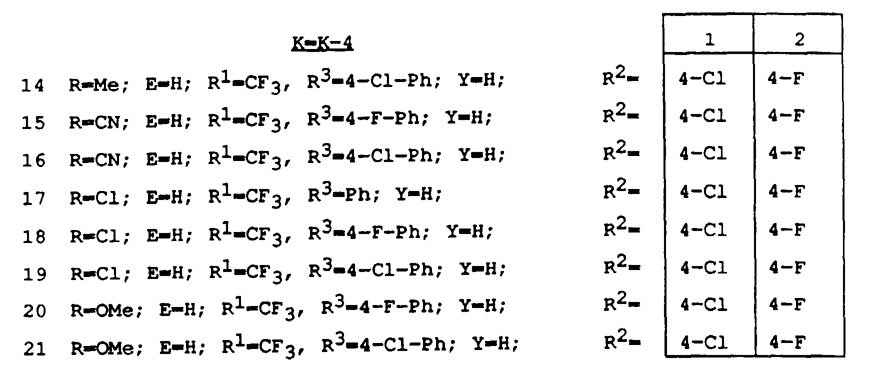

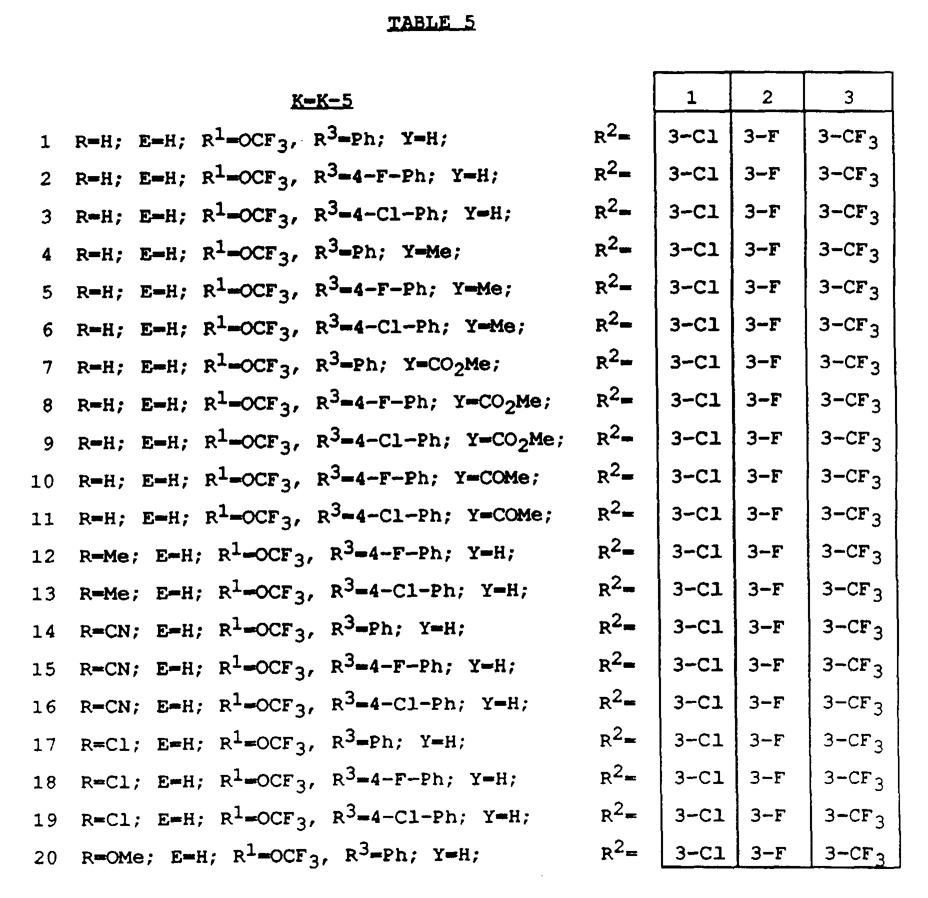

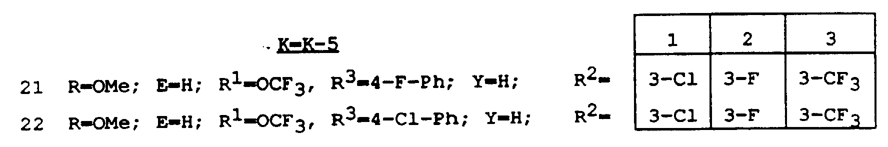

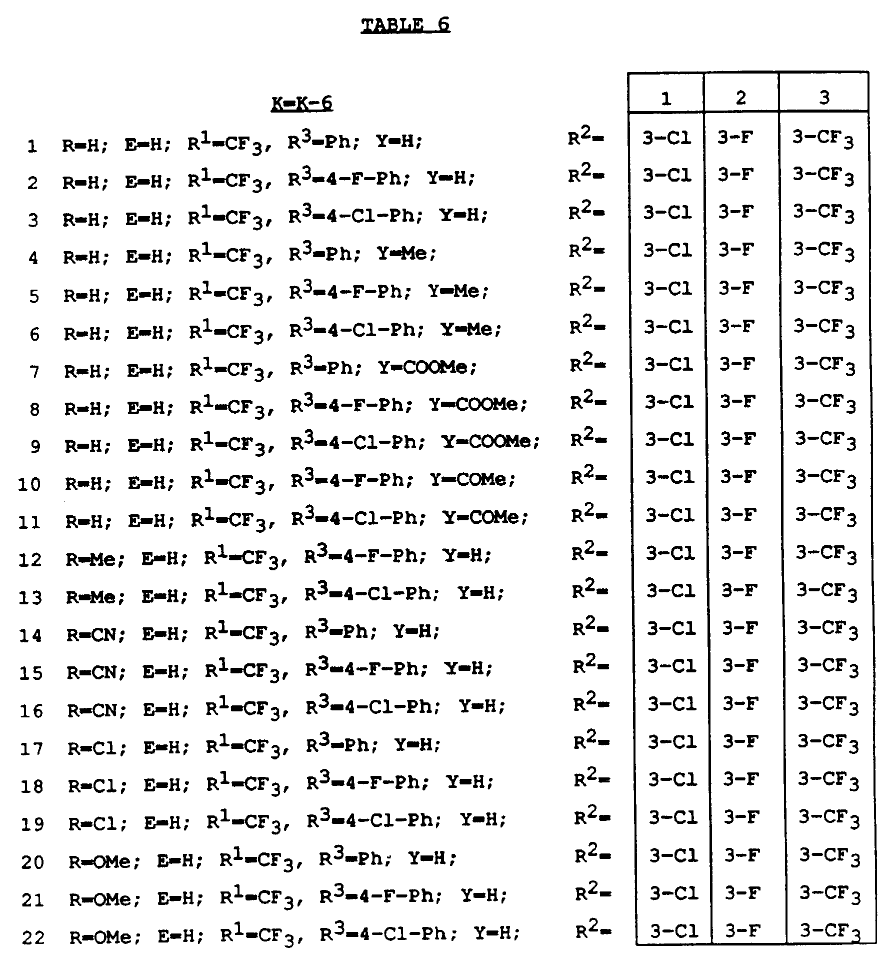

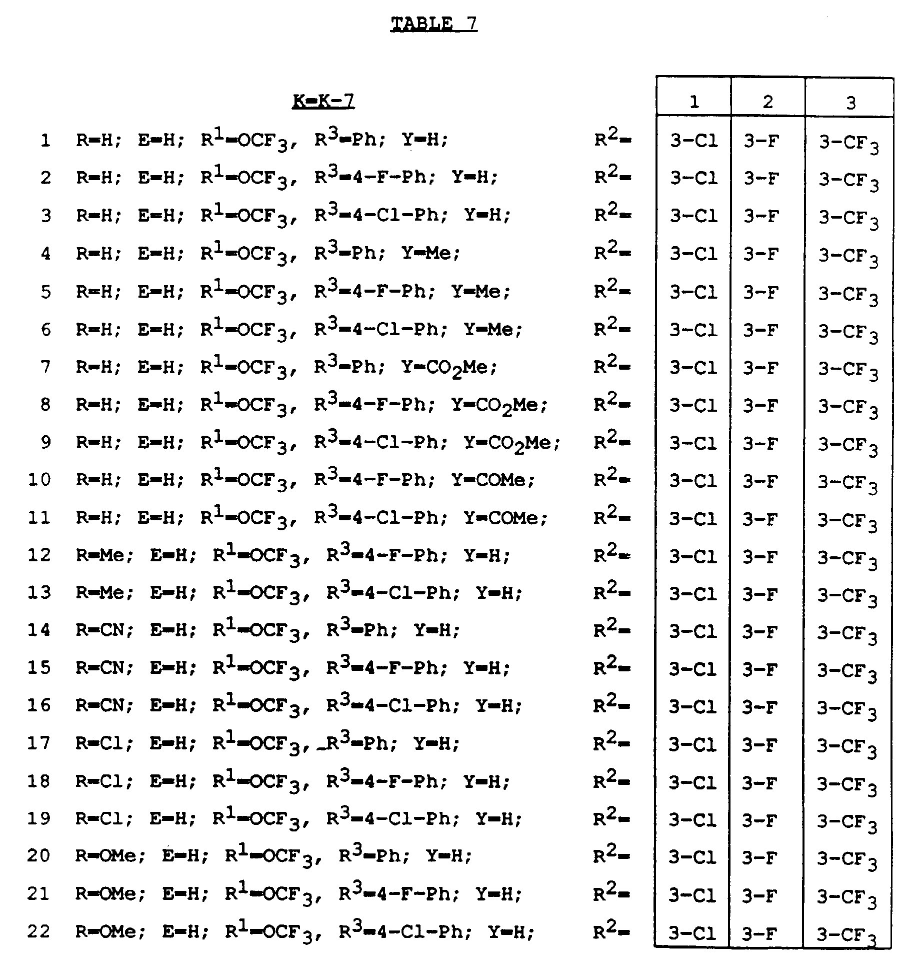

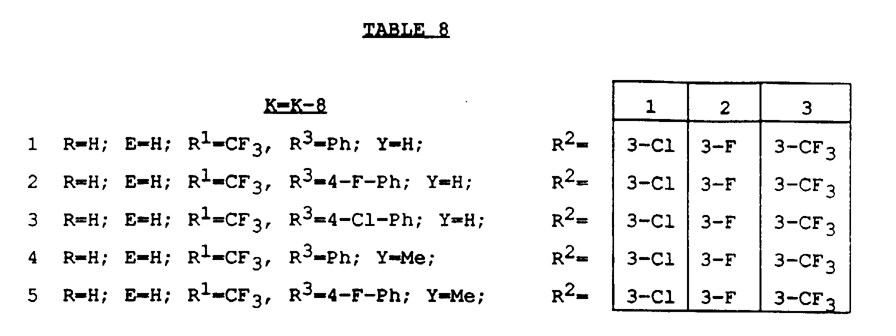

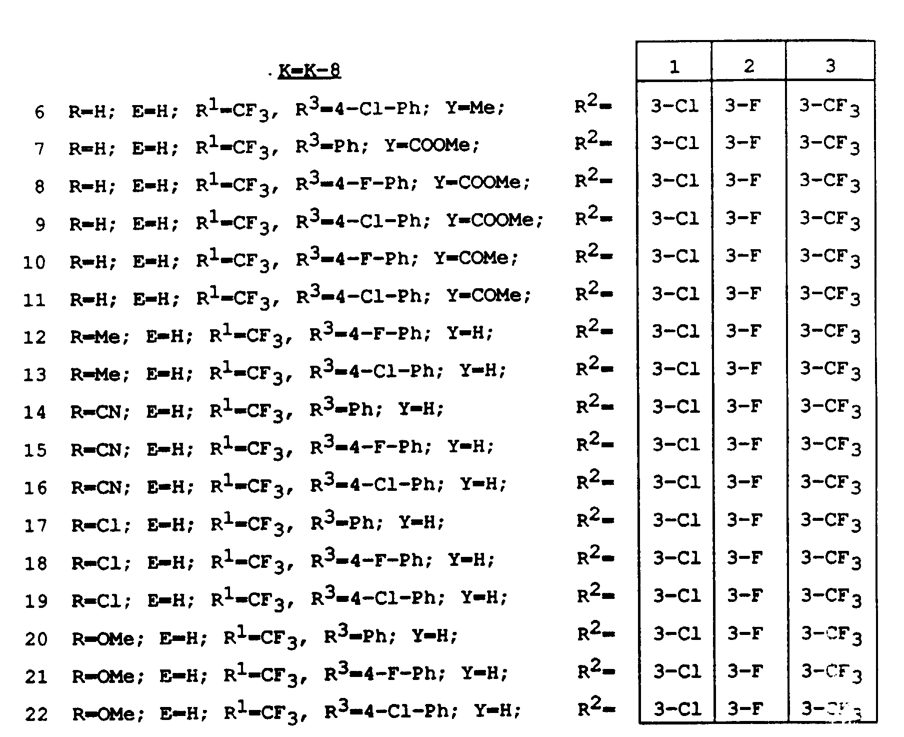

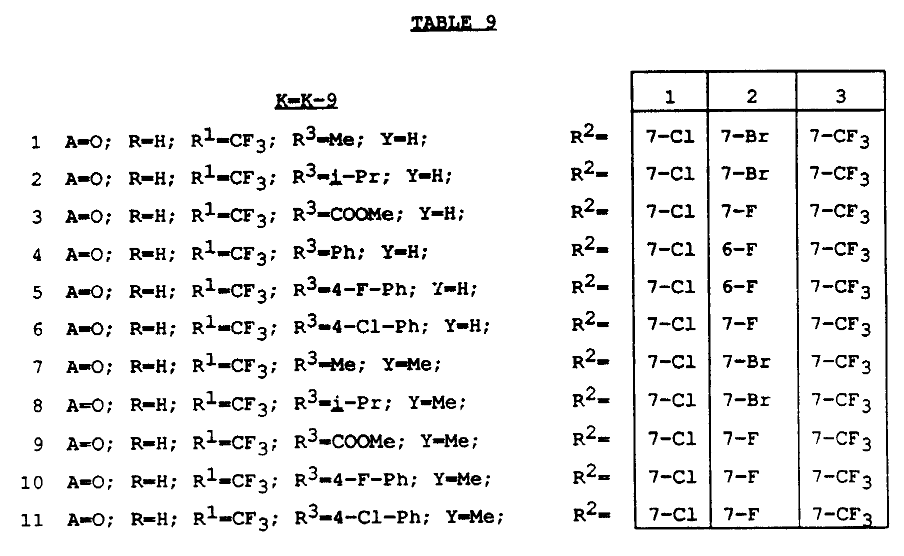

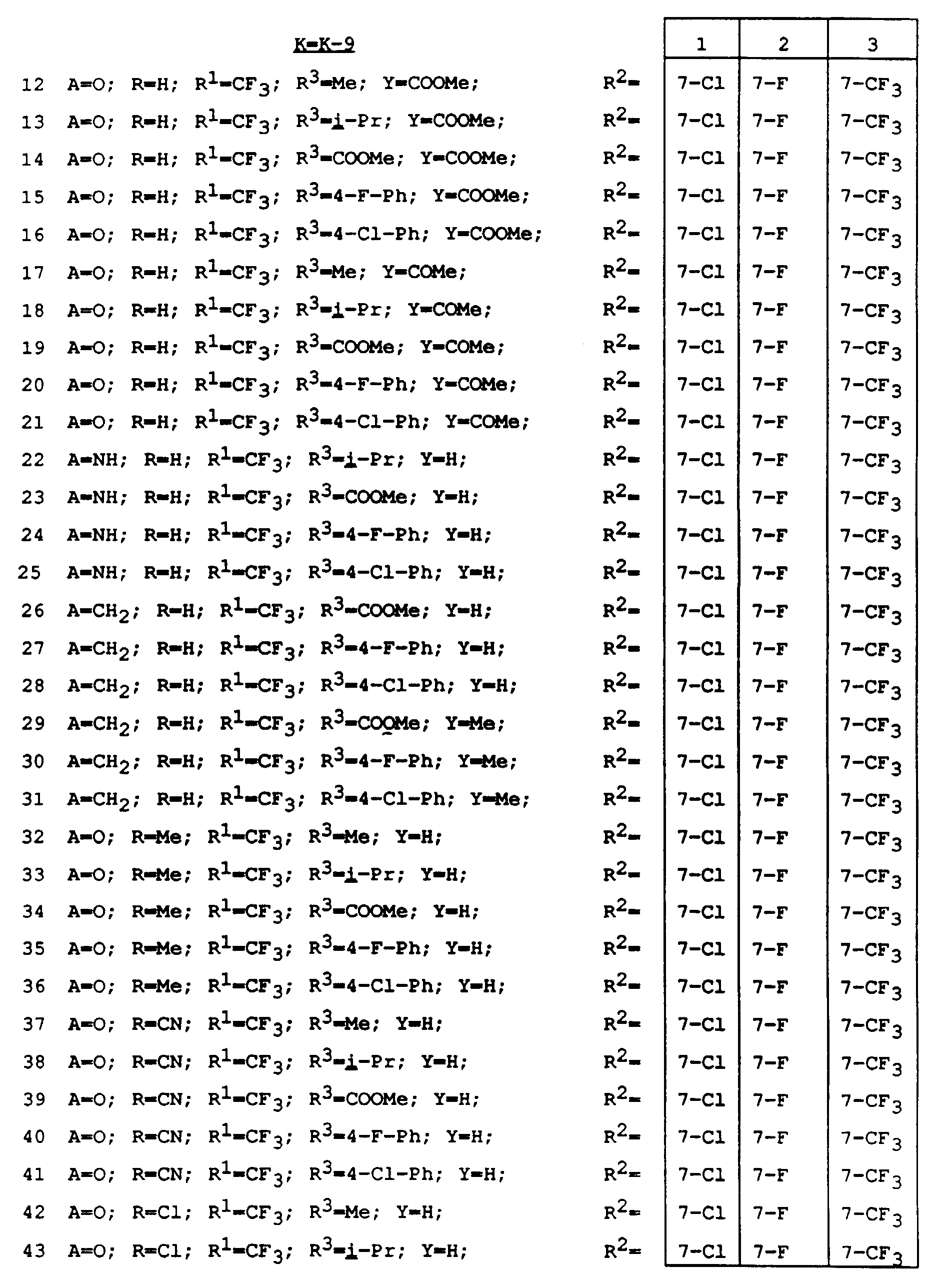

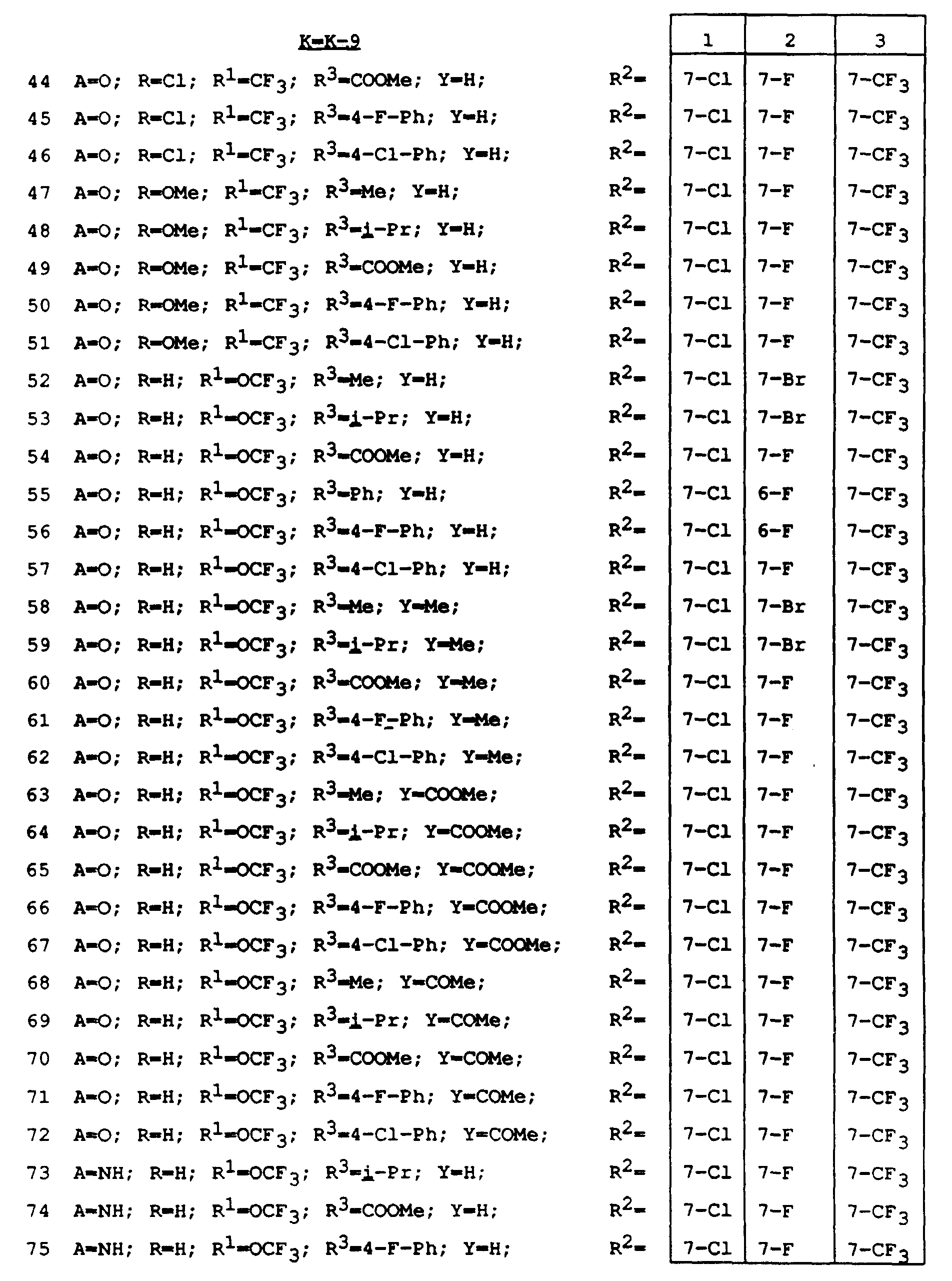

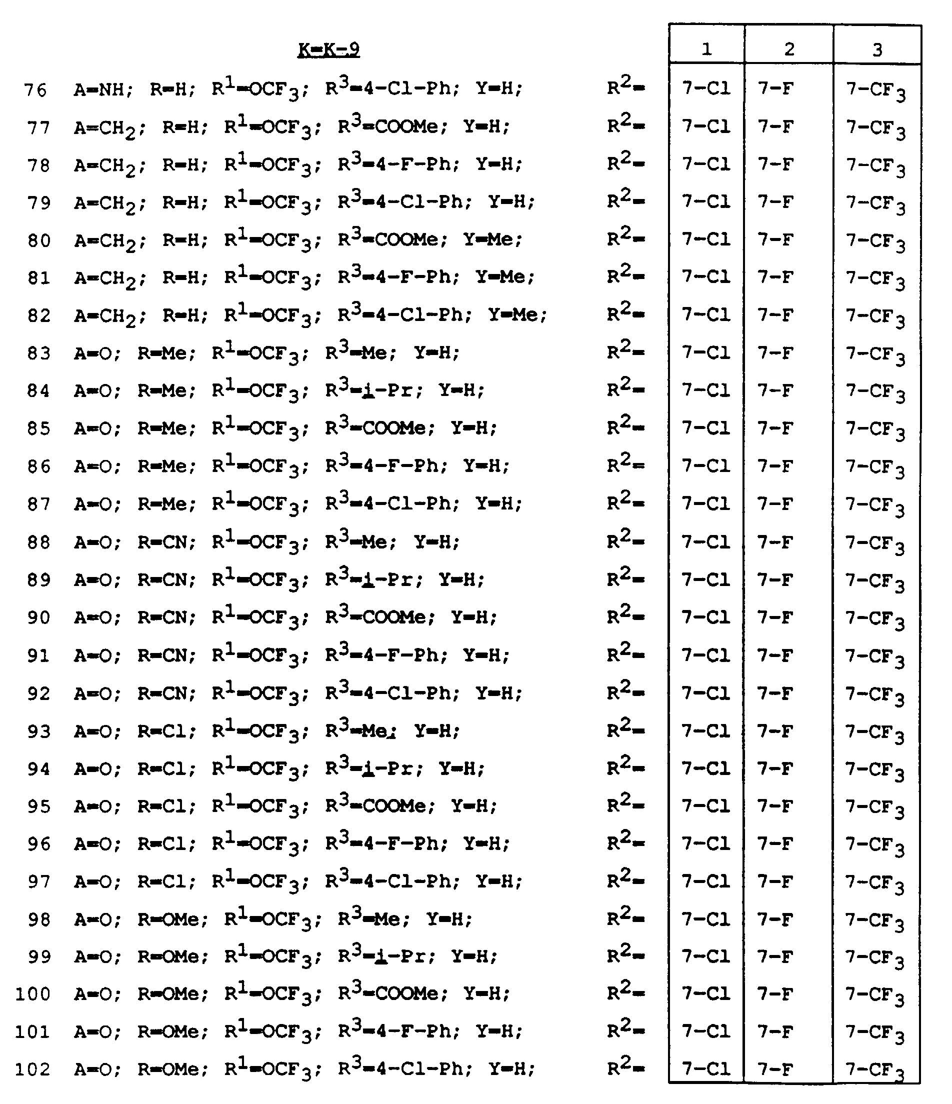

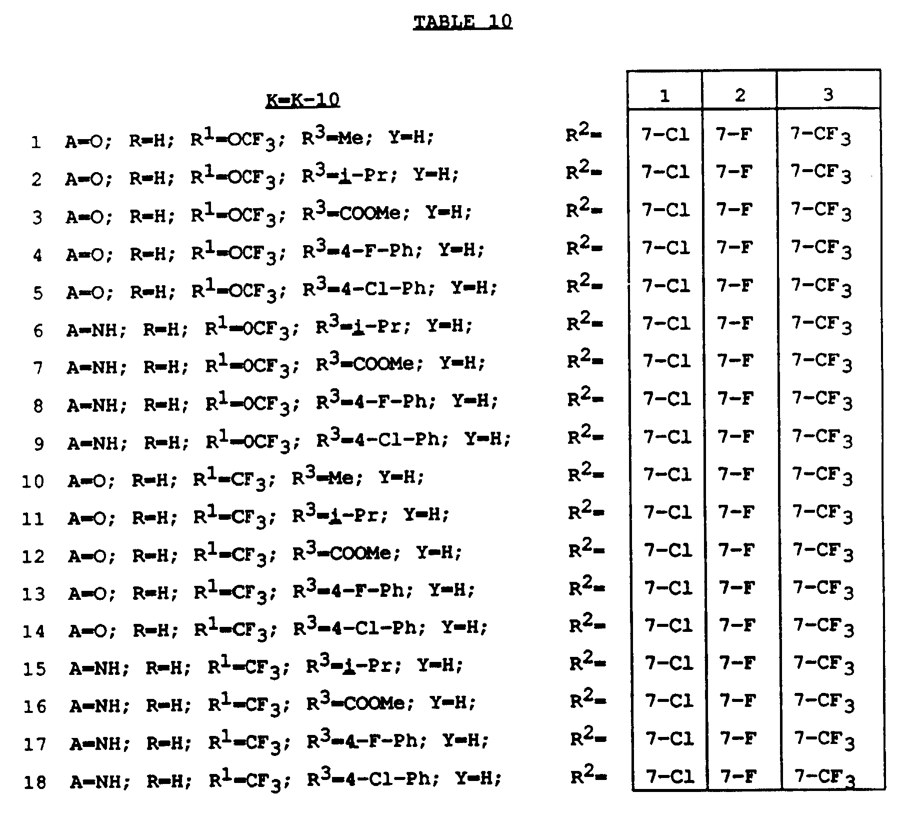

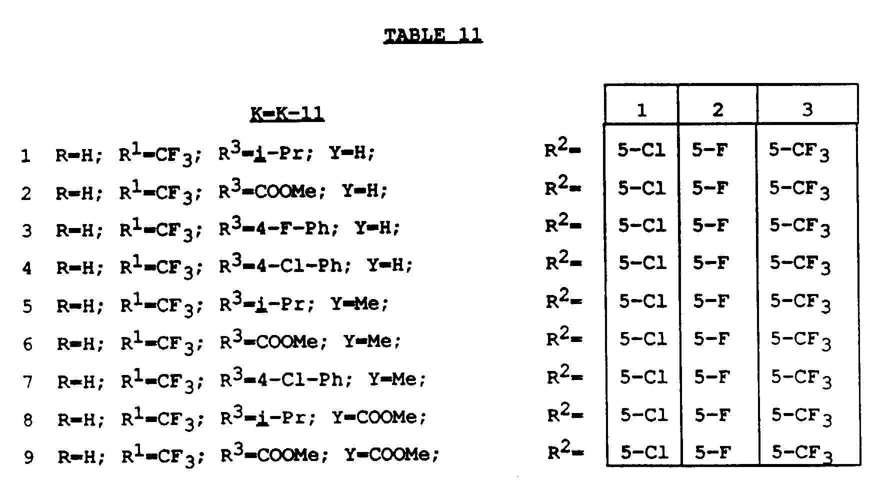

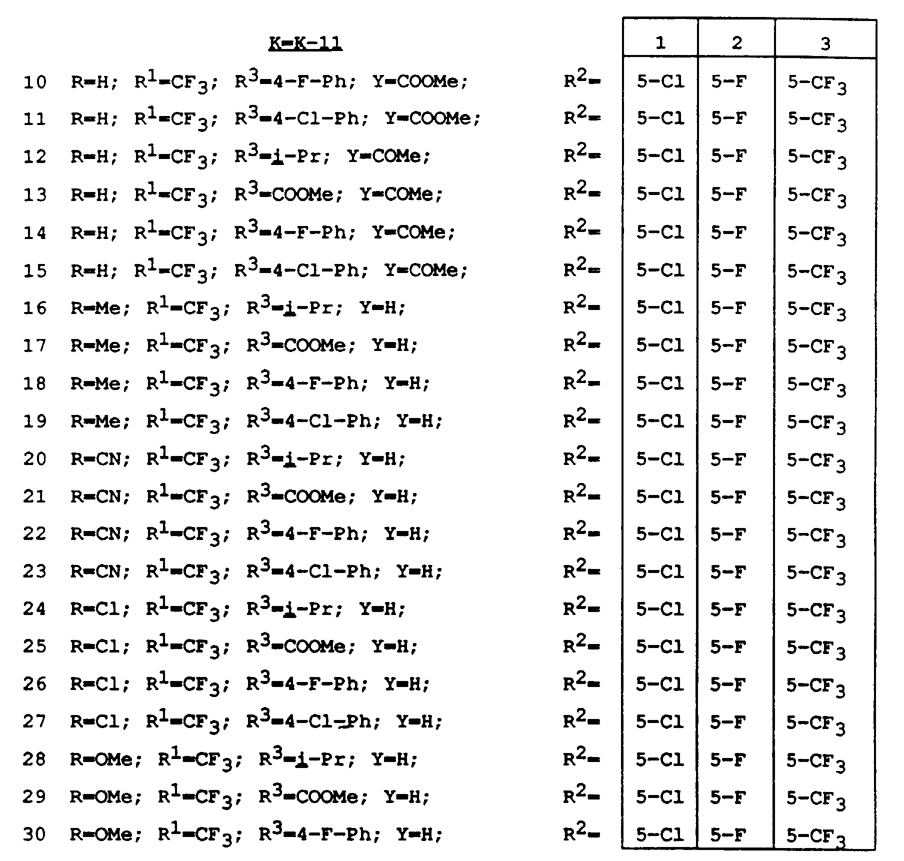

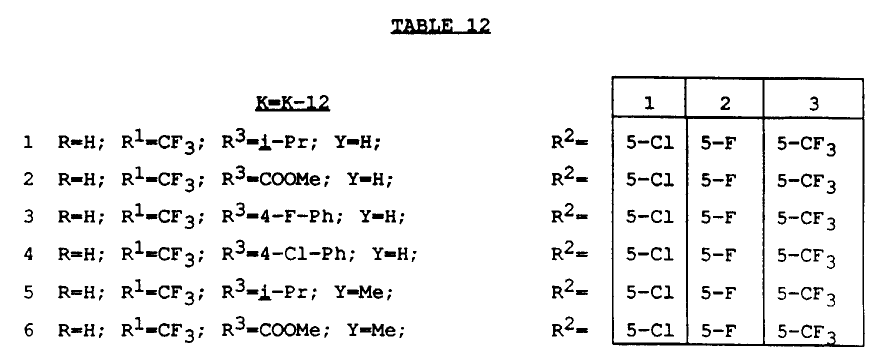

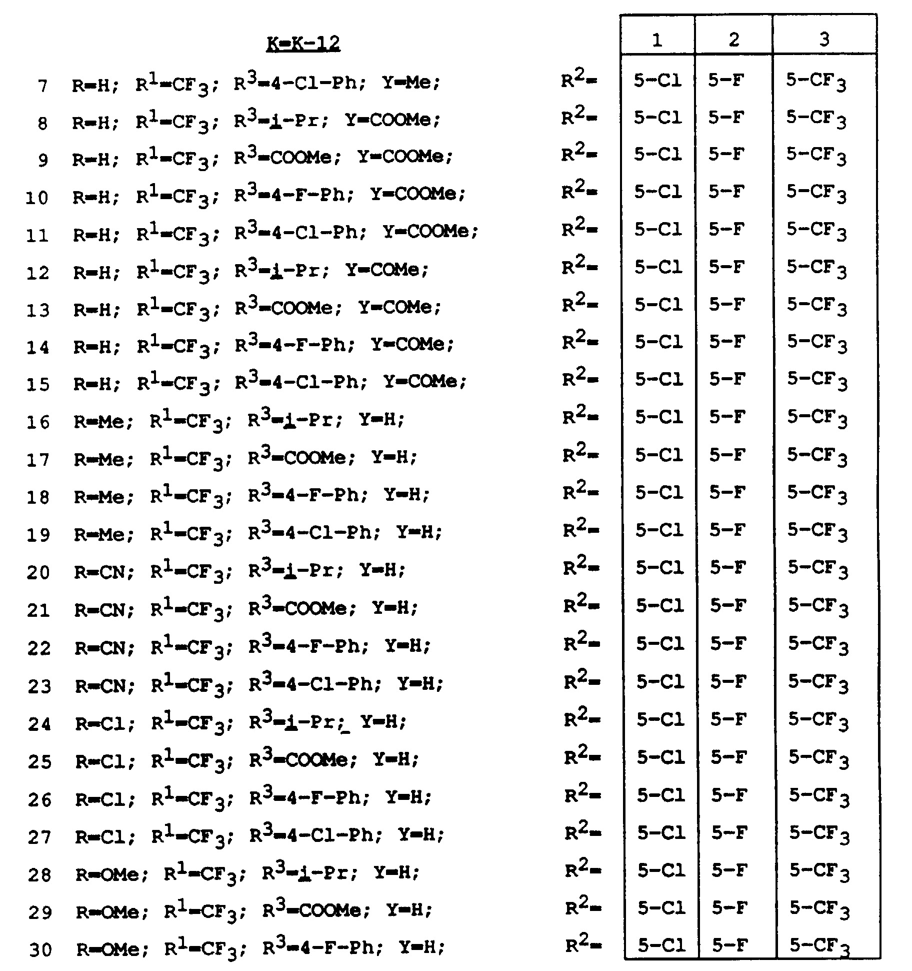

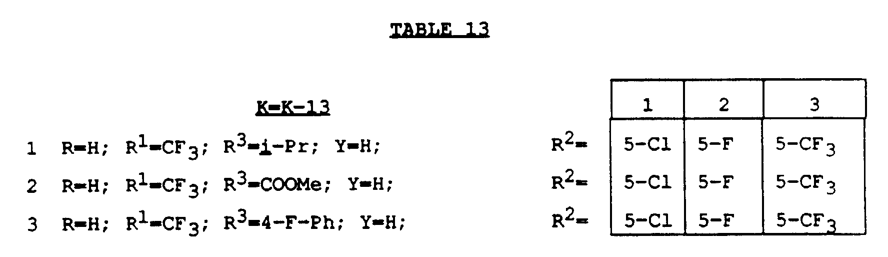

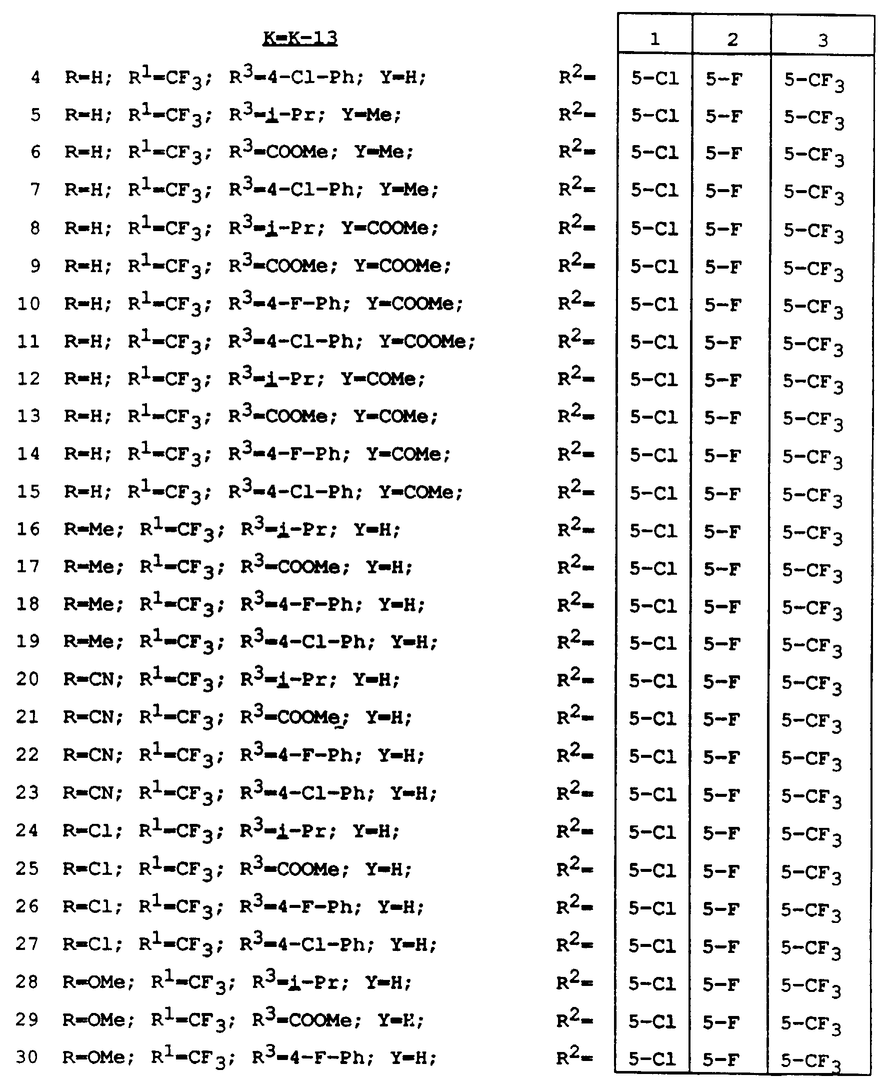

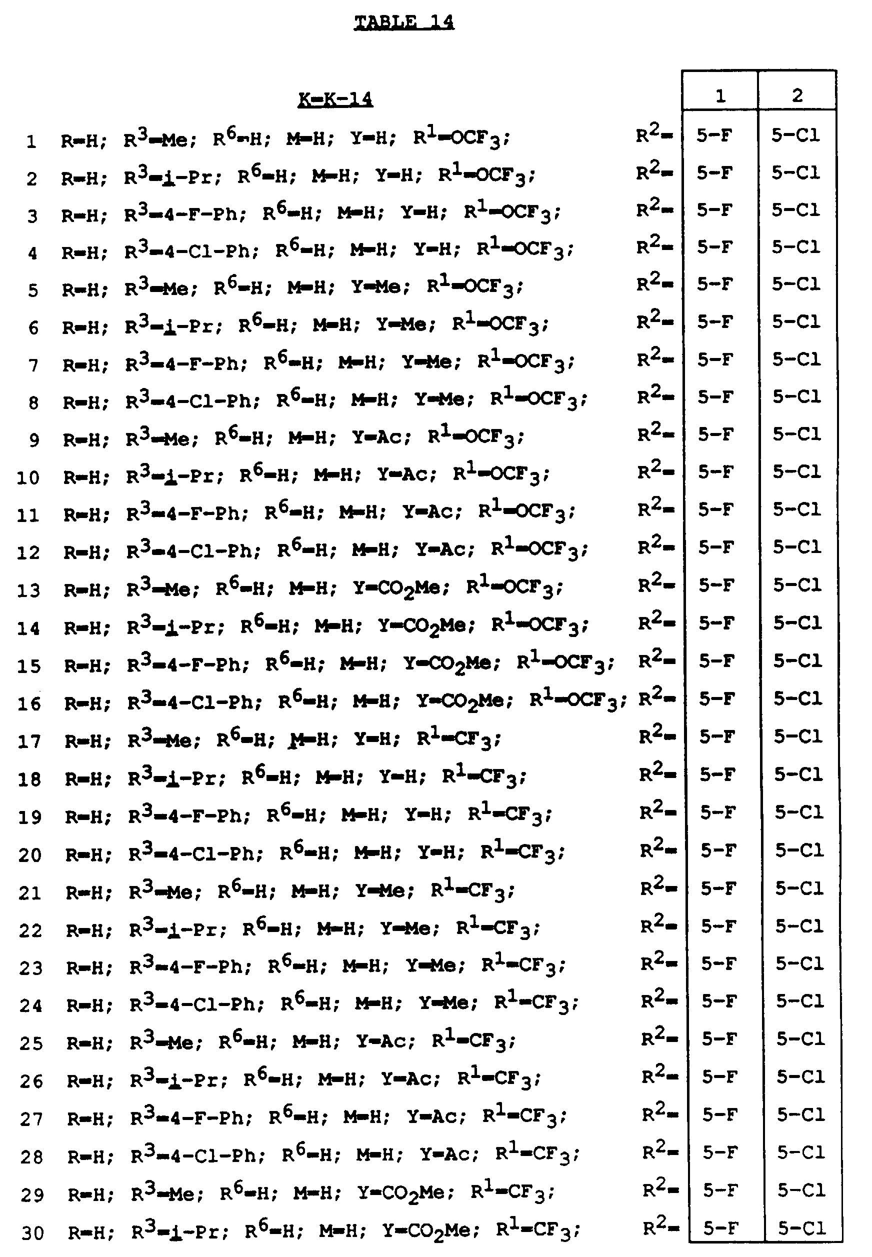

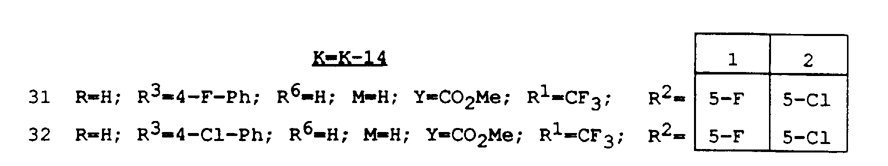

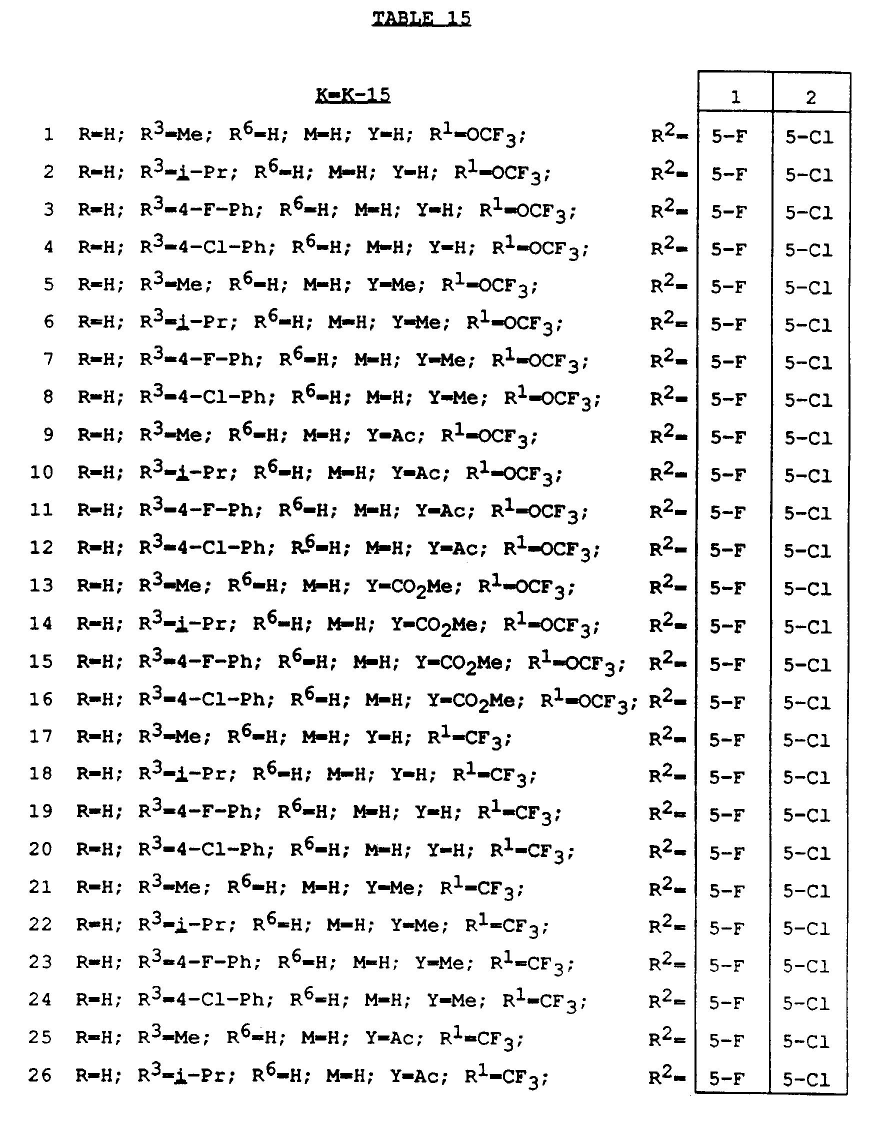

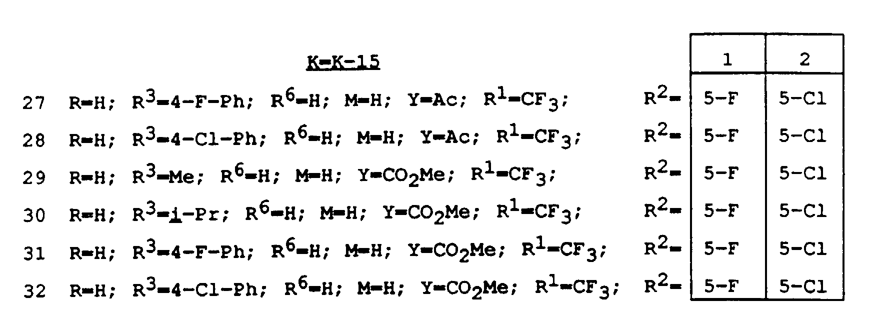

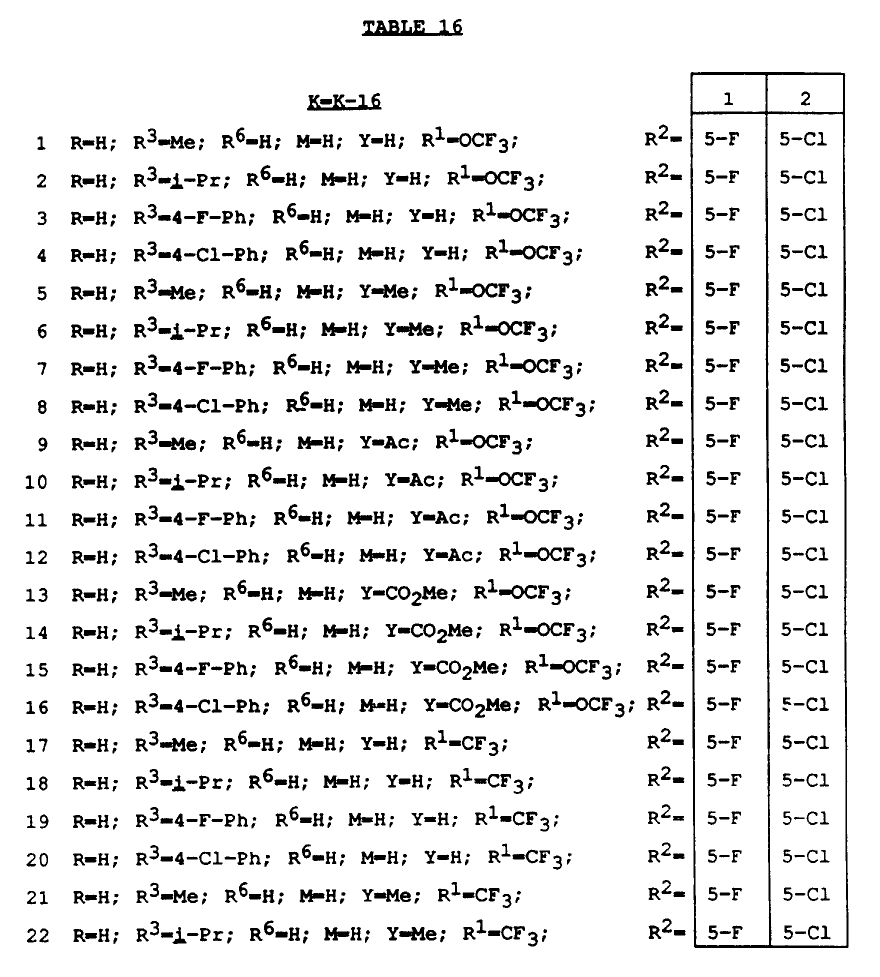

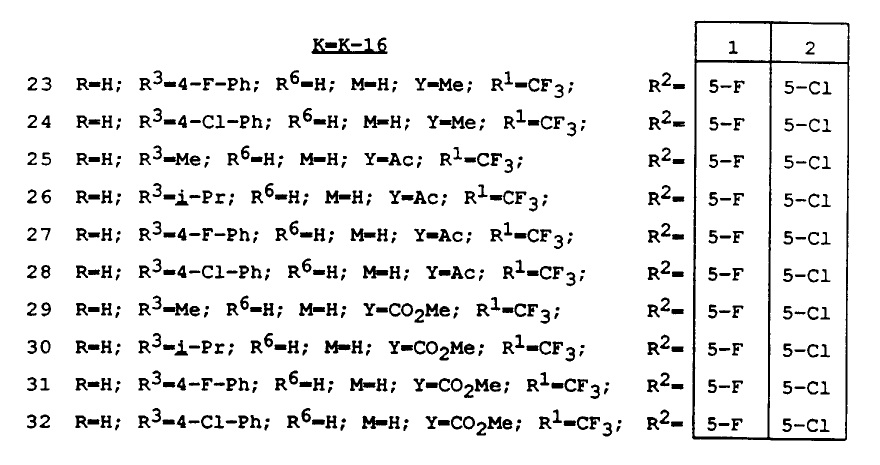

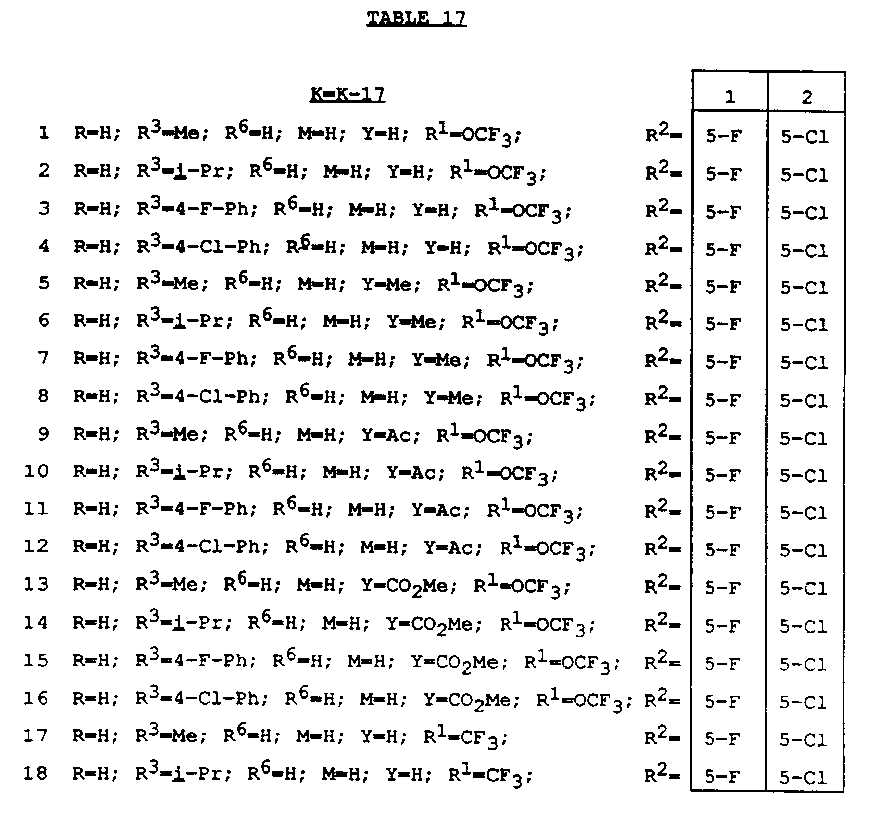

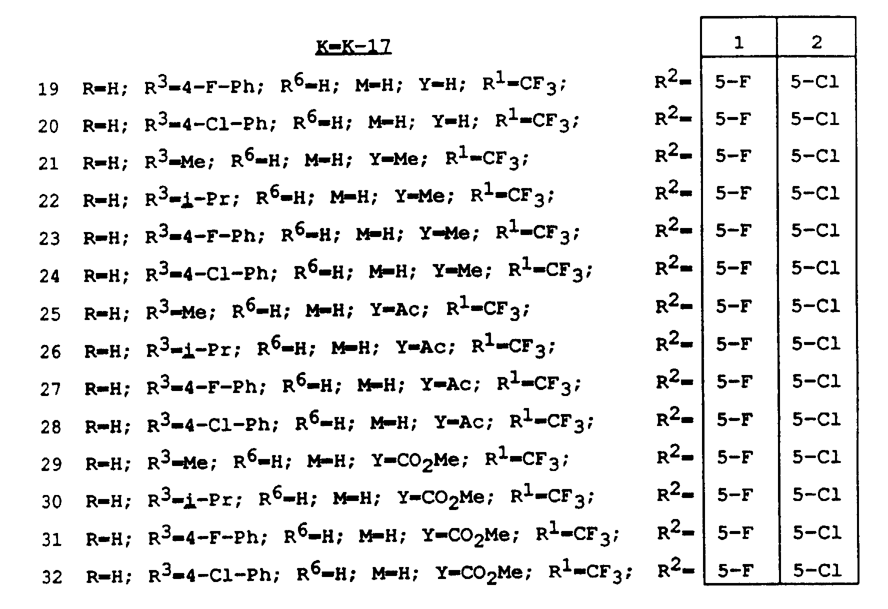

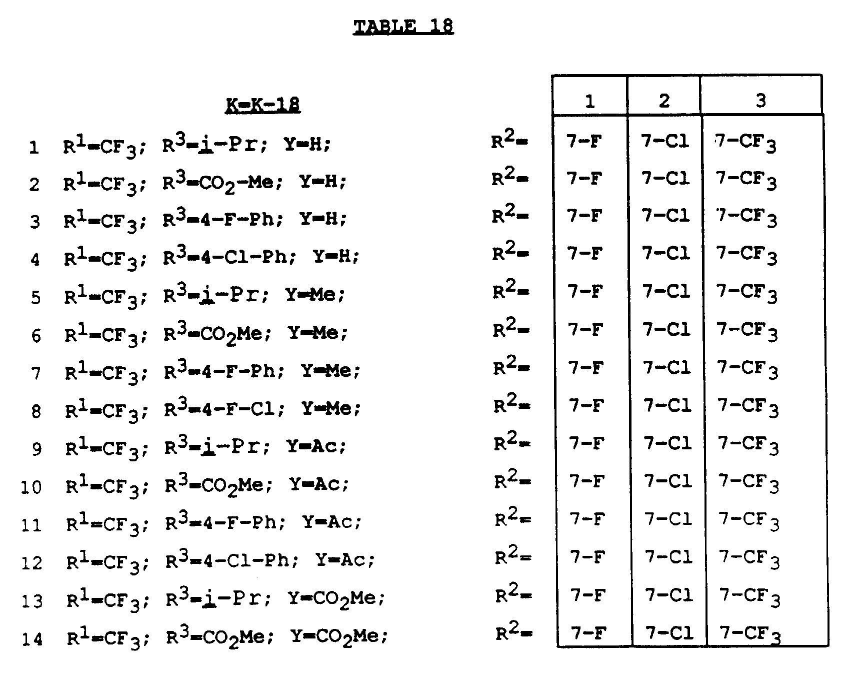

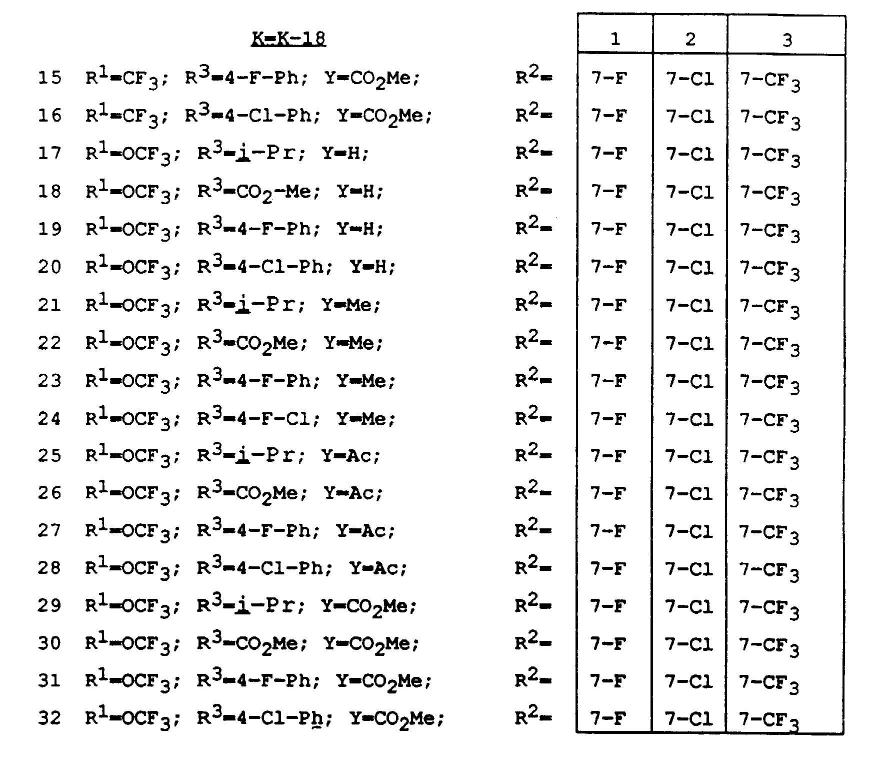

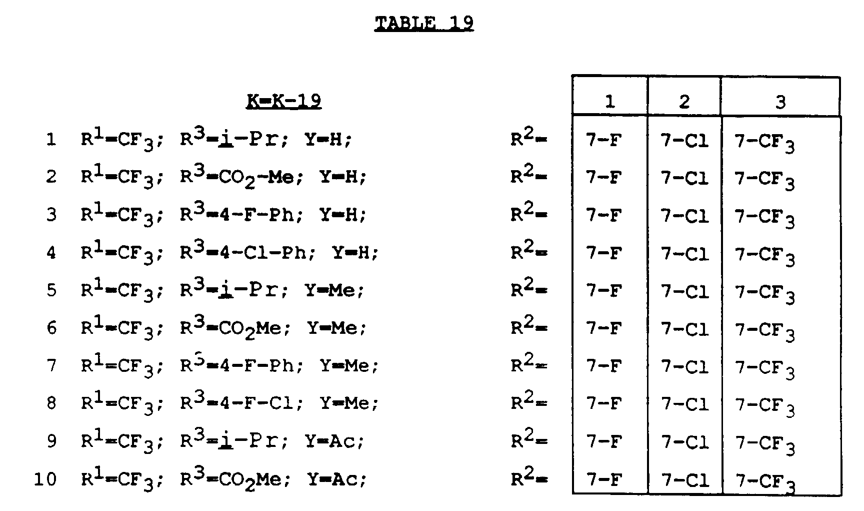

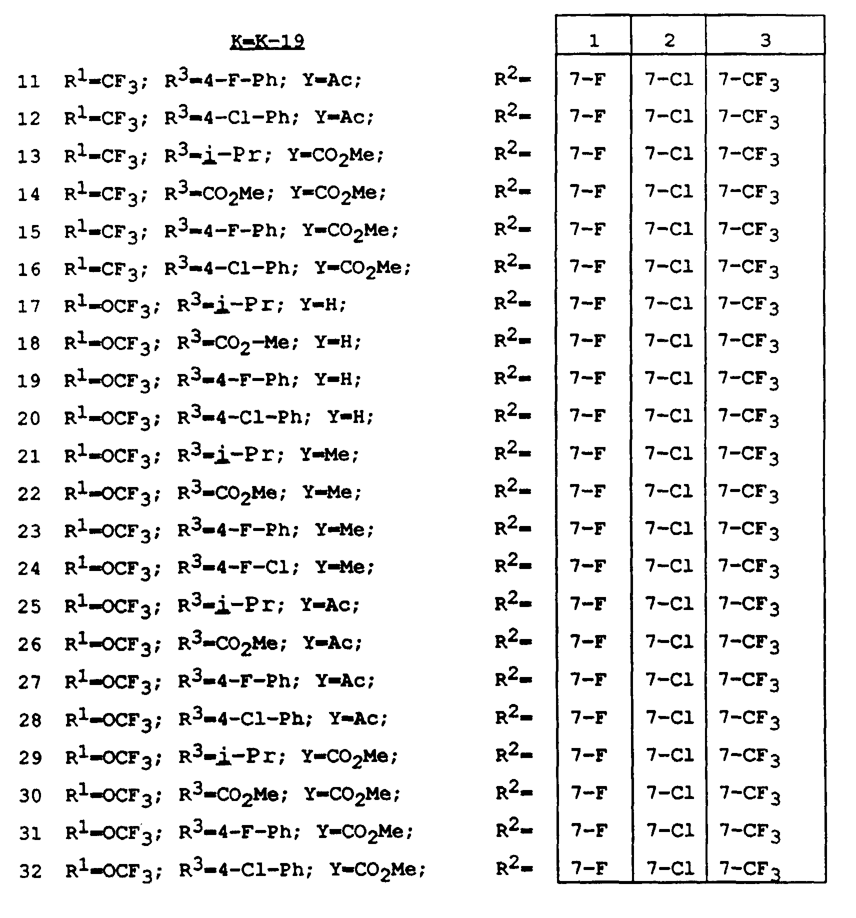

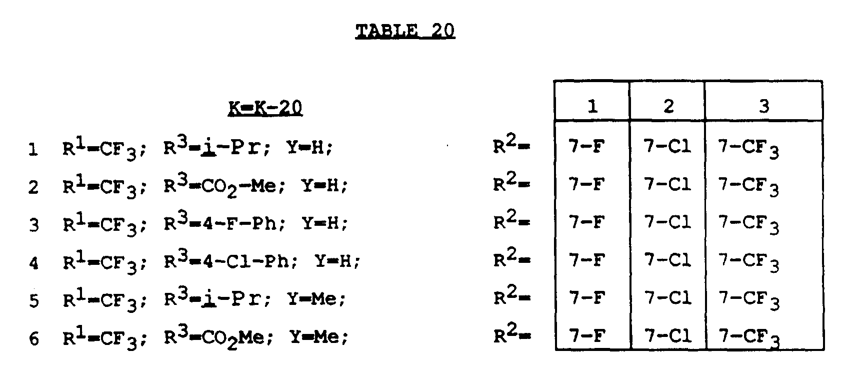

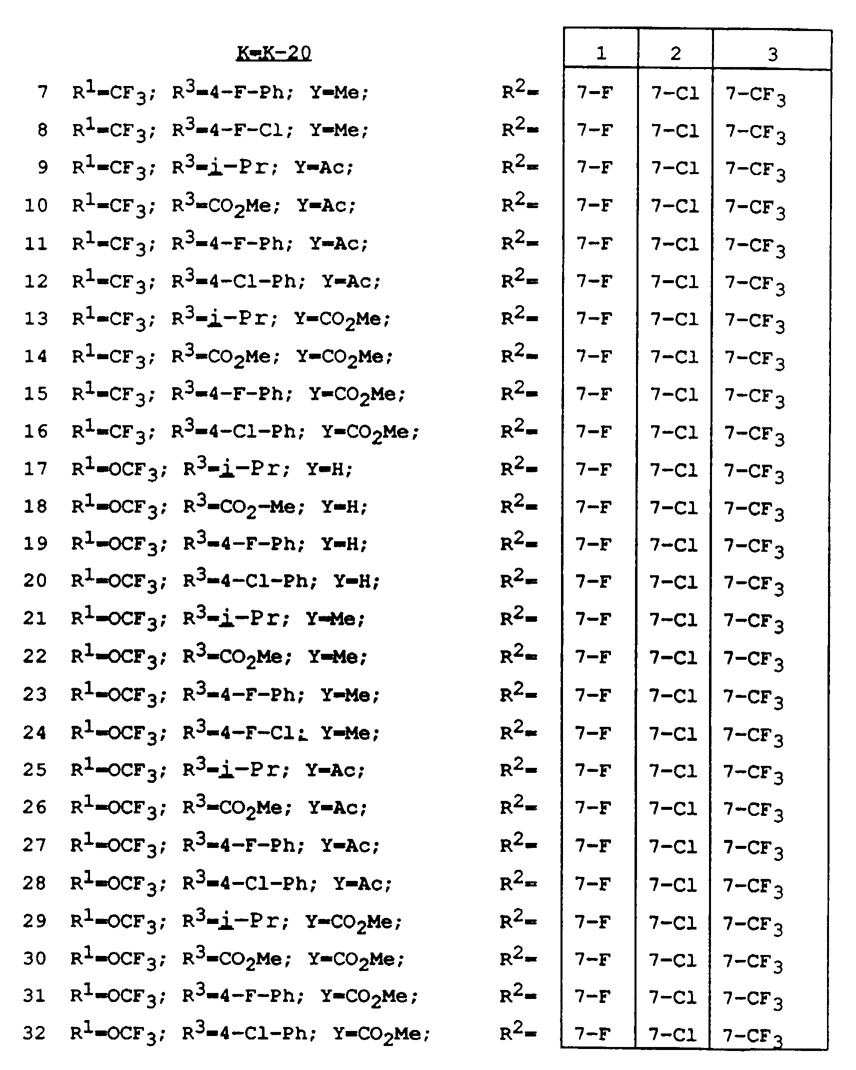

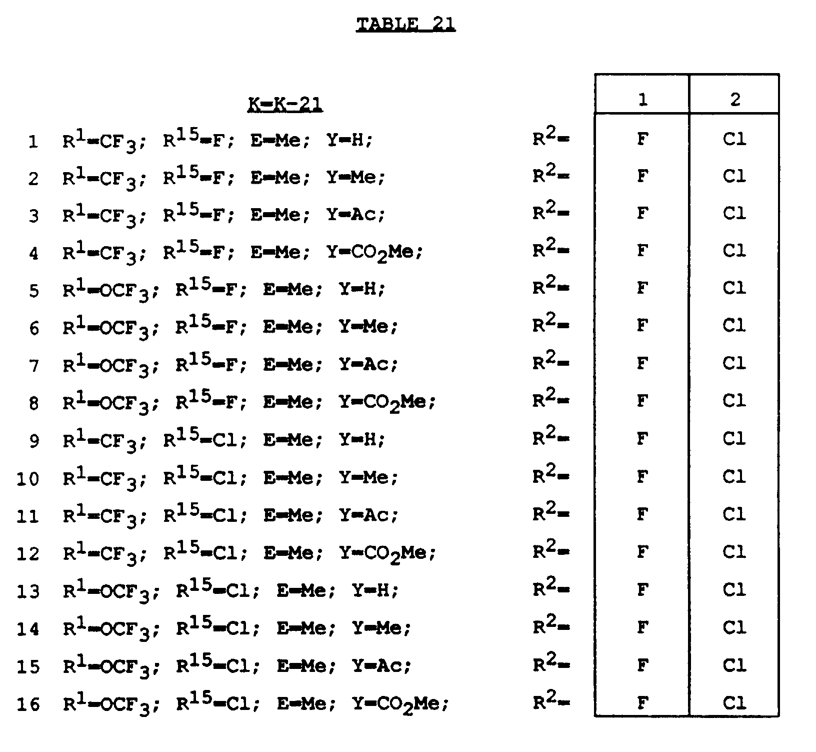

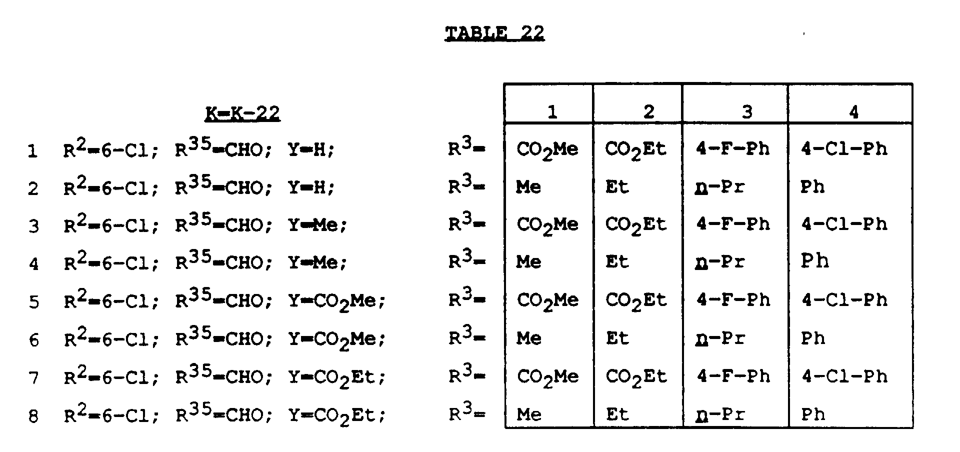

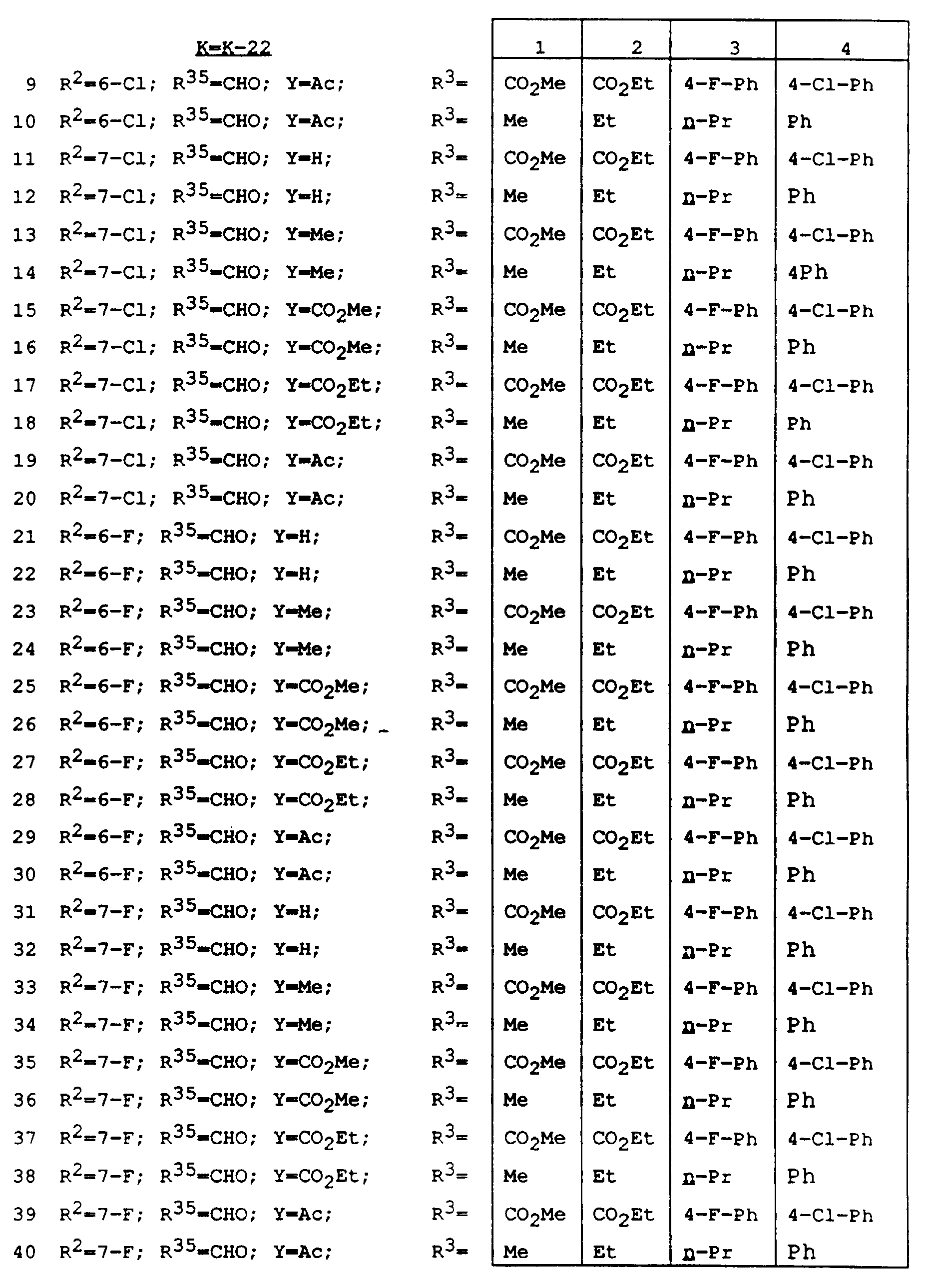

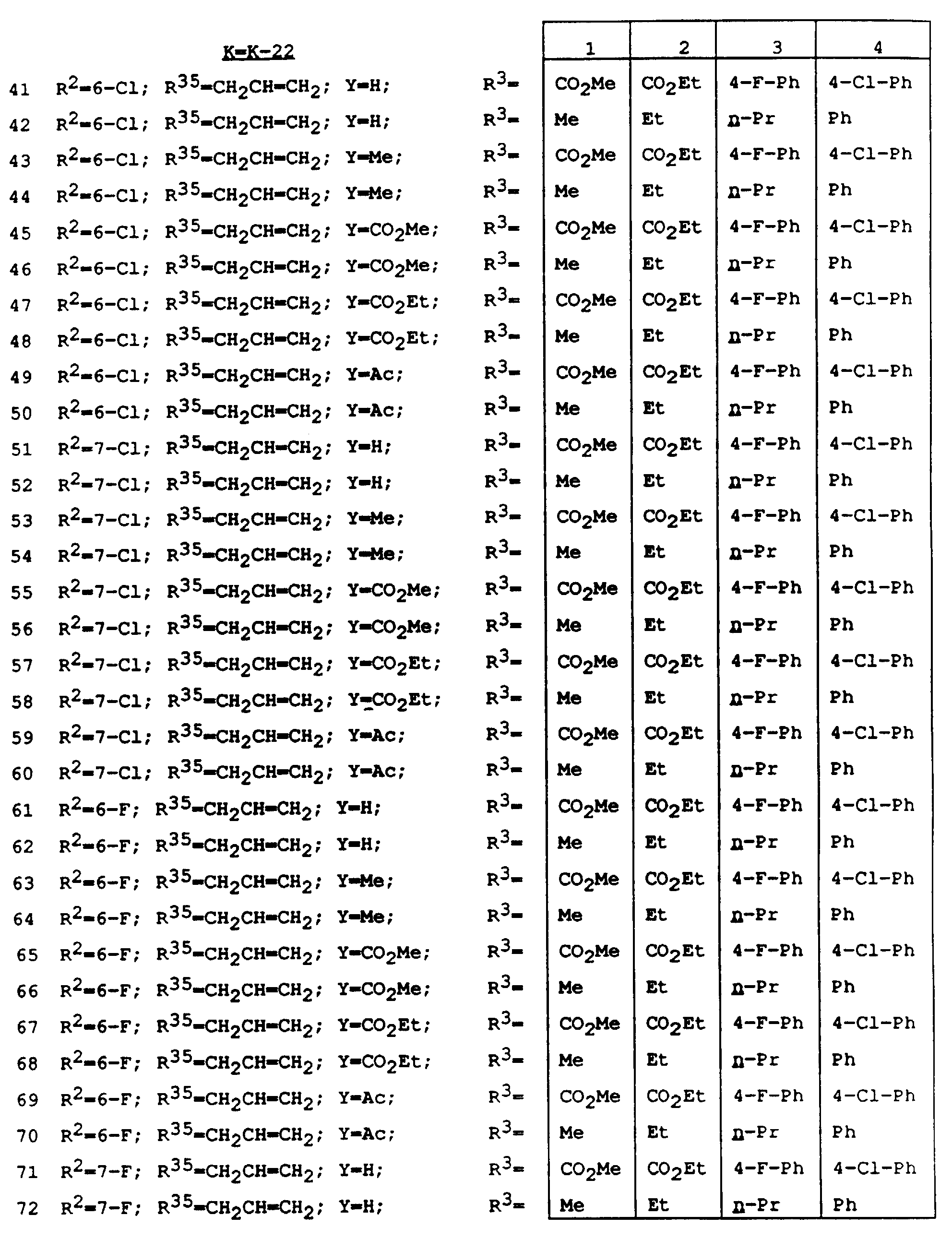

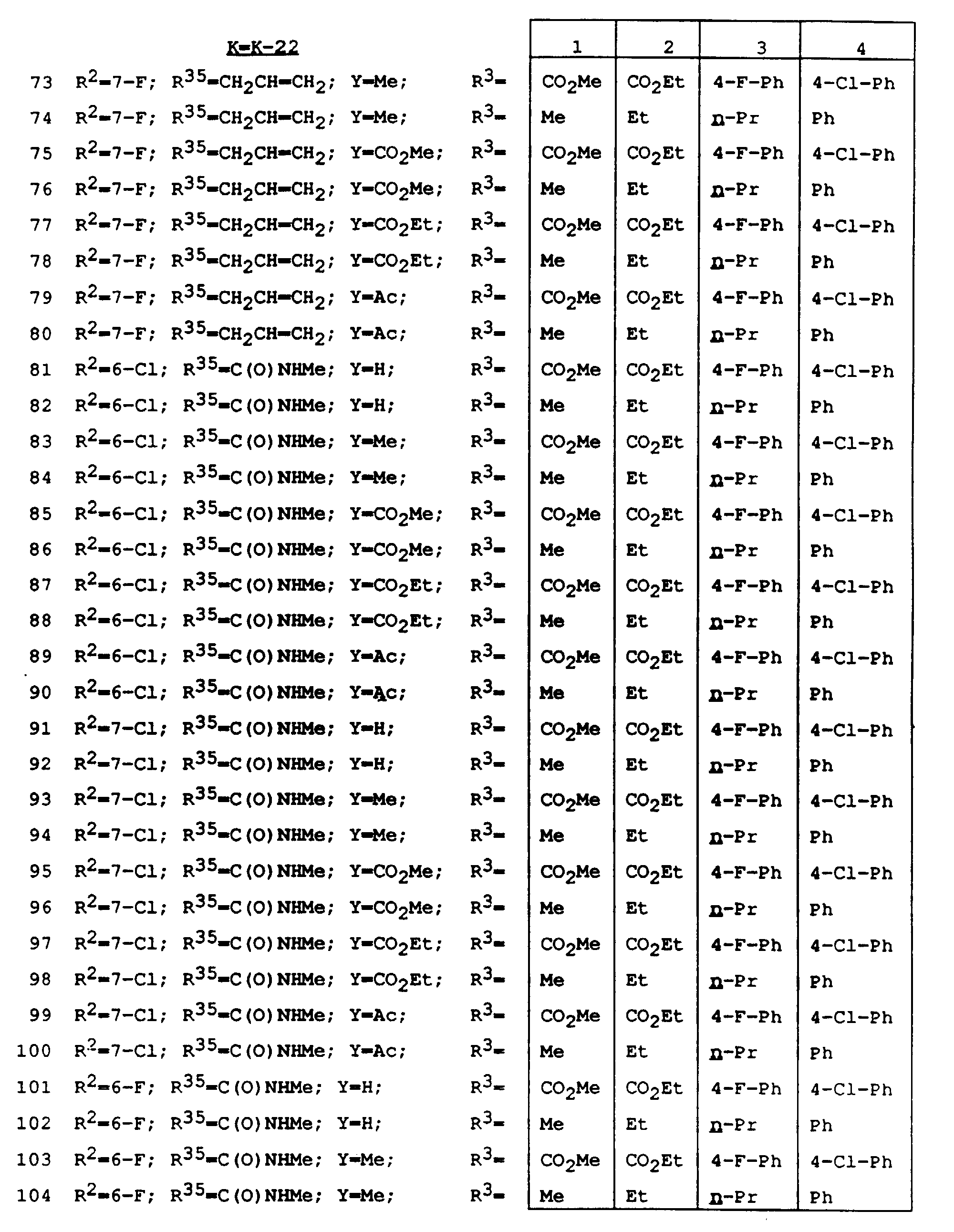

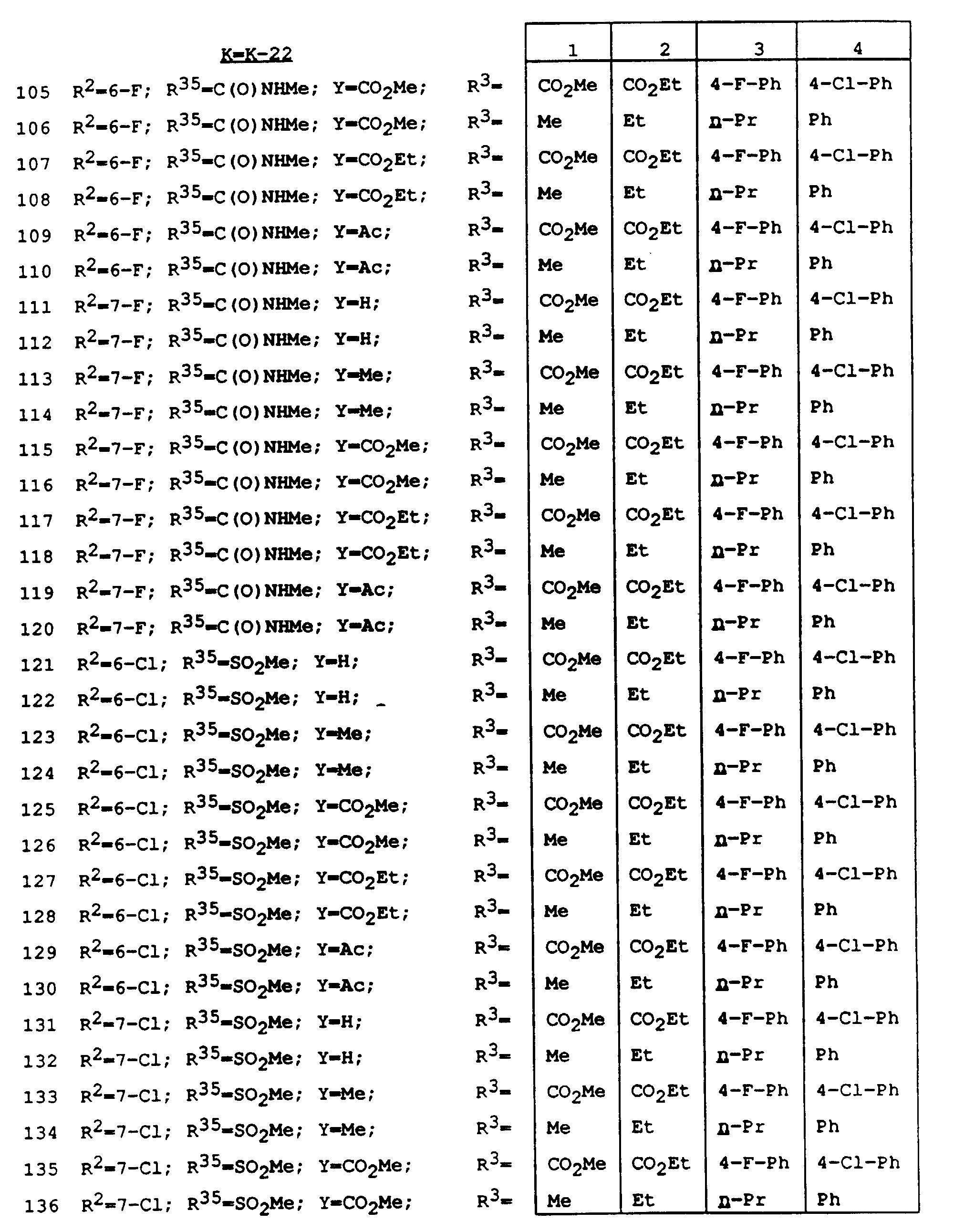

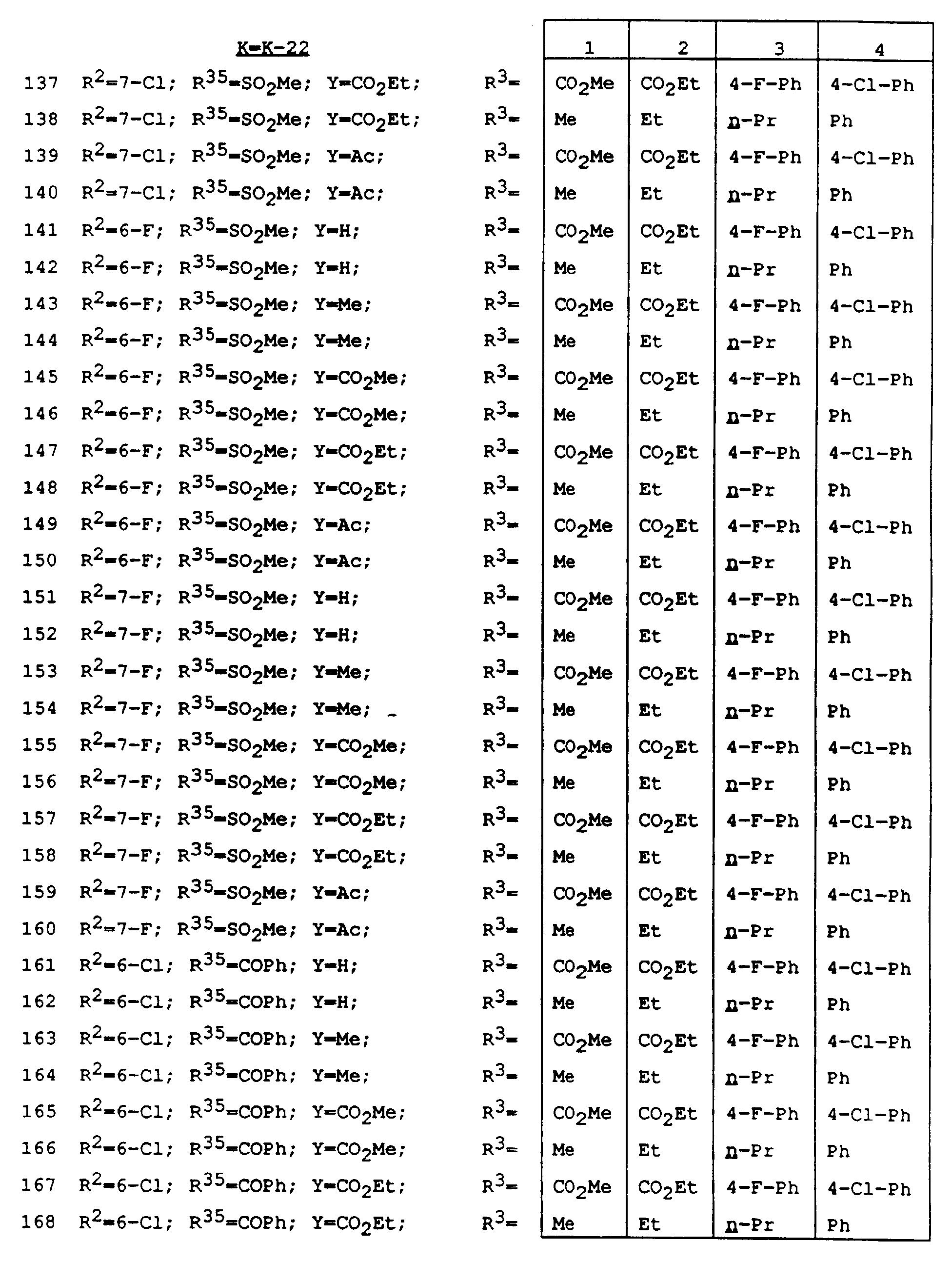

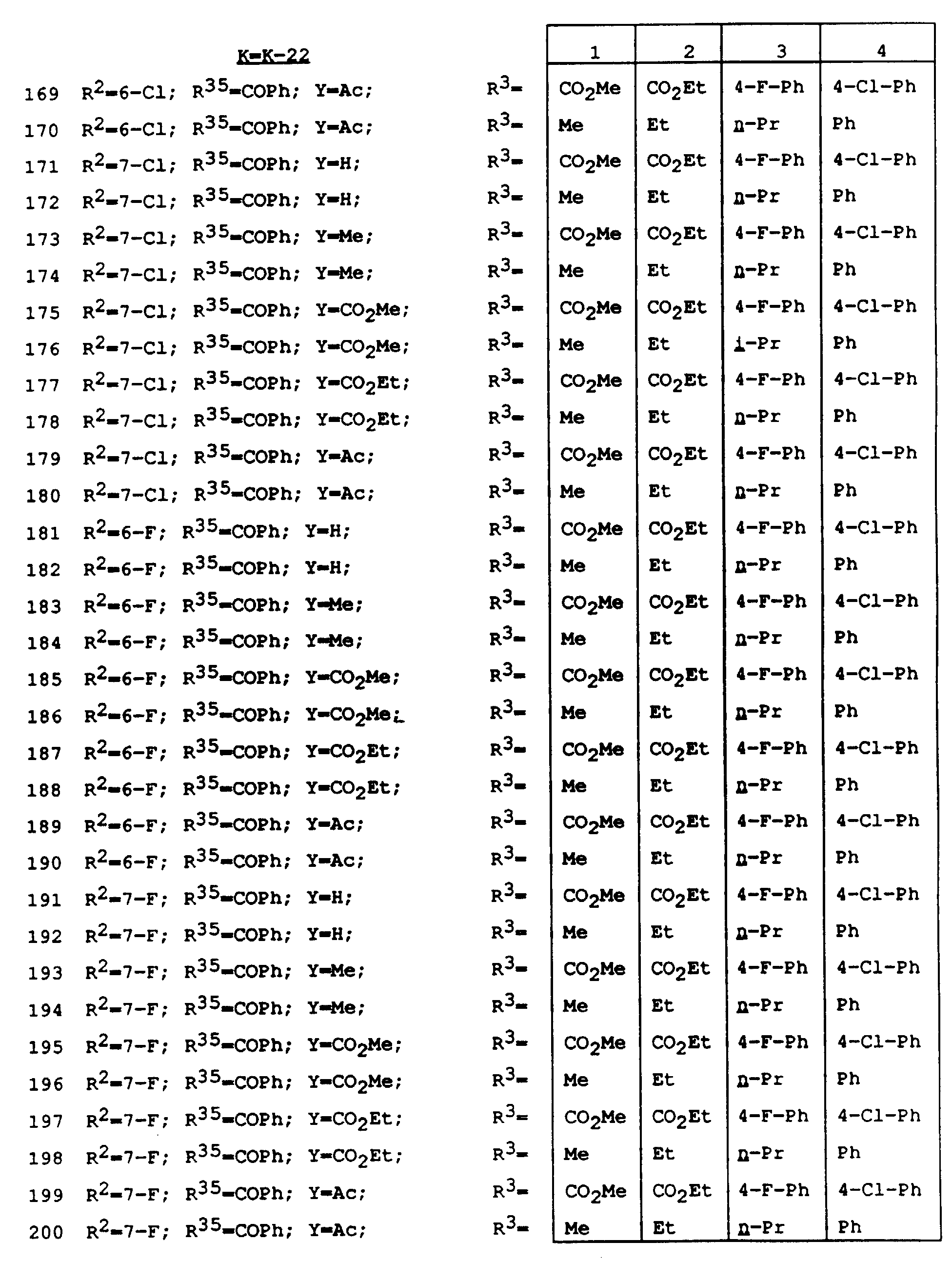

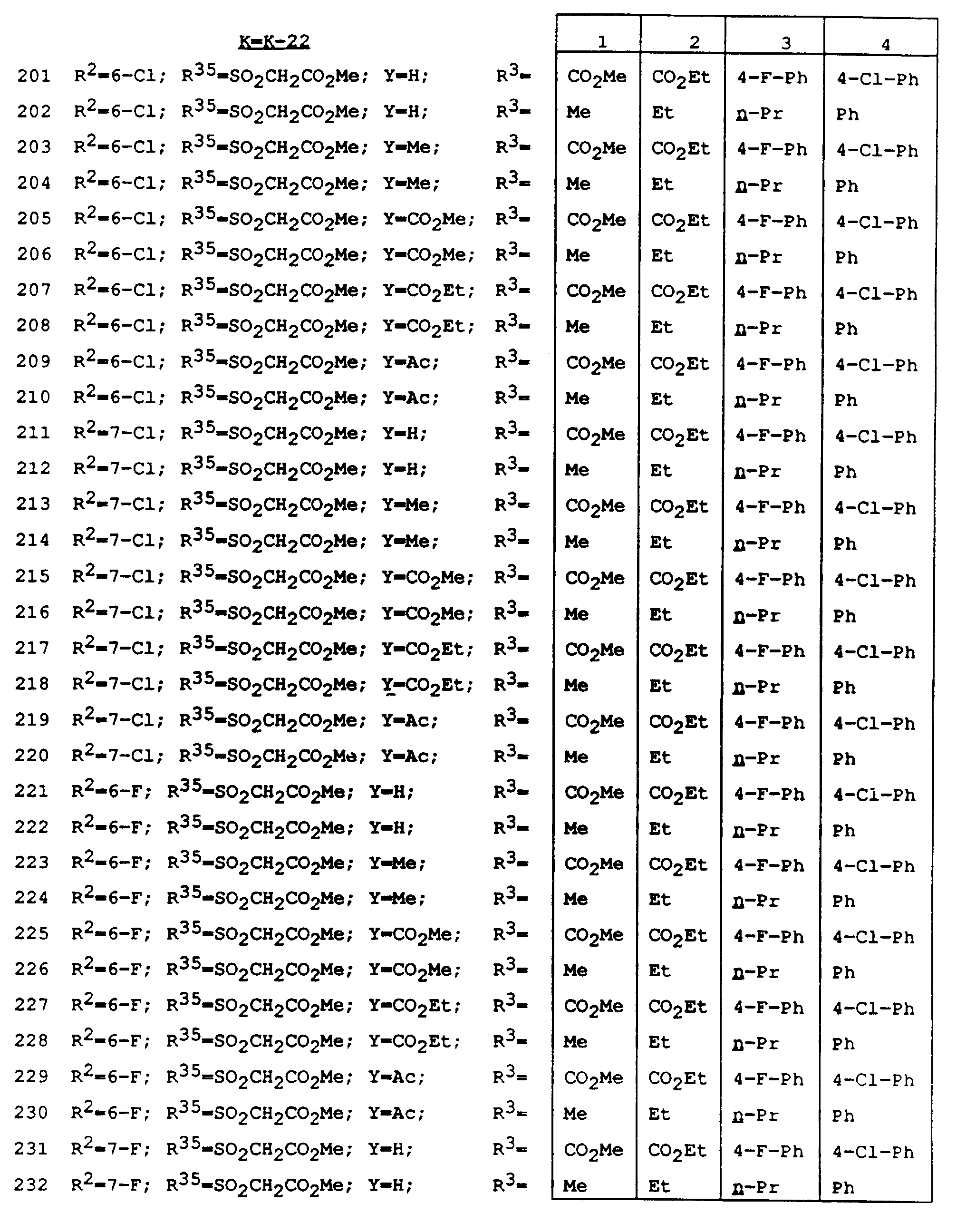

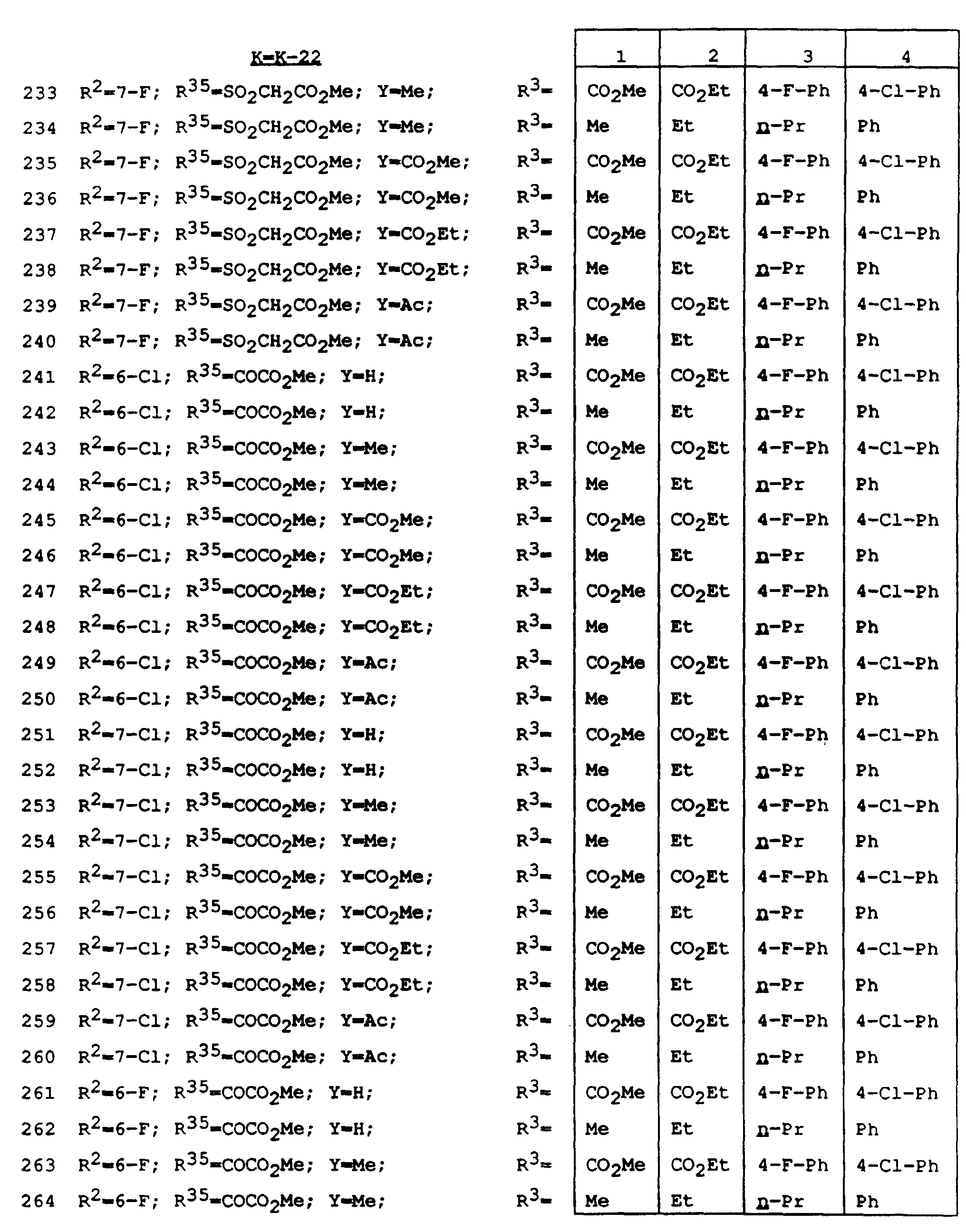

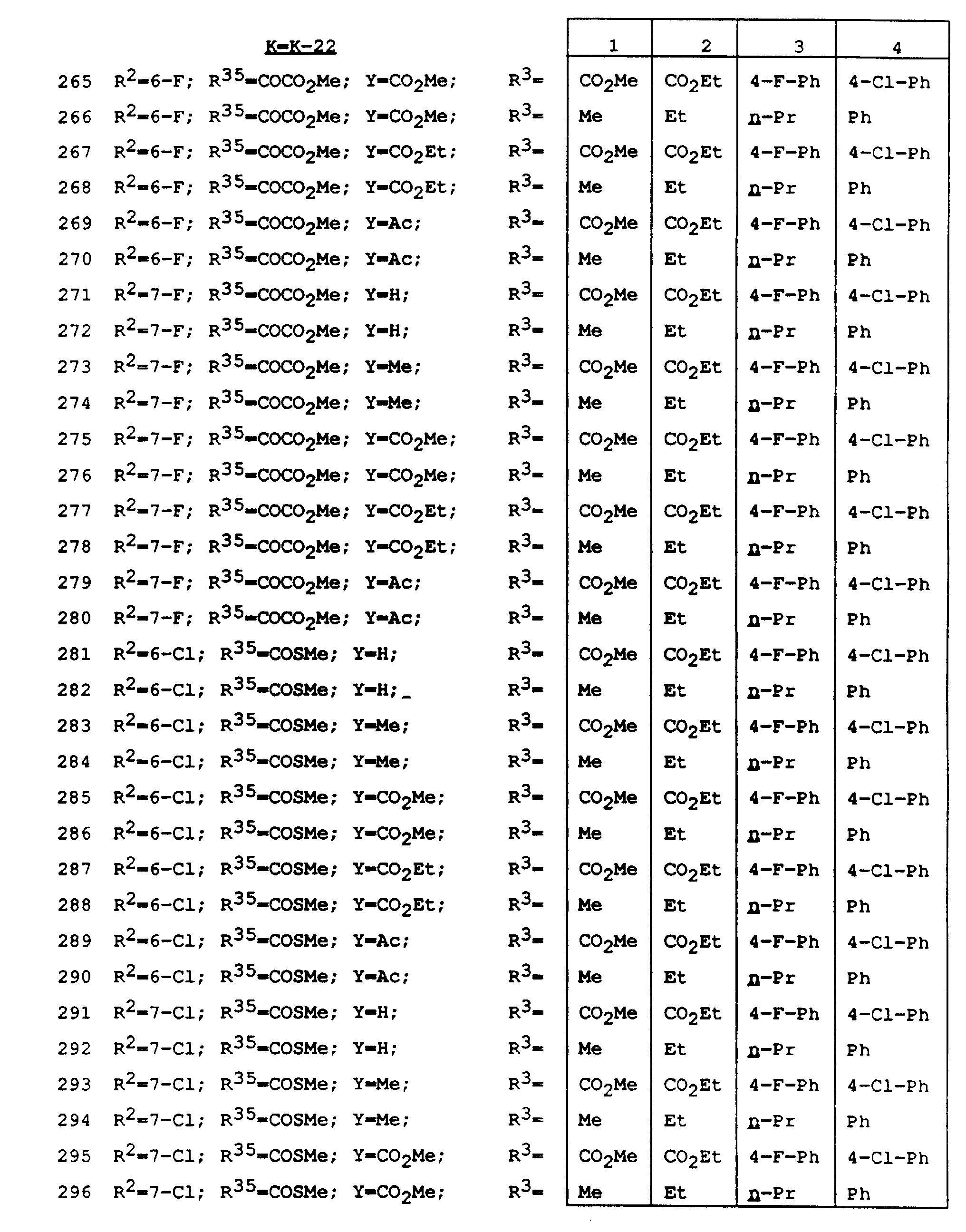

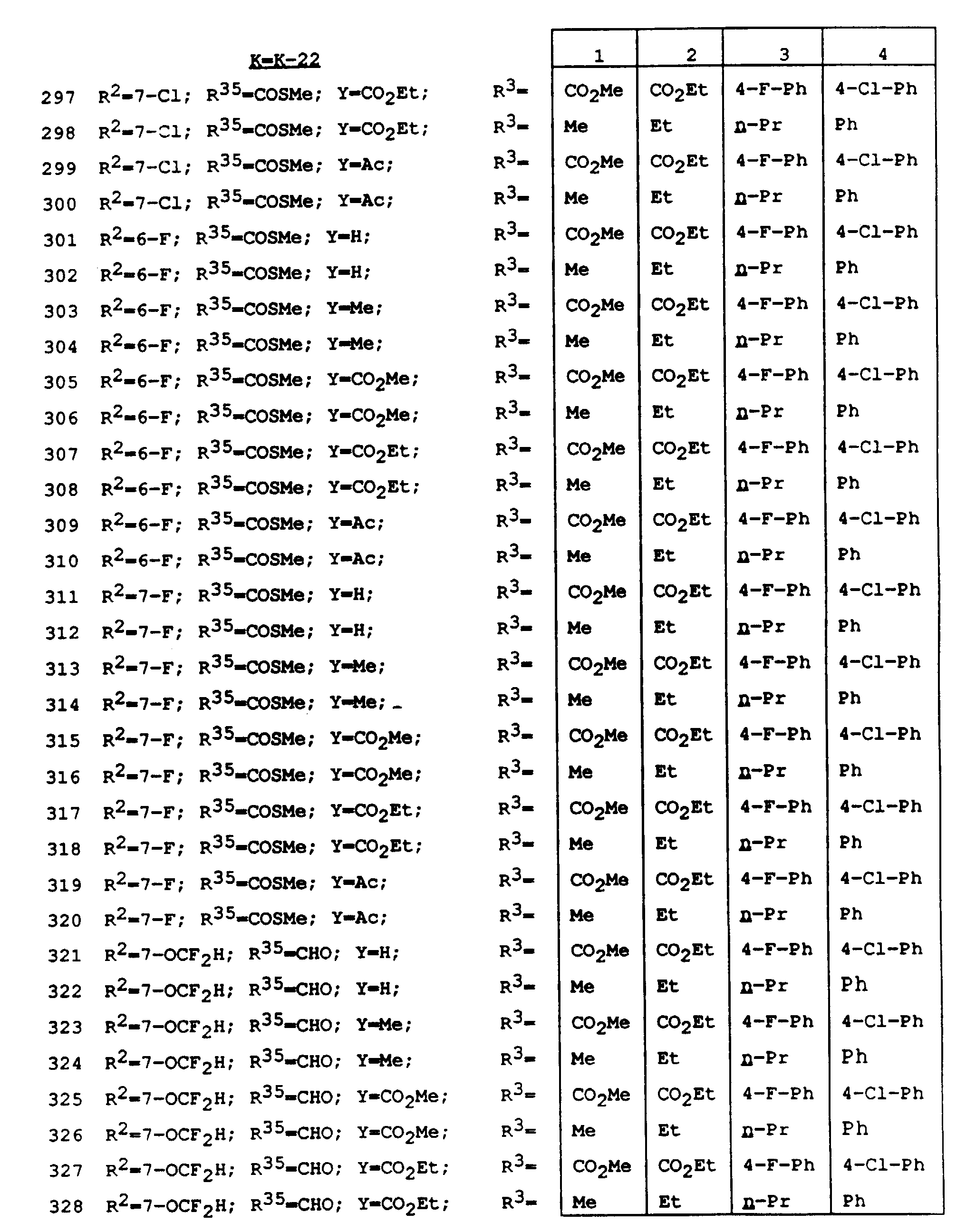

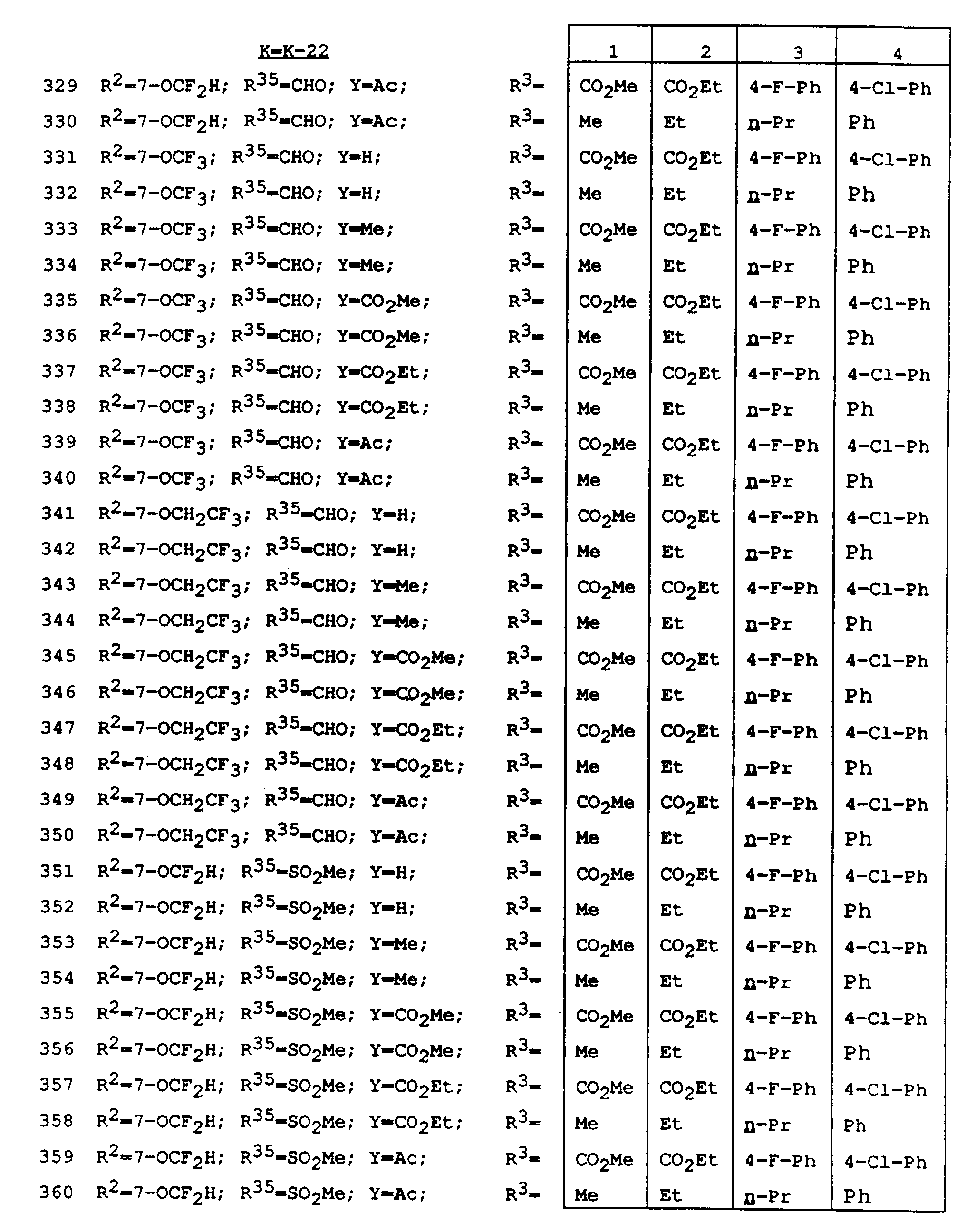

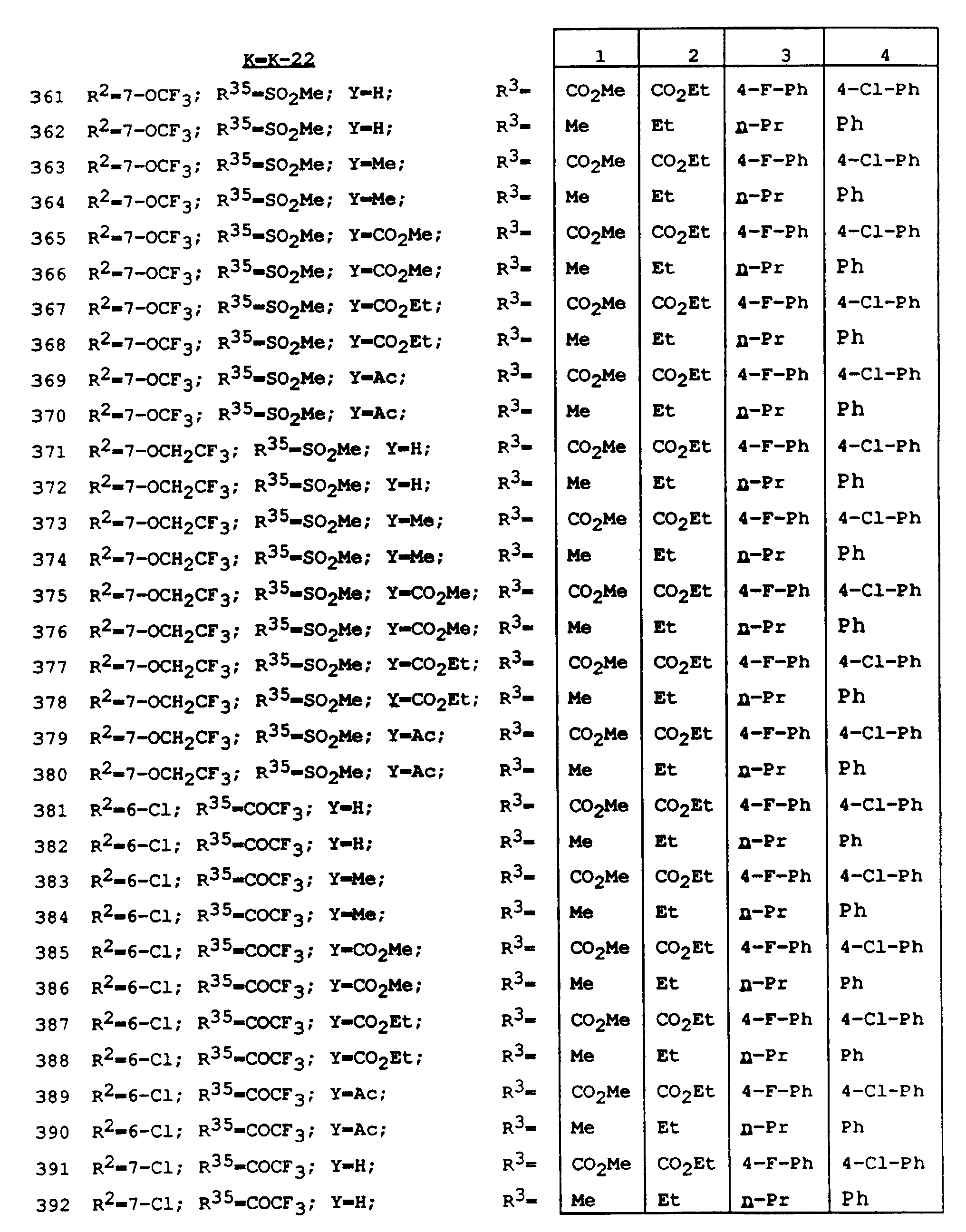

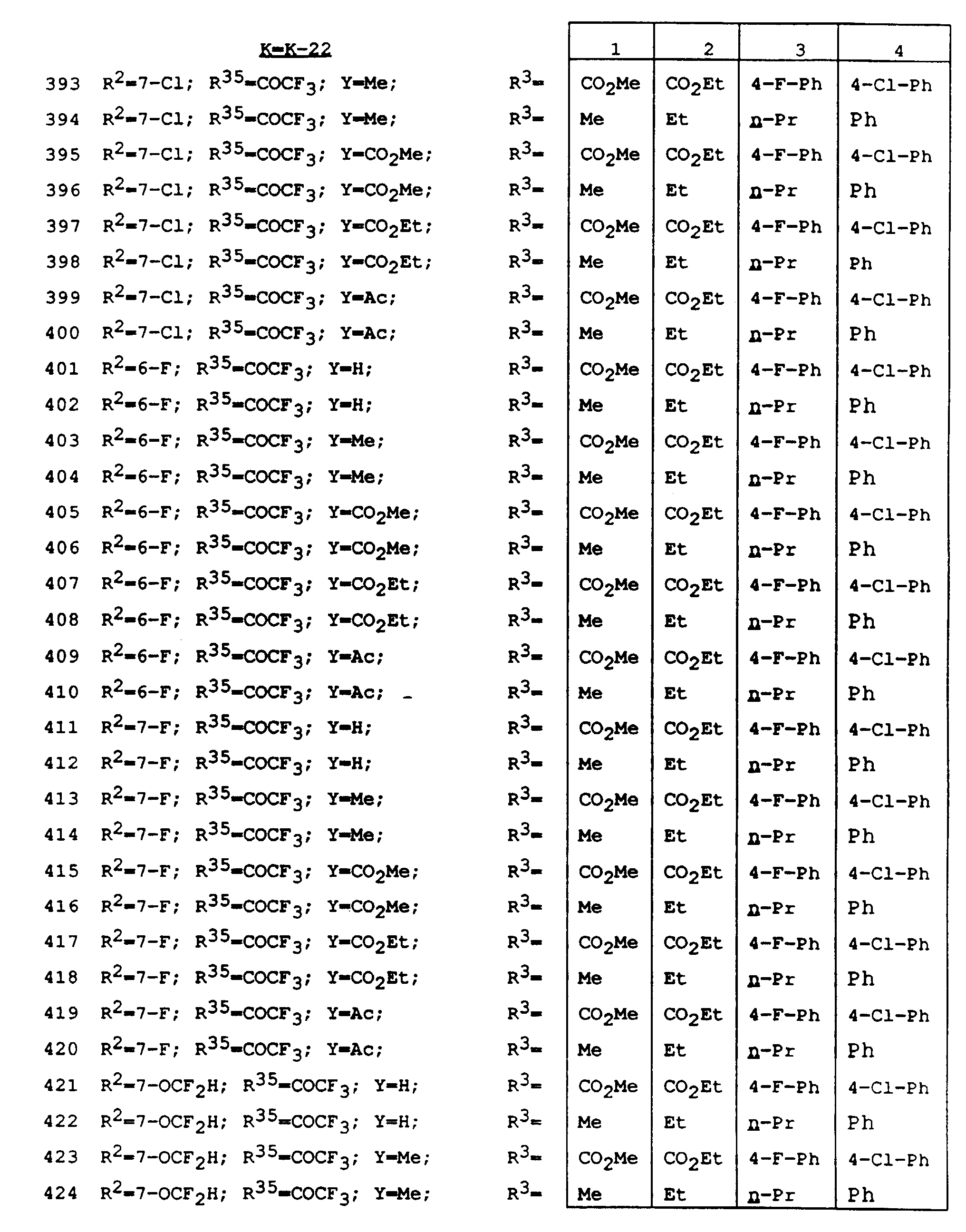

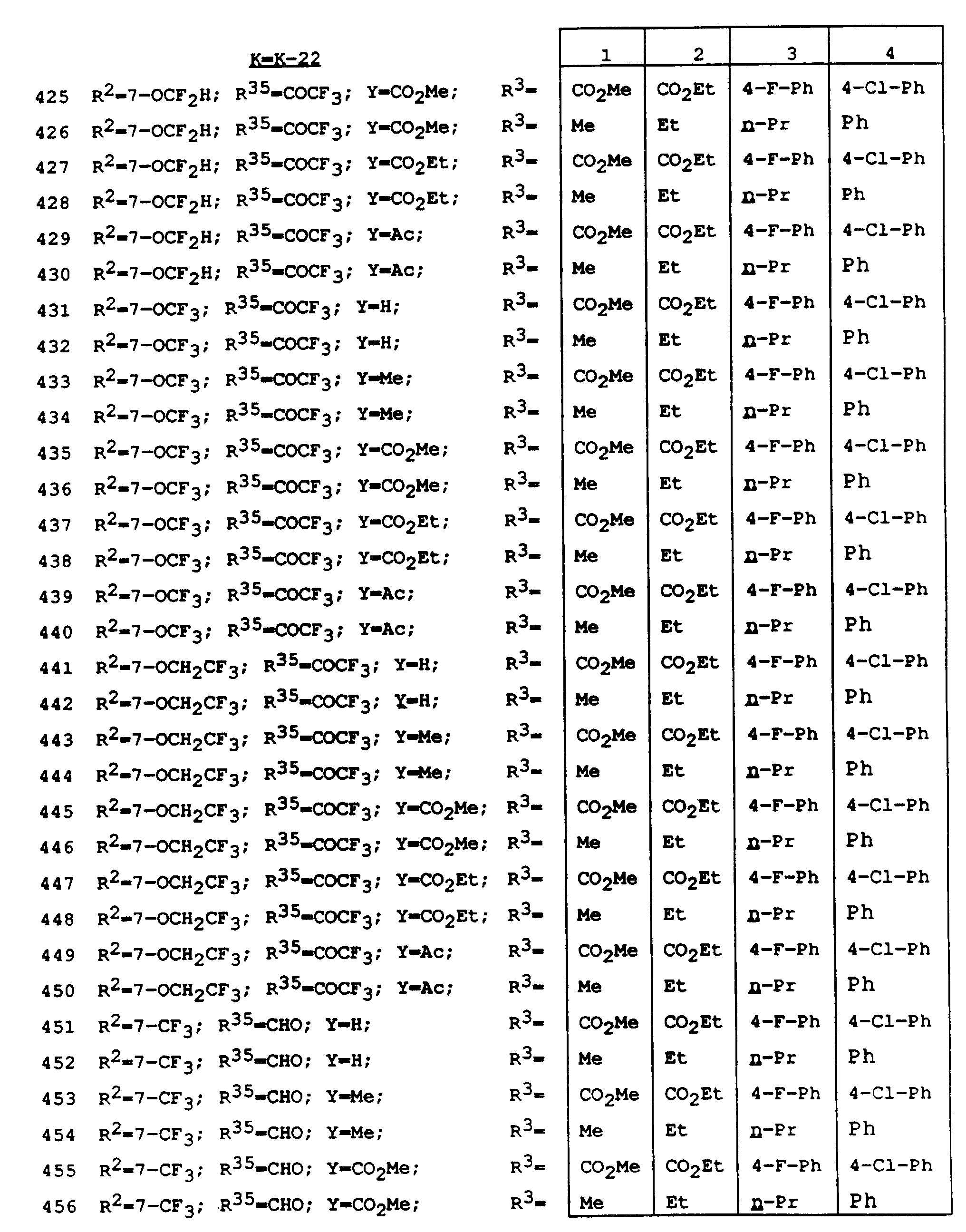

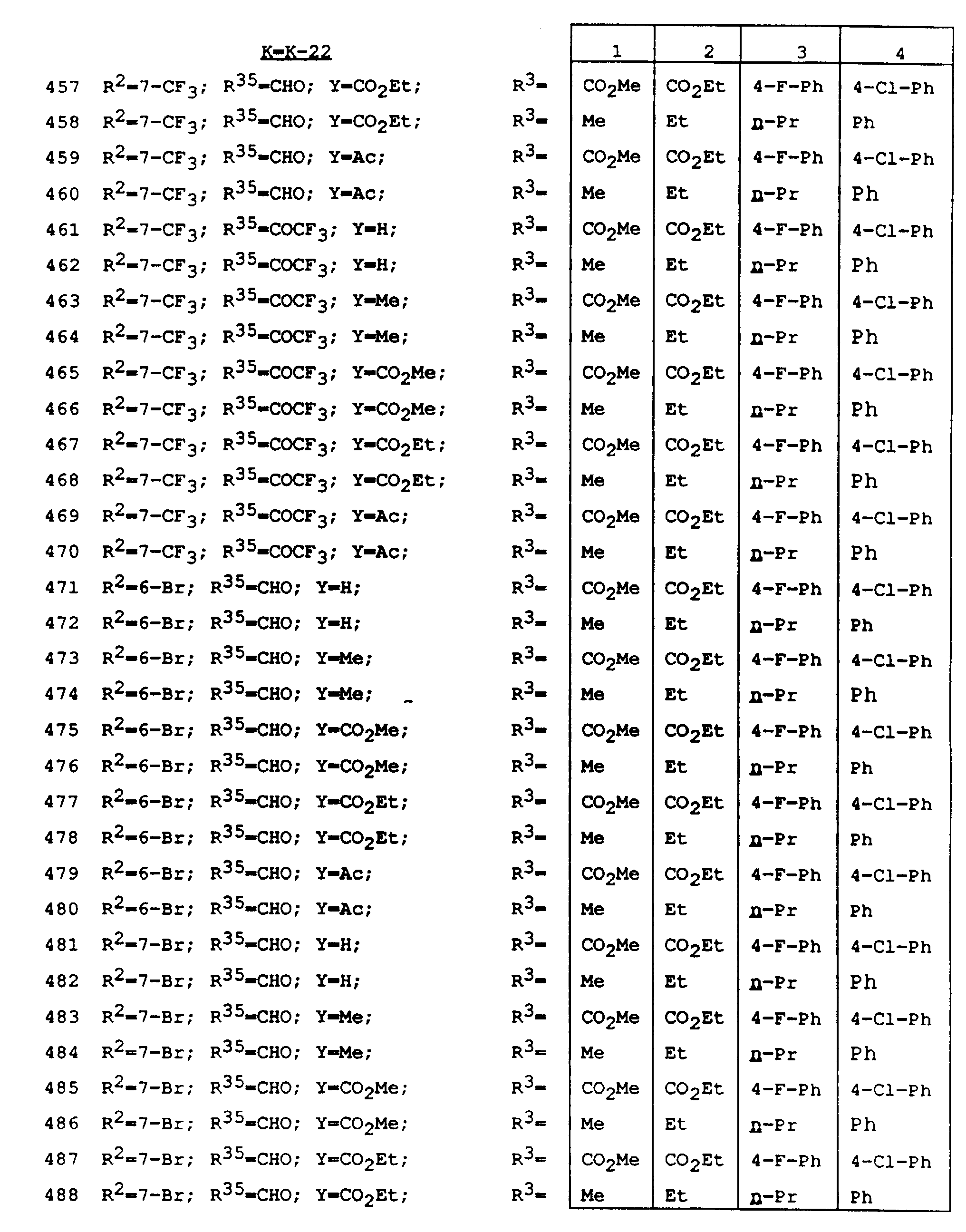

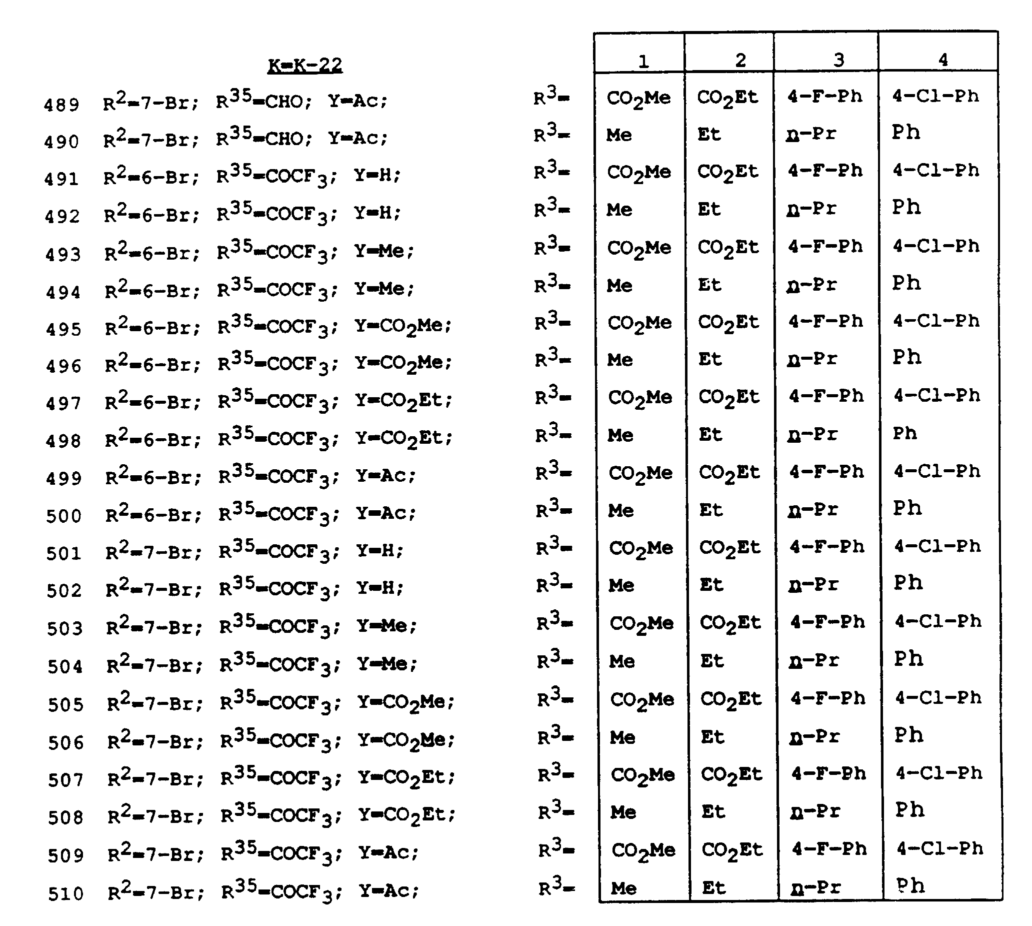

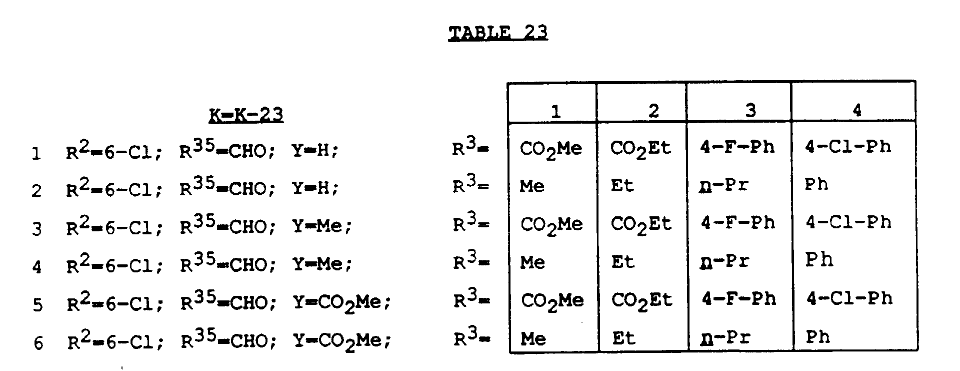

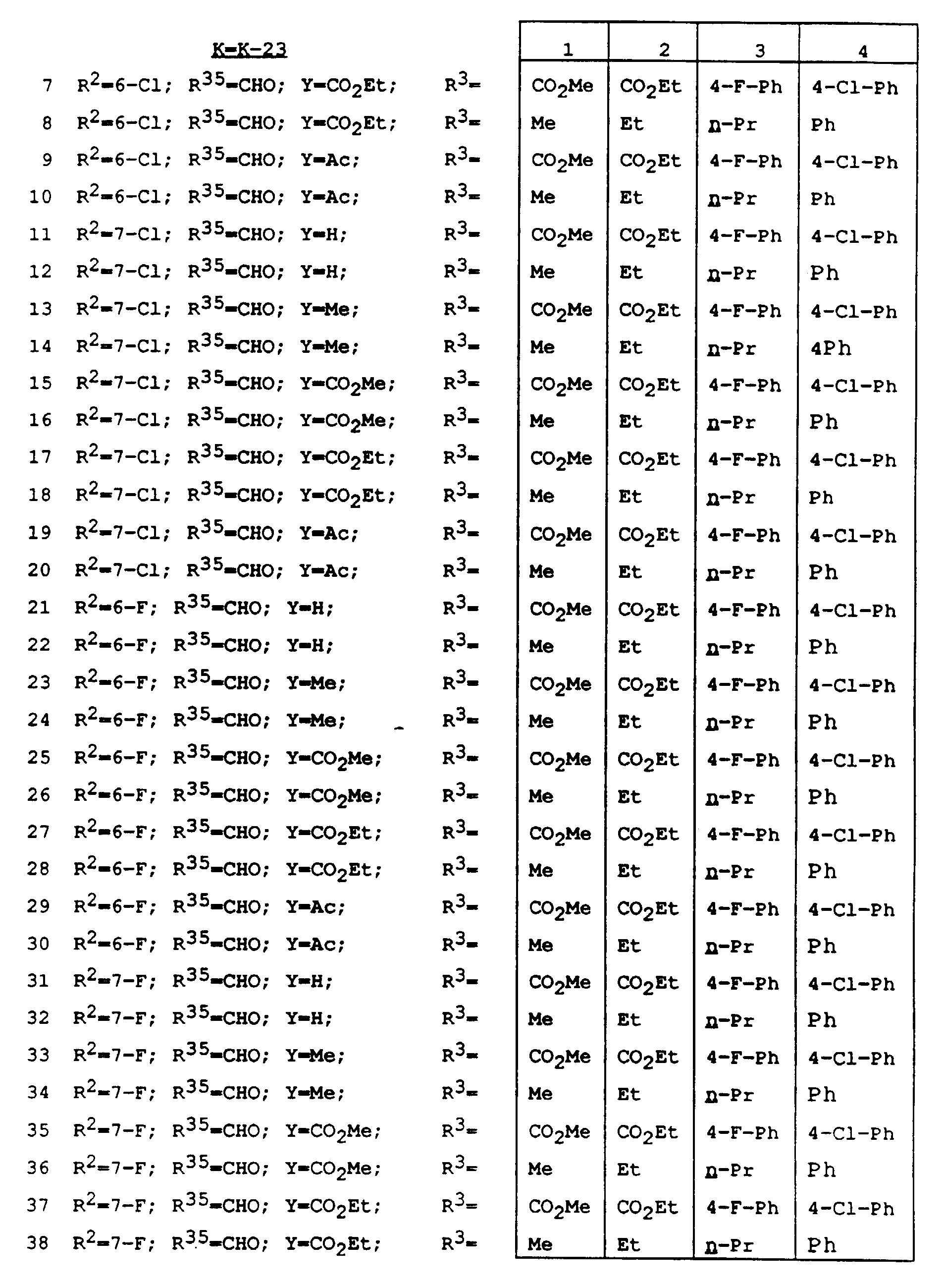

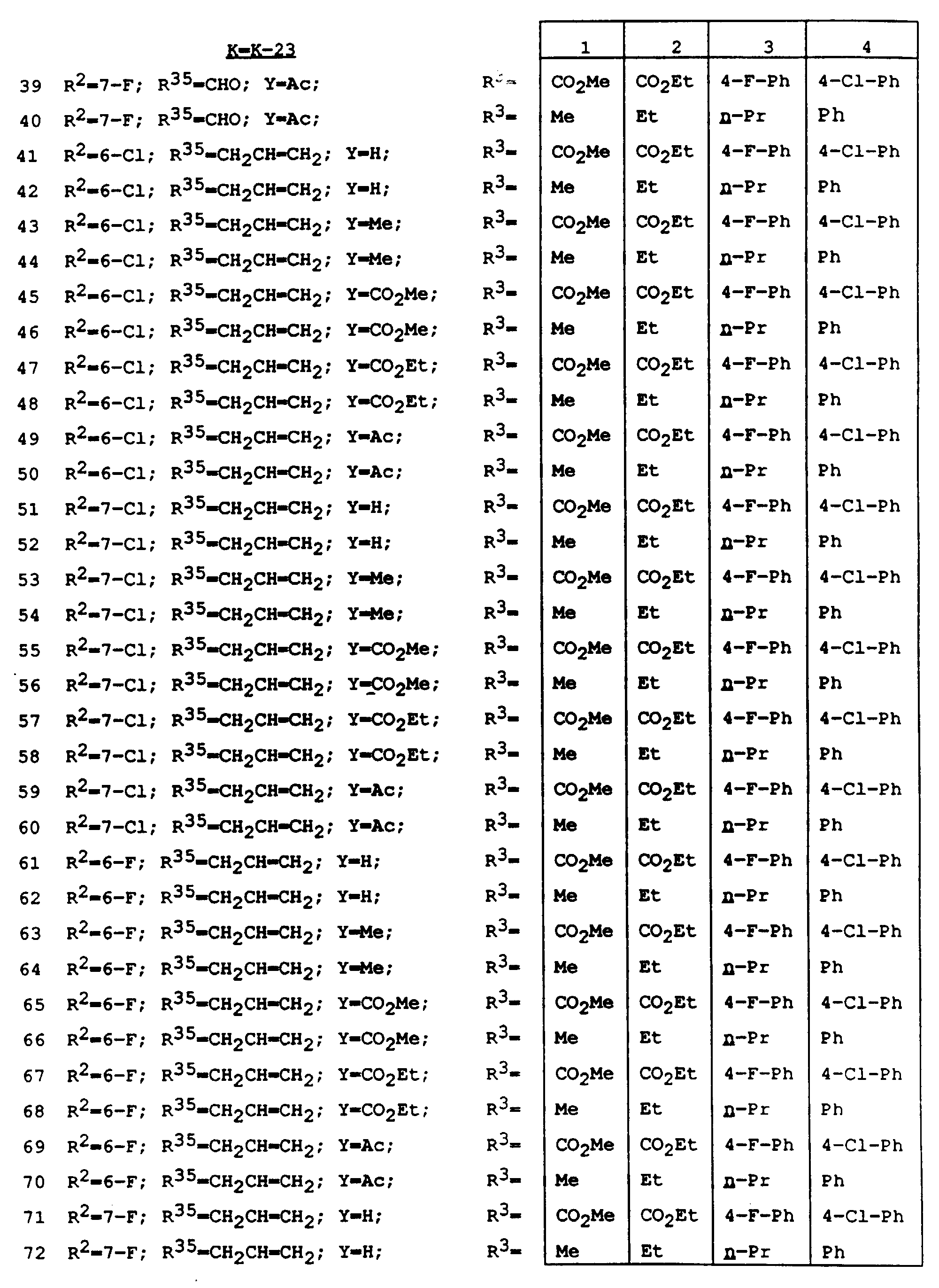

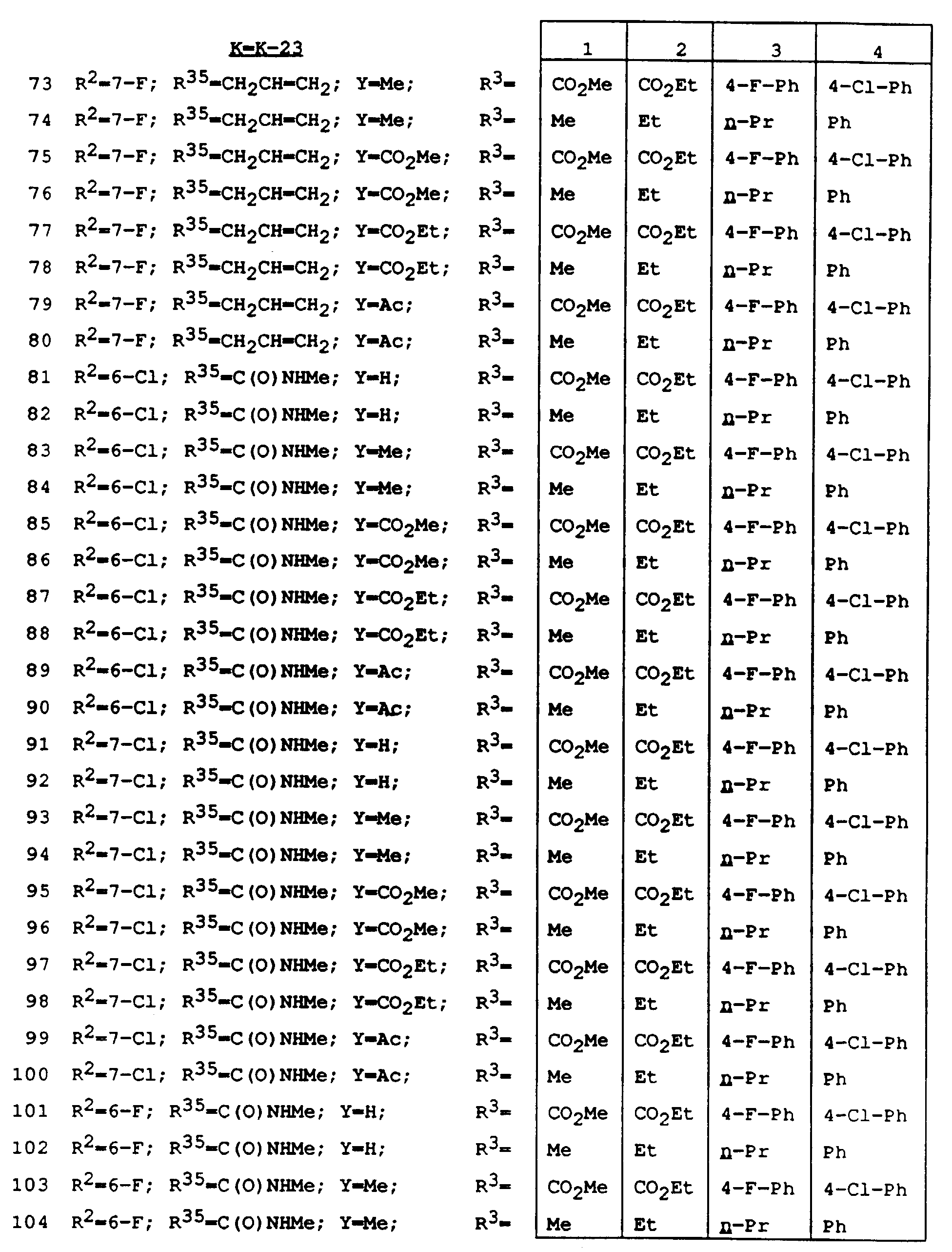

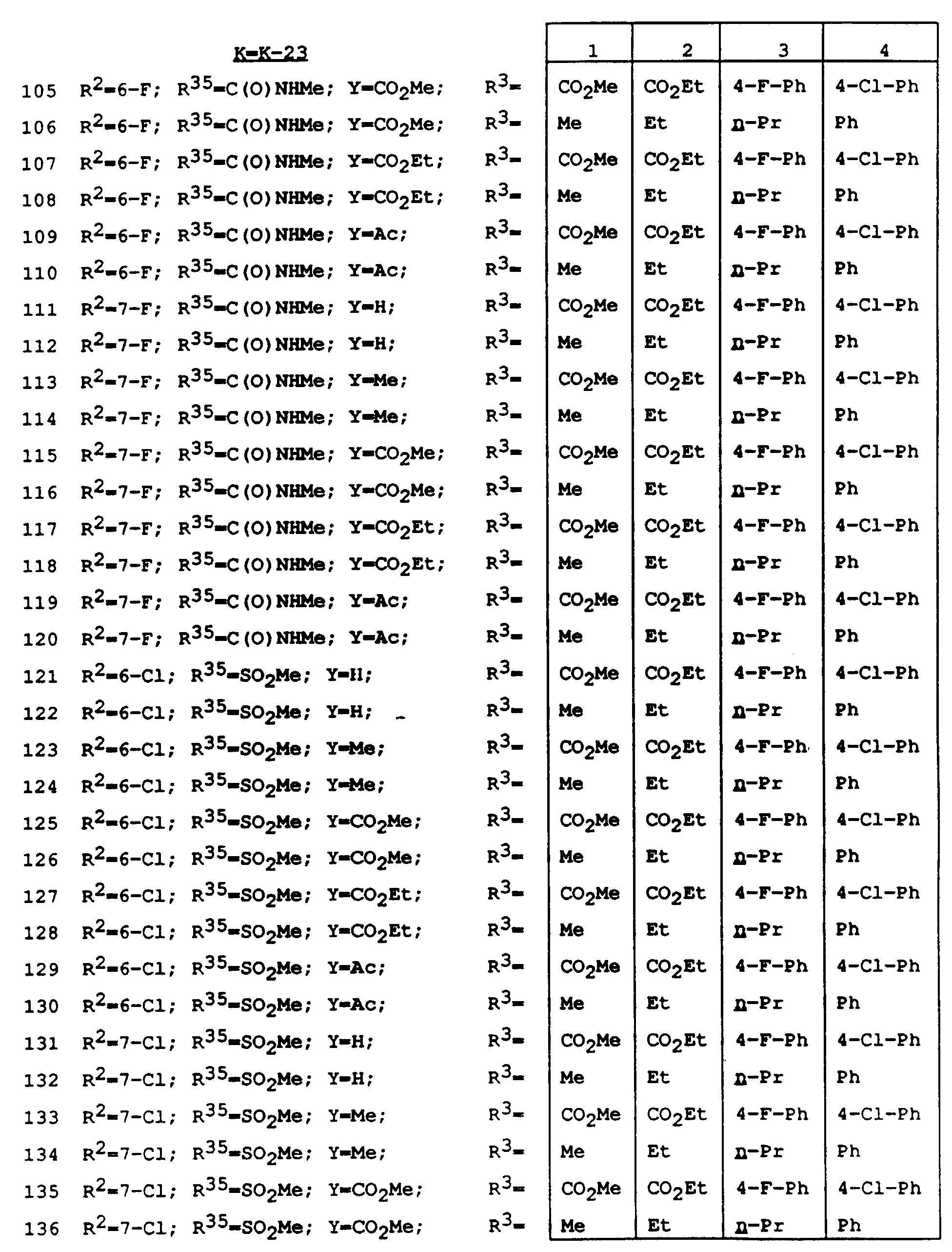

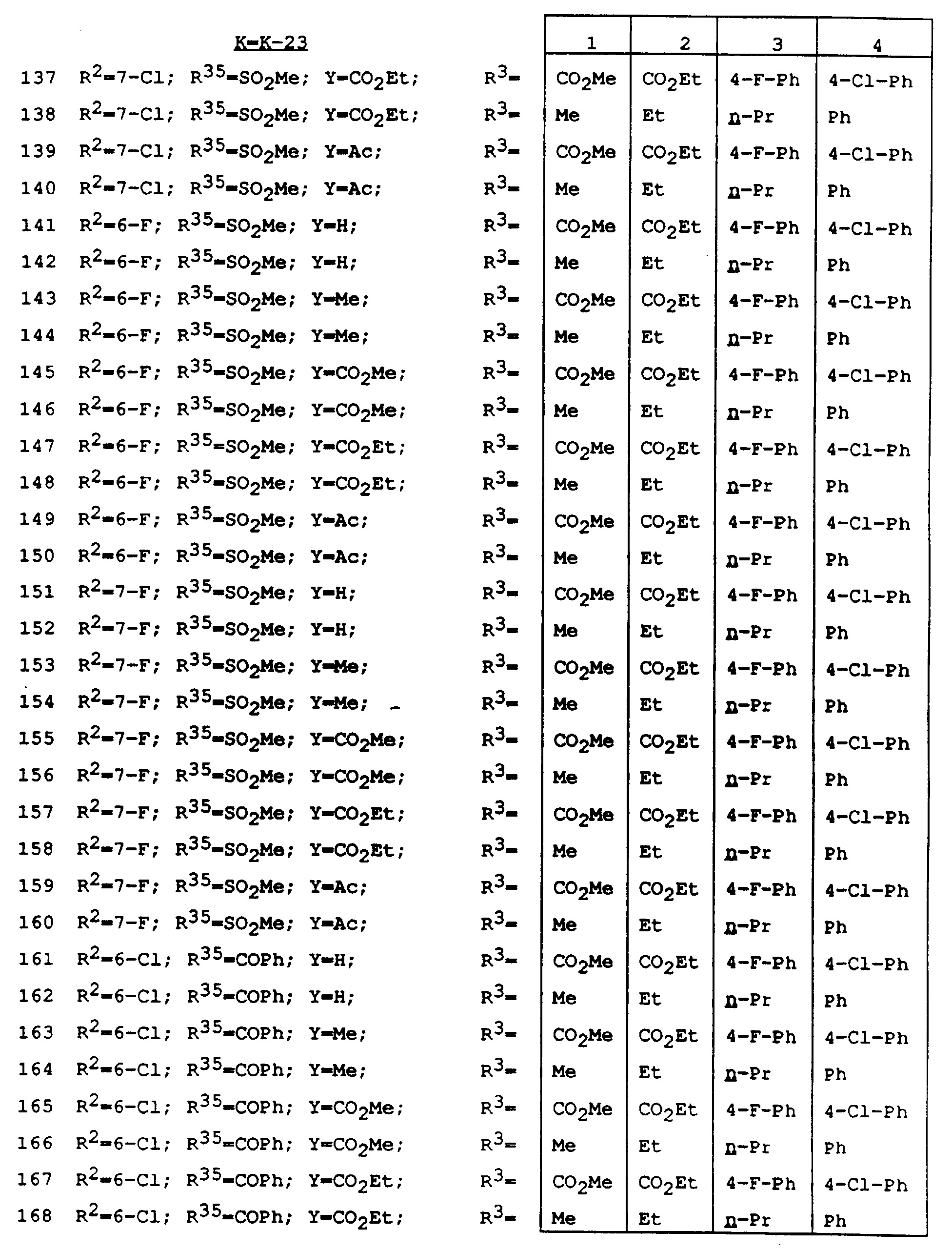

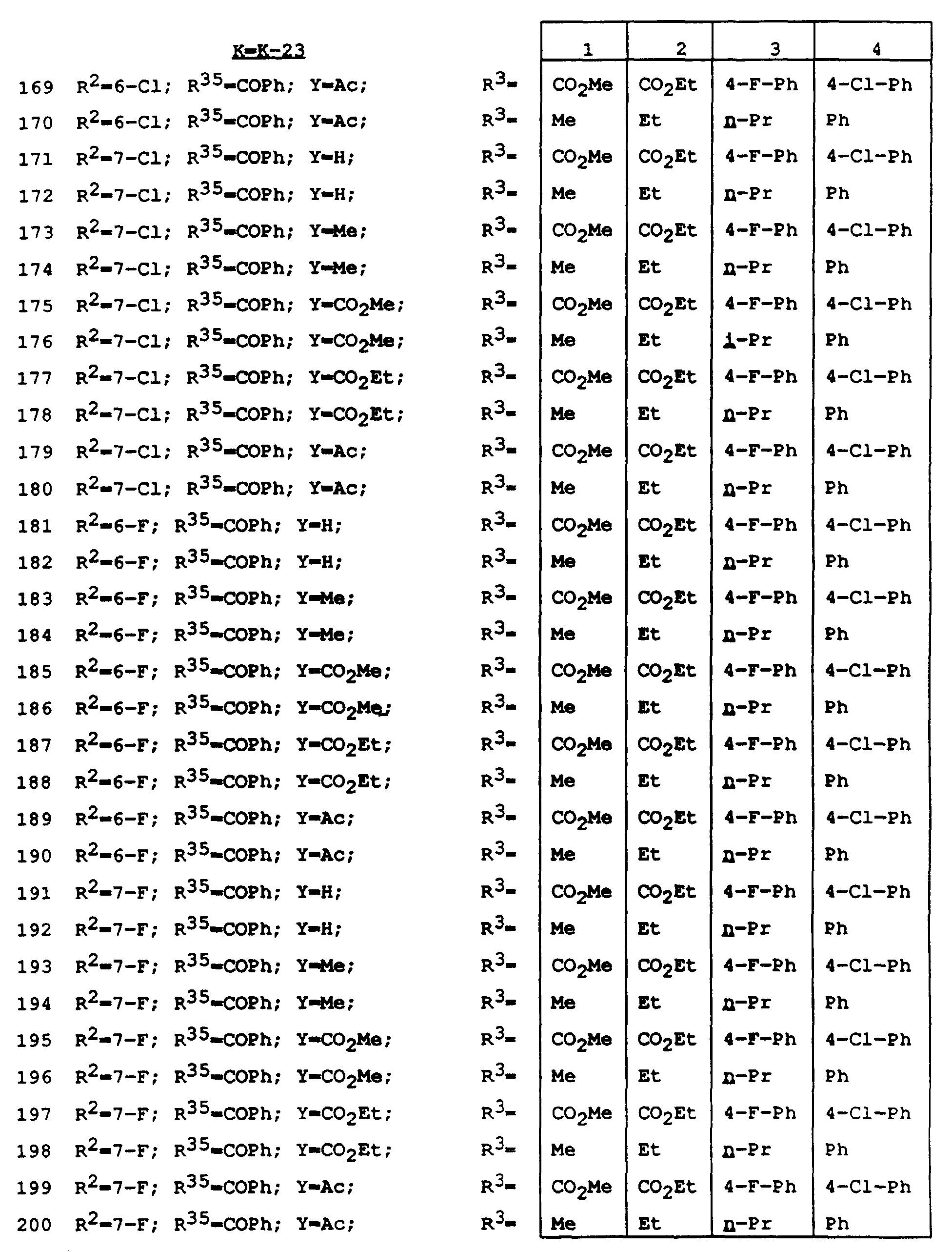

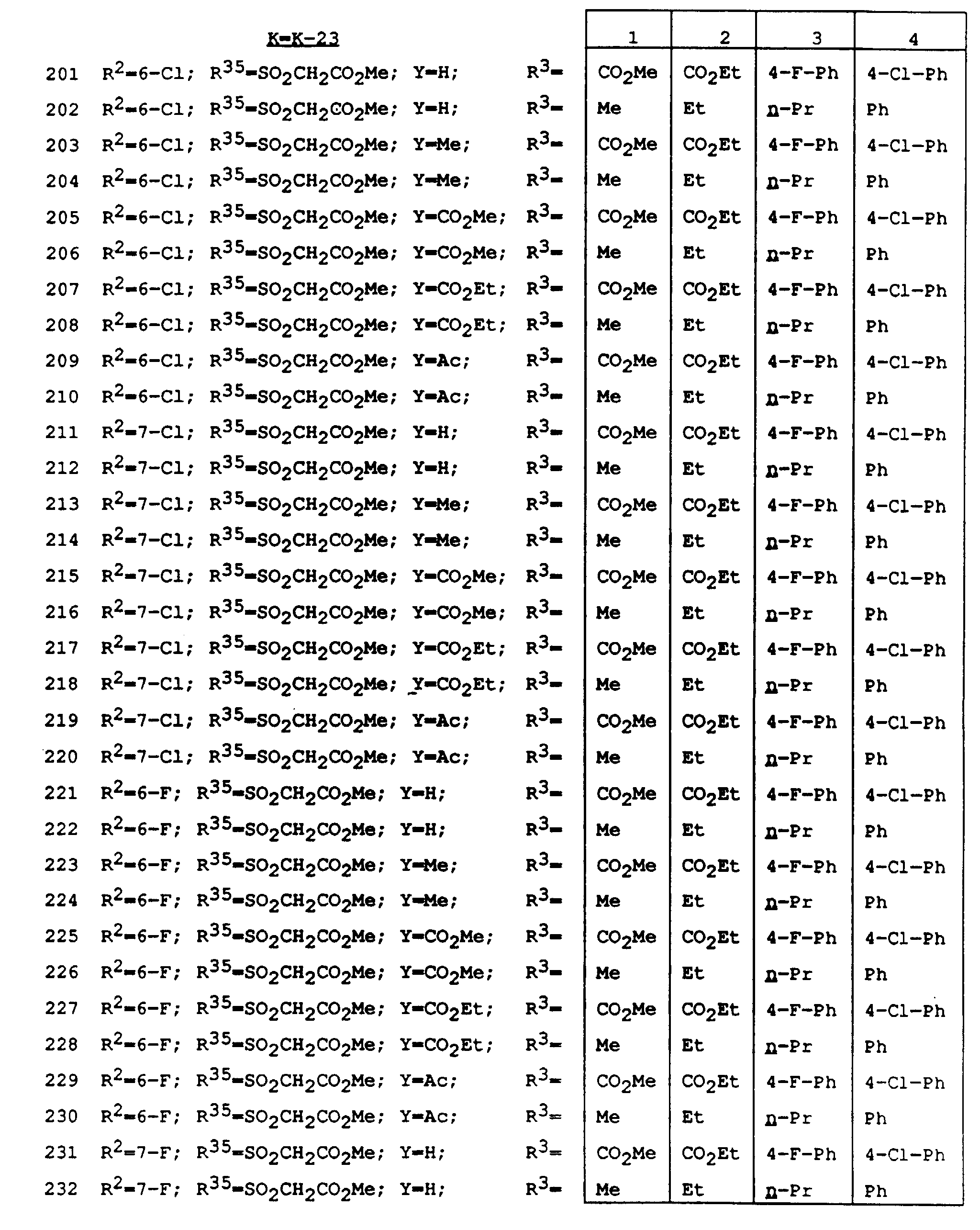

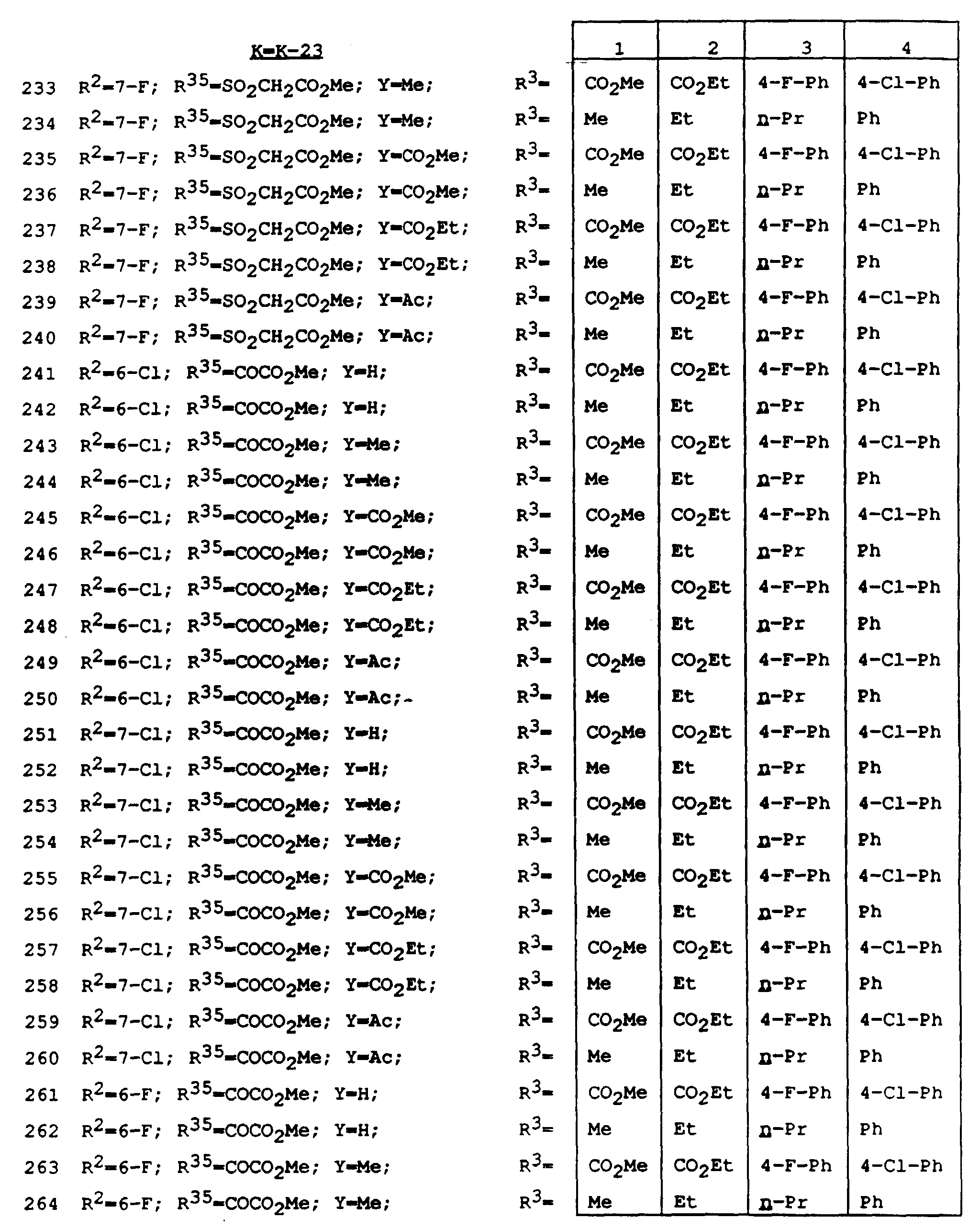

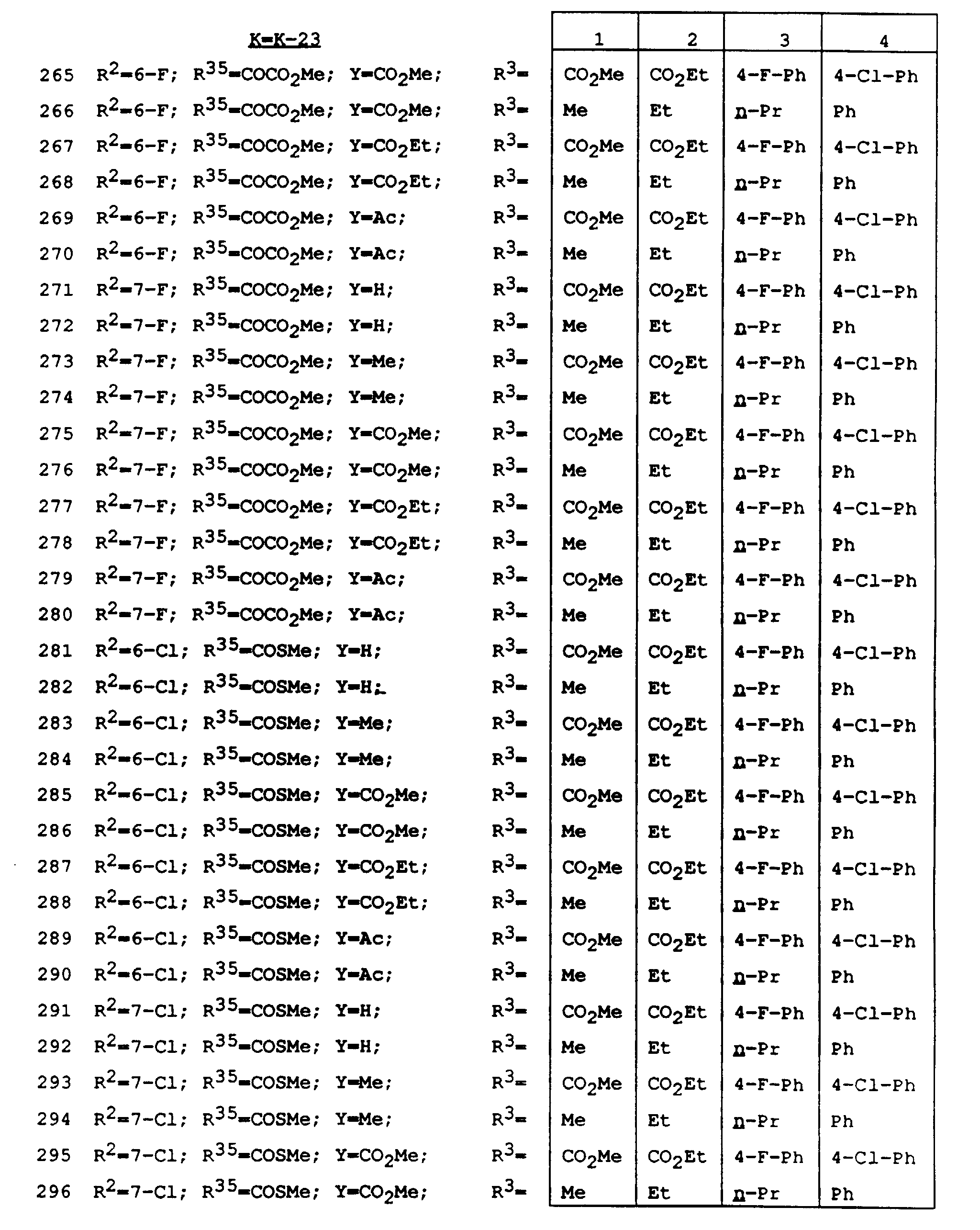

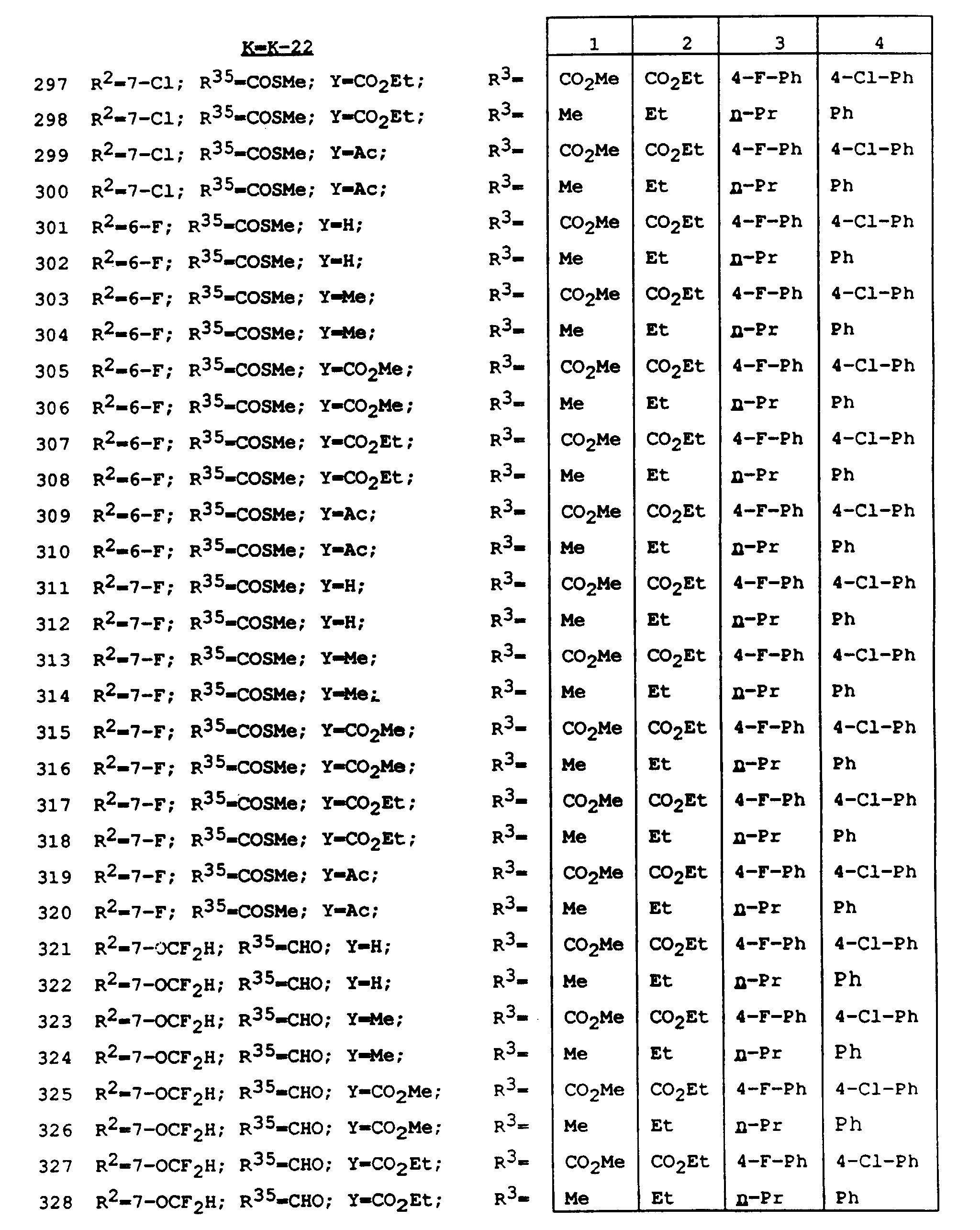

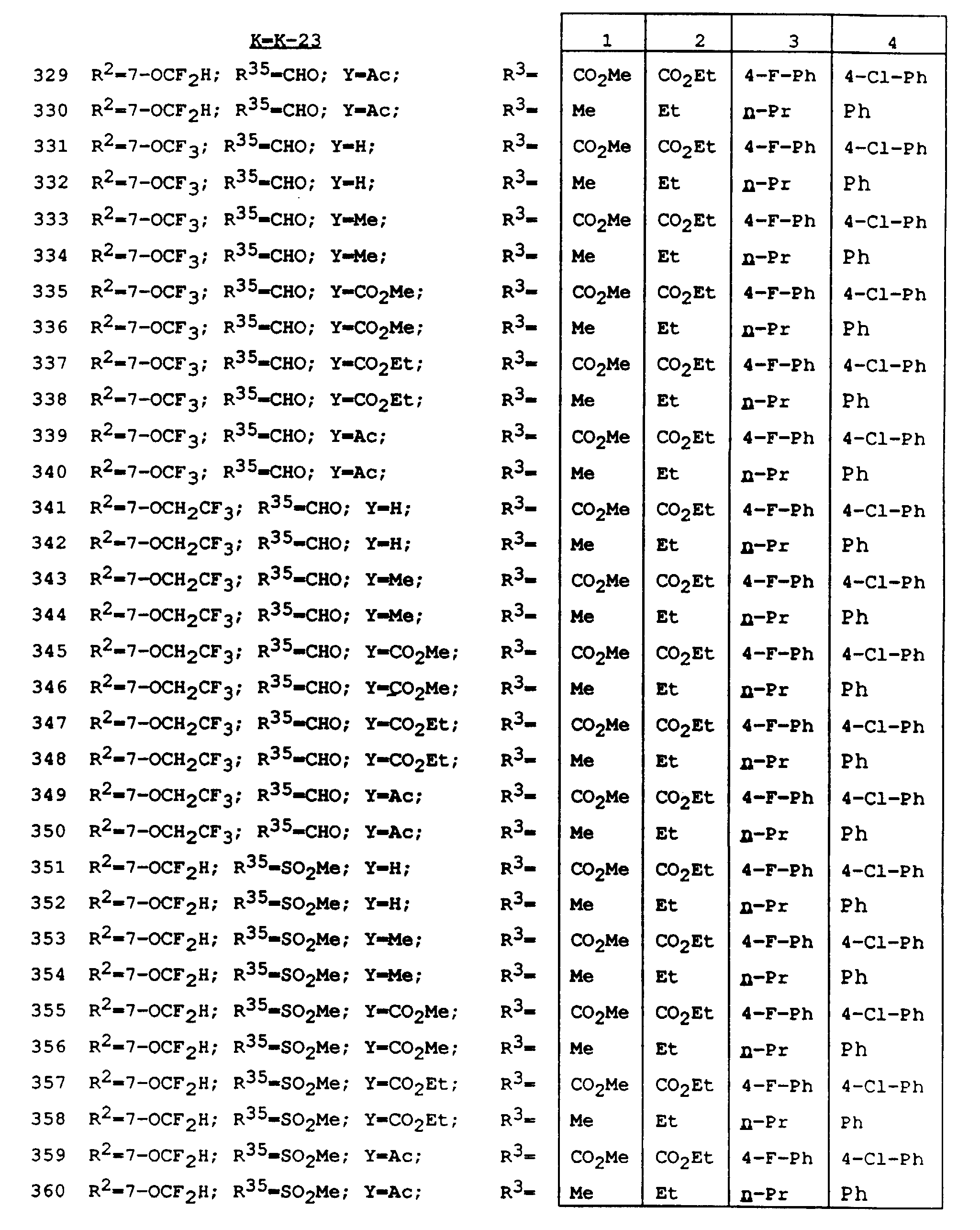

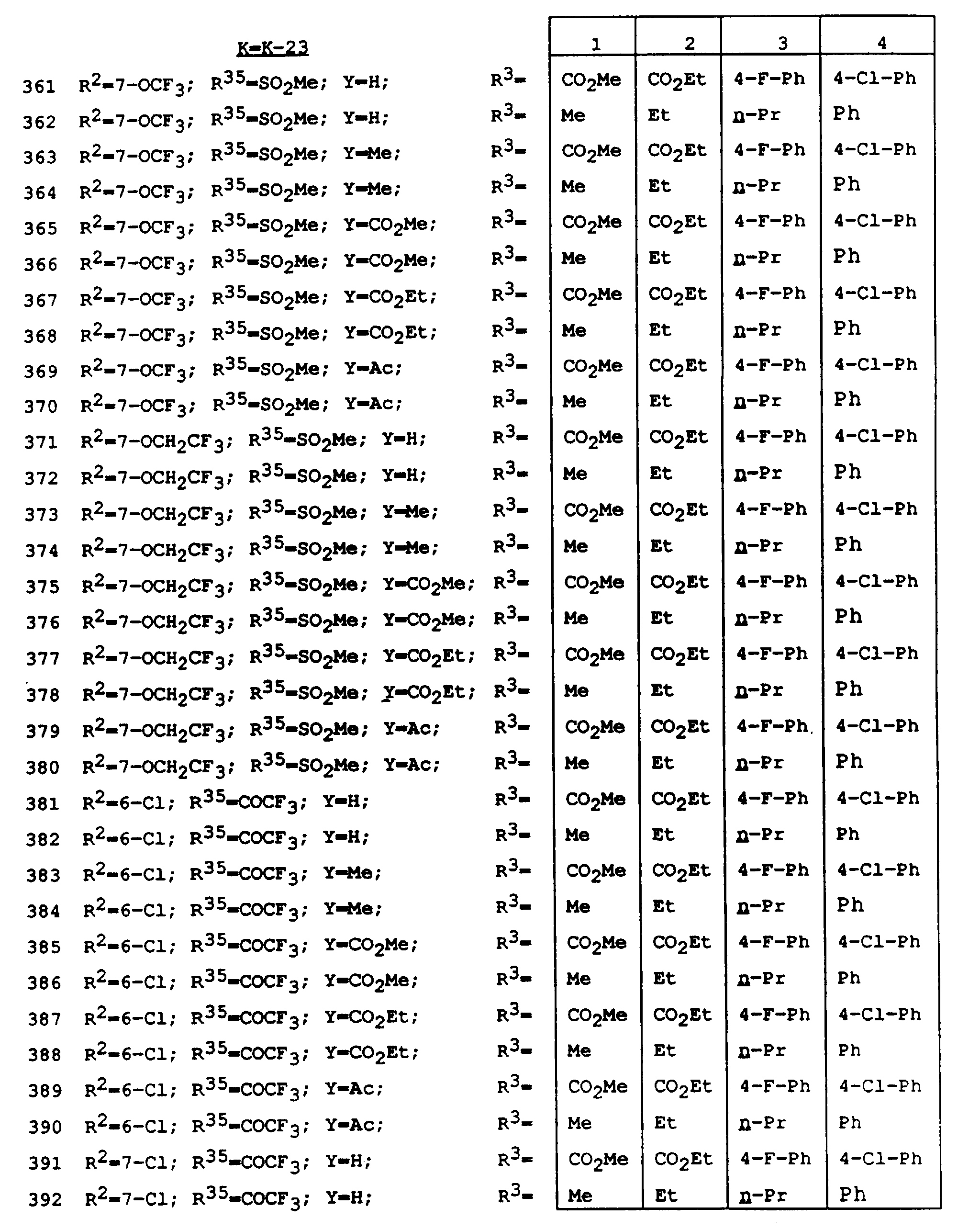

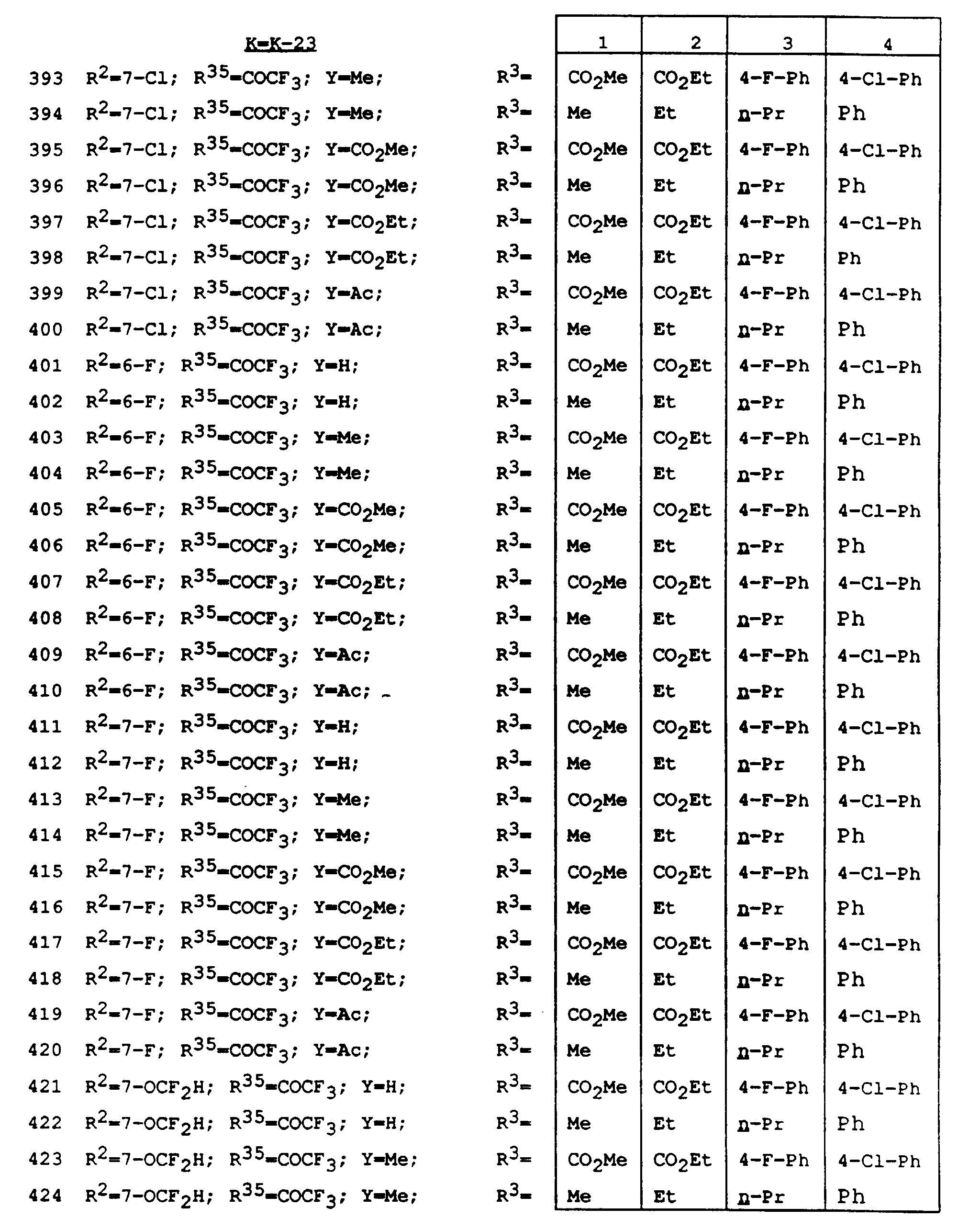

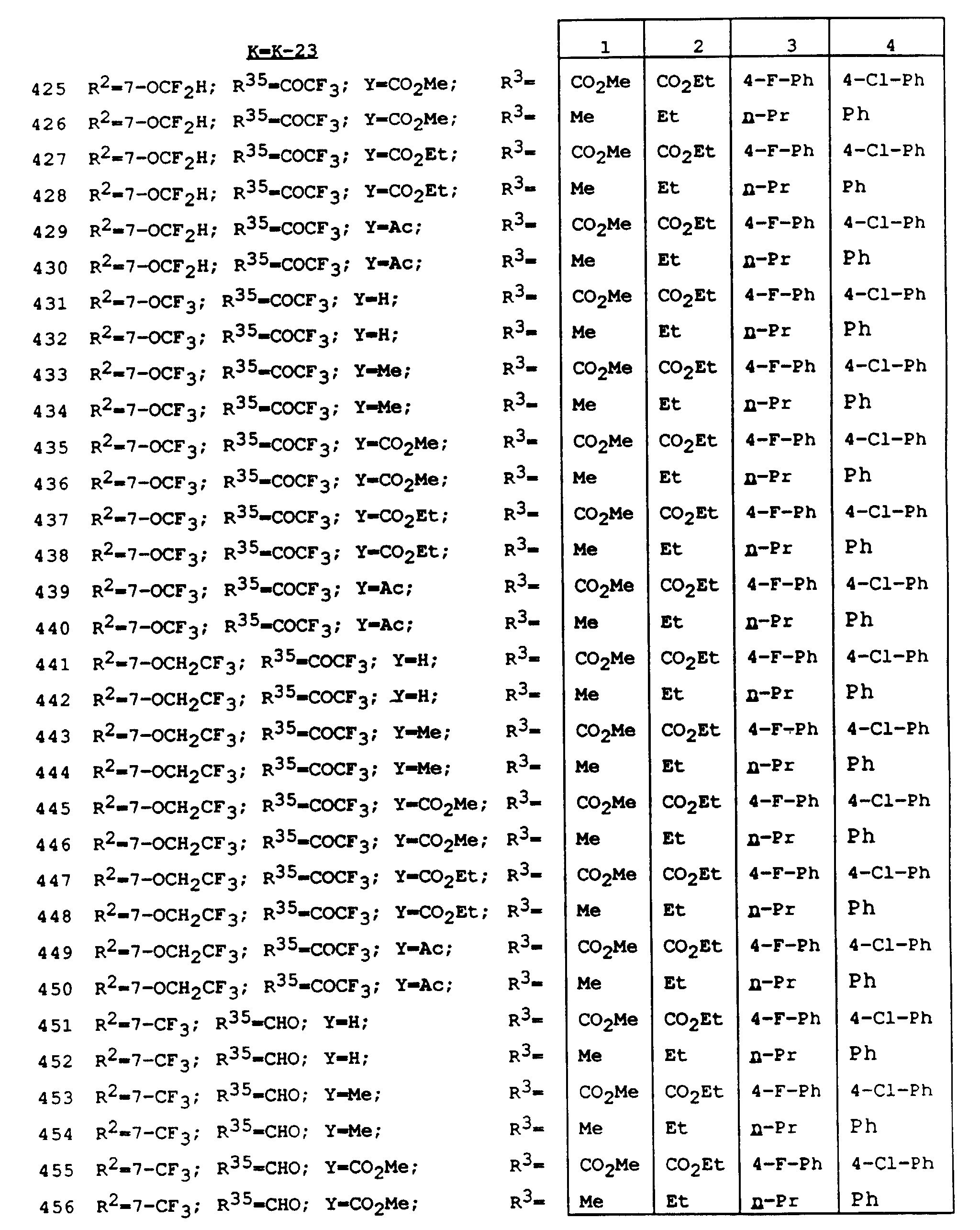

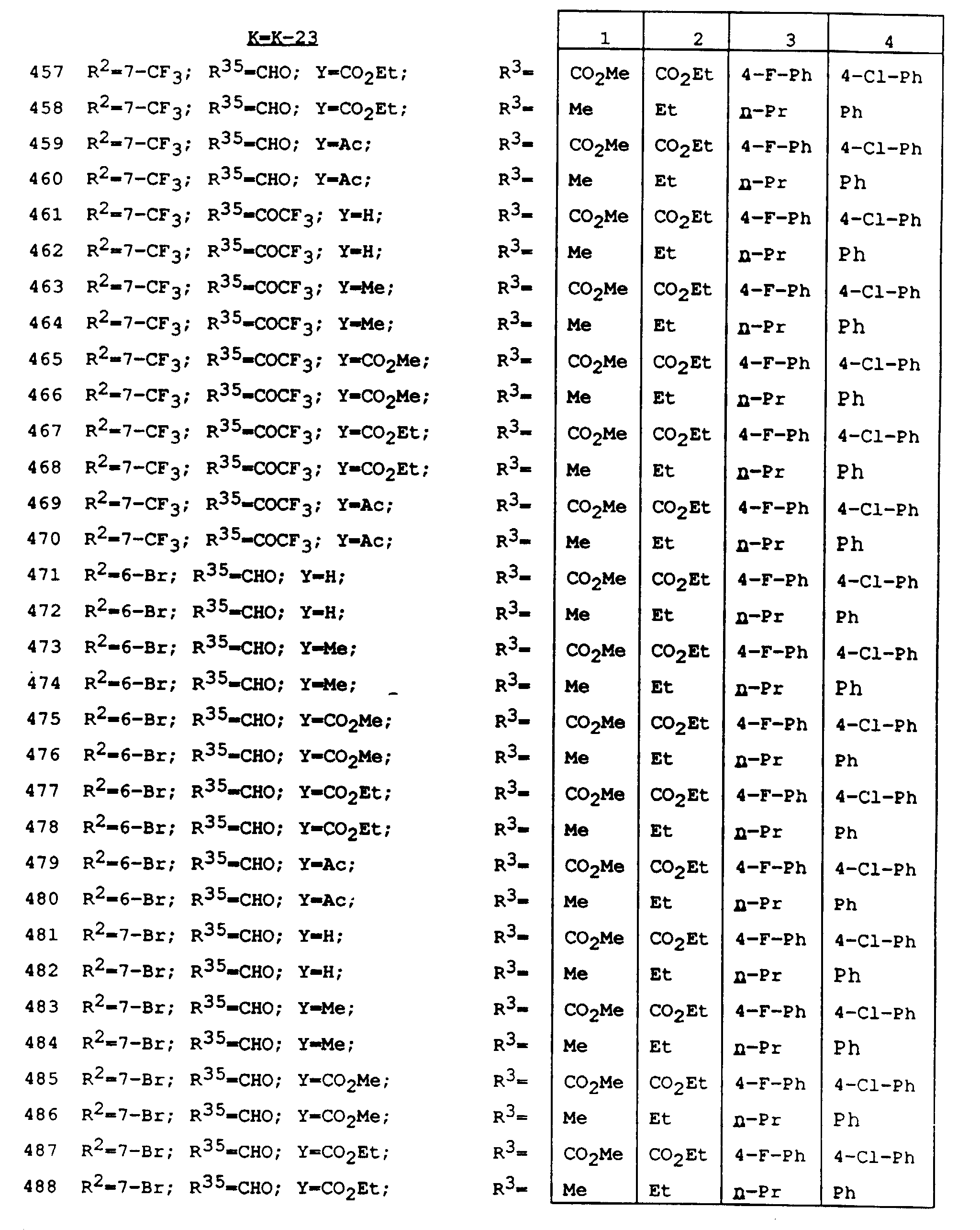

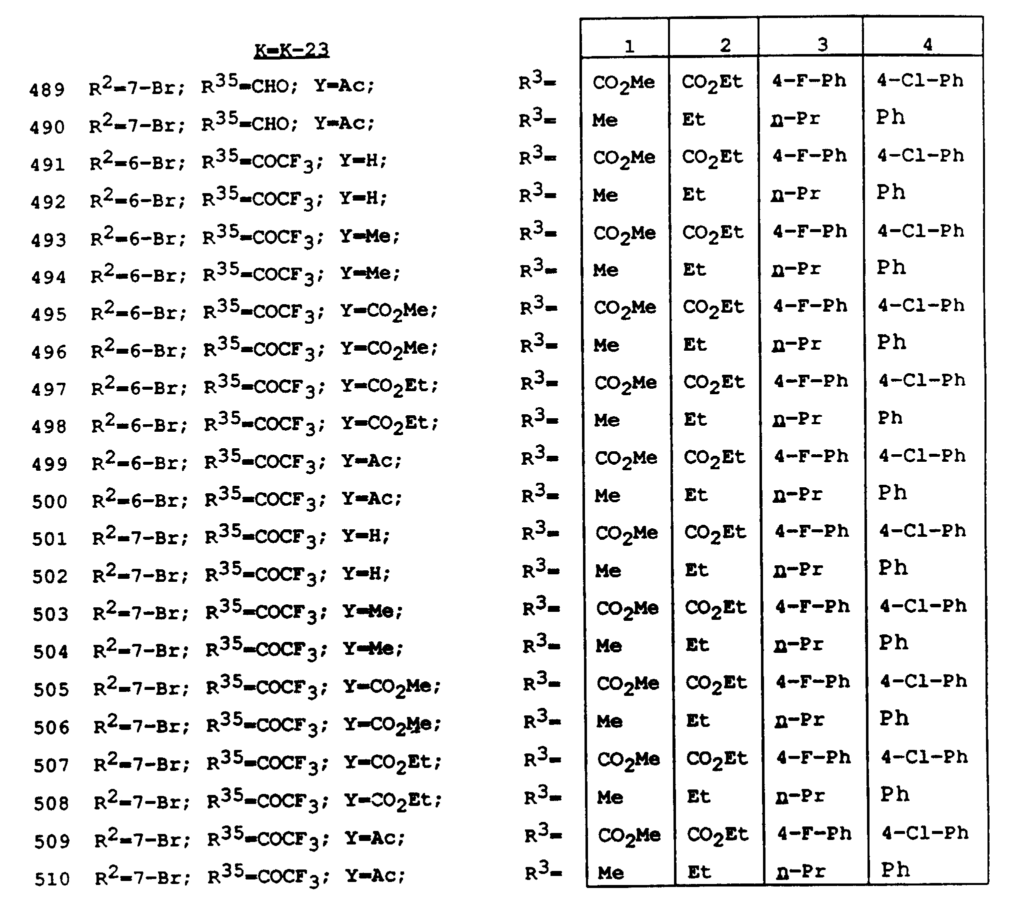

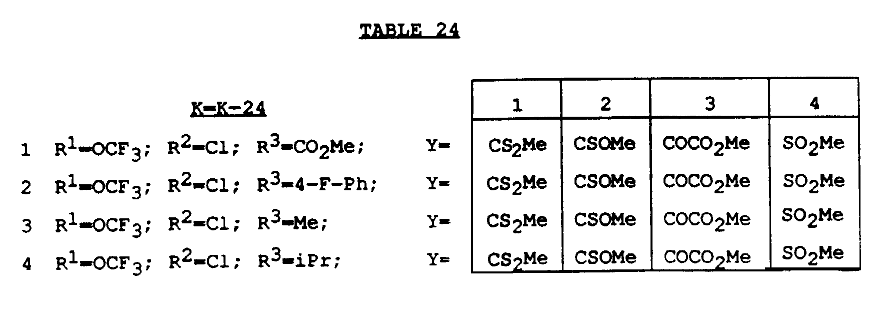

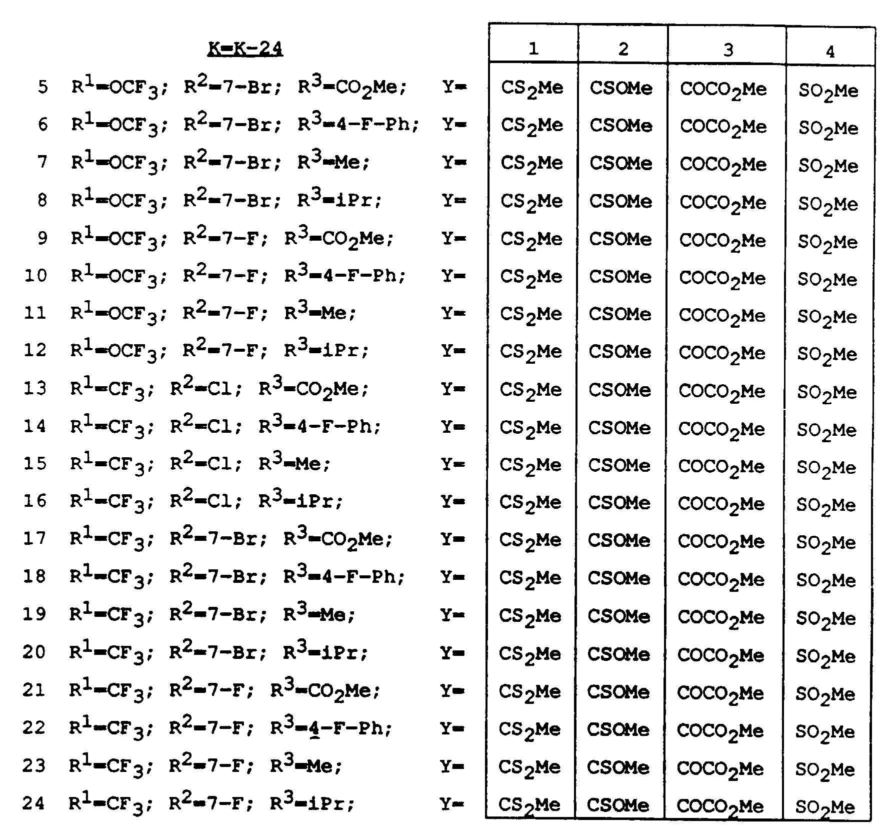

### Formulation/Utility

Compounds of this invention will generally be used in formulation with an agriculturally suitable carrier comprising a liquid or solid diluent or an organic solvent. Useful formulations include dusts, granules, baits, pellets, solutions, suspensions, emulsions, wettable powders, emulsifiable concentrates, dry flowables and the like, consistent with the physical properties of the active ingredient, mode of application and environmental factors such as soil type, moisture and temperature. Sprayable formulations can be extended in suitable media and used at spray volumes from about one to several hundred liters per hectare. High strength compositions are primarily used as intermediates for further formulation. The formulations will typically contain effective amounts of active ingredient, diluent and surfactant within the following approximate ranges which add up to 100 weight percent.

| | Weight Percent | | |
|---|---|---|---|
| | Active Ingredient | Diluent | Surfactant |
| Wettable Powders | 25-90 | 0-74 | 1-10 |
| Oil Suspensions, Emulsions, Solutions, (including Emulsifiable Concentrates) | 5-50 | 40-95 | 0-15 |
| Dusts | 1-25 | 70-99 | 0-5 |
| Granules, Baits and Pellets | 0.01-99 | 5-99.99 | 0-15 |
| High Strength Compositions | 90-99 | 0-10 | 0-2 |

Typical solid diluents are described in Watkins, et al., *Handbook of Insecticide Dust Diluents and Carriers,* 2nd Ed., Dorland Books, Caldwell, New Jersey. Typical liquid diluents and solvents are described in Marsden, *Solvents Guide,* 2nd Ed., Interscience, New York, 1950. *McCutcheon's Detergents and Emulsifiers Annual,* Allured Publ. Corp., Ridgewood, New Jersey, as well as Sisely and Wood, *Encyclopedia of Surface Active Agents,* Chemical Publ. Co., Inc., New York, 1964, list surfactants and recommended uses. All formulations can contain minor amounts of additives to reduce foam, caking, corrosion, microbiological growth, and the like.

Solutions are prepared by simply mixing the ingredients. Fine solid compositions are made by blending and, usually, grinding as in a hammer mill or fluid energy mill. Water-dispersible granules can be produced by agglomerating a fine powder composition; see for example, Cross et al., *Pesticide Formulations,* Washington, D.C., 1988, pp 251-259. Suspensions are prepared by wet-milling; see, for example, U.S. 3,060,084. Granules and pellets can be made by spraying the active material upon preformed granular carriers or by agglomeration techniques. See Browning, "Agglomeration", *Chemical Engineering,* December 4, 1967, pp 147-148, *Perry's Chemical Engineer's Handbook,* 4th Ed., McGraw-Hill, New York, 1963, pages 8-57 and following, and WO 91/13546. Pellets can be prepared by blending, grinding in a hammer mill, and compacting into pellets as further described in U.S. 4,172,714. Water-dispersible and water-soluble granules can be prepared as well-known to one skilled in the art.

For further information regarding the art of formulation, see U.S. 3,235,361, Col. 6, line 16 through Col. 7, line 19 and Examples 10-41; U.S. 3,309,192, Col. 5, line 43 through Col. 7, line 62 and Examples 8, 12, 15, 39, 41, 52, 53, 58, 132, 138-140, 162-164, 166, 167 and 169-182; U.S. 2,891,855, Col. 3, line 66 through Col. 5, line 17 and Examples 1-4; Klingman, *Weed Control as a Science,* John Wiley and Sons, Inc., New York, 1961, pp 81-96; and Hance et al., *Weed Control Handbook,* 8th Ed., Blackwell Scientific Publications, Oxford, 1989.

In the following Examples, all percentages are by weight and all formulations are worked up in conventional ways. Compound numbers refer to compounds in Index Table A.

| Example A | |
|---|---|
| Wettable Powder | |
| Compound 5 | 65.0% |
| dodecylphenol polyethylene glycol ether | 2.0% |
| sodium ligninsulfonate | 4.0% |
| sodium silicoaluminate | 6.0% |
| montmorillonite (calcined) | 23.0% |

| Example B | |
|---|---|
| Granule | |
| Compound 5 | 10.0% |
| attapulgite granules (low volatile matter, 0.71/Q.30 mm; U.S.S. No. 25-50 sieves) | 90.0% |

| Example C | |
|---|---|
| Extruded Pellet | |
| Compound 5 | 25.0% |
| anhydrous sodium sulfate | 10.0% |
| crude calcium ligninsulfonate | 5.0% |
| sodium alkylnaphthalenesulfonate | 1.0% |
| calcium/magnesium bentonite | 59.0% |

| Example D | |
|---|---|
| Emulsifiable Concentrate | |
| Compound 5 | 20.0% |
| blend of oil soluble sulfonates and polyoxyethylene ethers | 10.0% |
| isophorone | 70.0% |

The compounds of this invention exhibit activity against a wide spectrum of foliar-feeding, fruit-feeding, seed-feeding, aquatic and soil-inhabiting arthropods (term includes insects, mites and nematodes) which are pests of growing and stored agronomic crops, forestry, greenhouse crops, ornamentals, nursery crops, stored food and fiber products, livestock, household, and public and animal health. Those skilled in the art will appreciate that not all compounds are equally effective against all pests. Nevertheless, all of the compounds of this invention display activity against pests that include: eggs, larvae and adults of the Order Lepidoptera; eggs, foliar-feeding, fruit-feeding, root-feeding, seed-feeding larvae and adults of the Order Coleoptera; eggs, immatures and adults of the Orders Hemiptera and Homoptera; eggs, larvae, nymphs and adults of the Order Acari; eggs, immatures and adults of the Orders Thysanoptera, Orthoptera and Dermaptera; eggs, immatures and adults of the Order Diptera; and eggs, juveniles and adults of the Phylum Nemata. The compounds of this invention are also active against pests of the Orders Hymenoptera, Isoptera, Phthiraptera, Siphonoptera, Blattaria, Thysanaura and Pscoptera; pests belonging to the Class Arachnida and Phylum Platyhelminthes. The compounds are particularly active against southern corn rootworm *(Diabrotica undecimpunctata howardi),* aster leafhopper *(Mascrosteles fascifrons),* boll weevil *(Anthonomus grandis),* two-spotted spider mite *(Tetranychus urticae),* fall armyworm *(Spodoptera frugiperda),* black bean aphid *(Aphis fabae),* tobacco budworm *(Heliothis virescens),* rice water weevil *(Lissorhoptrus oryzophilus),* rice leaf beetle *(Oulema oryzae),* whitebacked planthopper *(Sogatella furcifera),* green leafhopper *(Nephotettix cincticeps),* brown planthopper *(Nilaparvata lugens),* small brown planthopper *(Laodelphax striatellus),* rice stem borer *(Chilo suppressalis),* rice leafroller *(Cnaphalocrocis medinalis),* black rice stink bug *(Scotinophara lurida),* rice stink bug *(Lagynotomus elongatus),* slender rice bug *(Cletus puntiger),* and southern green stink bug *(Nezara viridula).* See WO 90/10623 and WO 92/00673 for more detailed pest descriptions.

Compounds of this invention can also be mixed with one or more other insecticides, fungicides, nematocides, bactericides, acaricides, semiochemicals, repellants, attractants, pheromones, feeding stimulants or other biologically active compounds to form a multicomponent pesticide giving an even broader spectrum of agricultural protection. Examples of other agricultural protectants with which compounds of this invention can be formulated are: insecticides such as monocrotophos, carbofuran, tetrachlorvinphos, malathion, parathion-methyl, methomyl, chlordimeform, diazinon, deltamethrin, oxamyl, fenvalerate, esfenvalerate, permethrin, profenofos, sulprofos, triflumuron, diflubenzuron, methoprene, buprofezin, thiodicarb, acephate, azinphosmethyl, chlorpyrifos, dimethoate, fipronil, flufenprox, fonophos, isofenphos, methidathion, methamidophos, phosmet, phosphamidon, phosalone, pirimicarb, phorate, terbufos, trichlorfon, methoxychlor, bifenthrin, biphenate, cyfluthrin, fenpropathrin, fluvalinate, flucythrinate, tralomethrin, metaldehyde and rotenone; fungicides such as carbendazim, thiuram, dodine, maneb, chloroneb, benomyl, cymoxanil, fenpropidine, fenpropimorph, triadimefon, captan, thiophanate-methyl, thiabendazole, phosethyl-Al, chlorothalonil, dichloran, metalaxyl, captafol, iprodione, oxadixyl, vinclozolin, kasugamycin, myclobutanil, tebuconazole, difenoconazole, diniconazole, fluquinconazole, ipconazole, metconazole, penconazole, propiconazole, uniconzole, flutriafol, prochloraz, pyrifenox, fenarimol, triadimenol, diclobutrazol, copper oxychloride, furalaxyl, folpet, flusilazol, blasticidin S, diclomezine, edifenphos, isoprothiolane, iprobenfos, mepronil, neo-asozin, pencycuron, probenazole, pyroquilon, tricyclazole, validamycin, and flutolanil; nematocides such as aldoxycarb, fenamiphos and fosthietan; bactericides such as oxytetracyline, streptomycin and tribasic copper sulfate; acaricides such as binapacryl, oxythioquinox, chlorobenzilate, dicofol, dienochlor, cyhexatin, hexythiazox, amitraz, propargite, tebufenpyrad and fenbutatin oxide; and biological agents such as Bacillus thuringiensis, baculovirus and avermectin B.

In certain instances, combinations with other arthropodicides having a similiar spectrum of control but a different mode of action will be particularly advantageous for resistance management.

Arthropod pests are controlled and protection of agronomic crops, animal and human health is achieved by applying one or more of the compounds of this invention, in an effective amount, to the environment of the pests including the agronomic and/or nonagronomic locus of infestation, to the area to be protected, or directly on the pests to be controlled. A preferred method of application is by spraying. Alternatively, granular formulations of these compounds can be applied to the plant foliage or the soil. Other methods of application include direct and residual sprays, aerial sprays, systemic uptake, baits, eartags, boluses, foggers, fumigants, aerosols, and many others. The compounds can be incorporated into baits that are consumed by the arthropods or in devices such as traps and the like.

The compounds of this invention can be applied in their pure state, but most often application will be of a formulation comprising one or more compounds with suitable carriers, diluents, and surfactants and possibly in combination with a food depending on the contemplated end use. A preferred method of application involves spraying a water dispersion or refined oil solution of the compounds. Combinations with spray oils, spray oil concentrations, spreader stickers, adjuvants, and synergists and other solvents such as piperonyl butoxide often enhance compound efficacy.

The rate of application required for effective control will depend on such factors as the species of arthropod to be controlled, the pest's life cycle, life stage, its size, location, time of year, host crop or animal, feeding behavior, mating behavior, ambient moisture, temperature, and the like. Under normal circumstances, application rates of about 0.01 to 2 kg of active ingredient per hectare are sufficient to control pests in agronomic ecosystems, but as little as 0.001 kg/hectare may be sufficient or as much as 8 kg hectare may be required. For nonagronomic applications, effective use rates will range from about 1.0 to 50 mg/square meter but as little as 0.1 mg/square meter may be sufficient or as much as 150 mg/square meter may be required.

The following Tests demonstrate the control efficacy of compounds of this invention on specific pests. The pest control protection afforded by the compounds is not limited, however, to these species. See Index Tables A-E for compound descriptions.

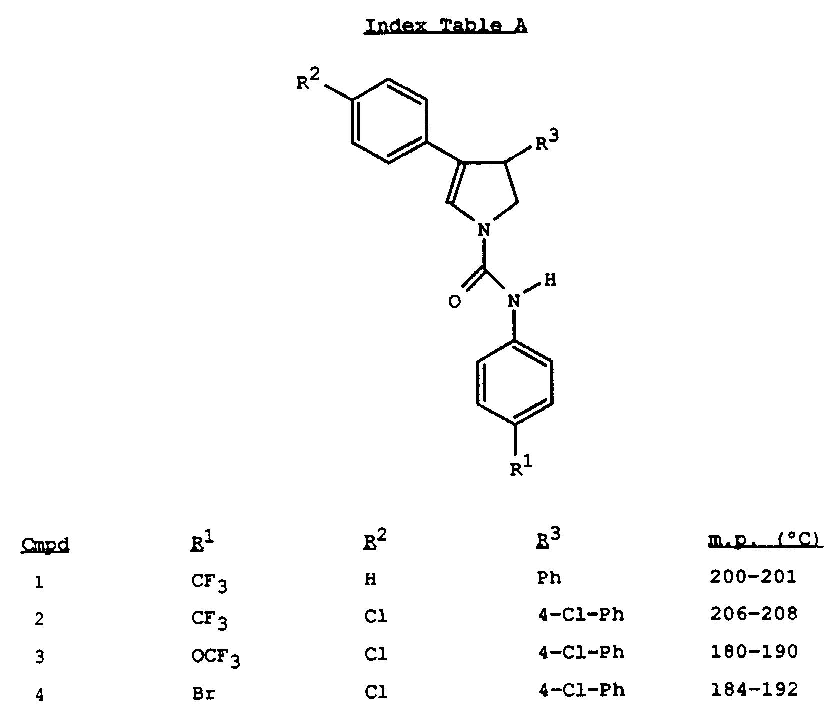

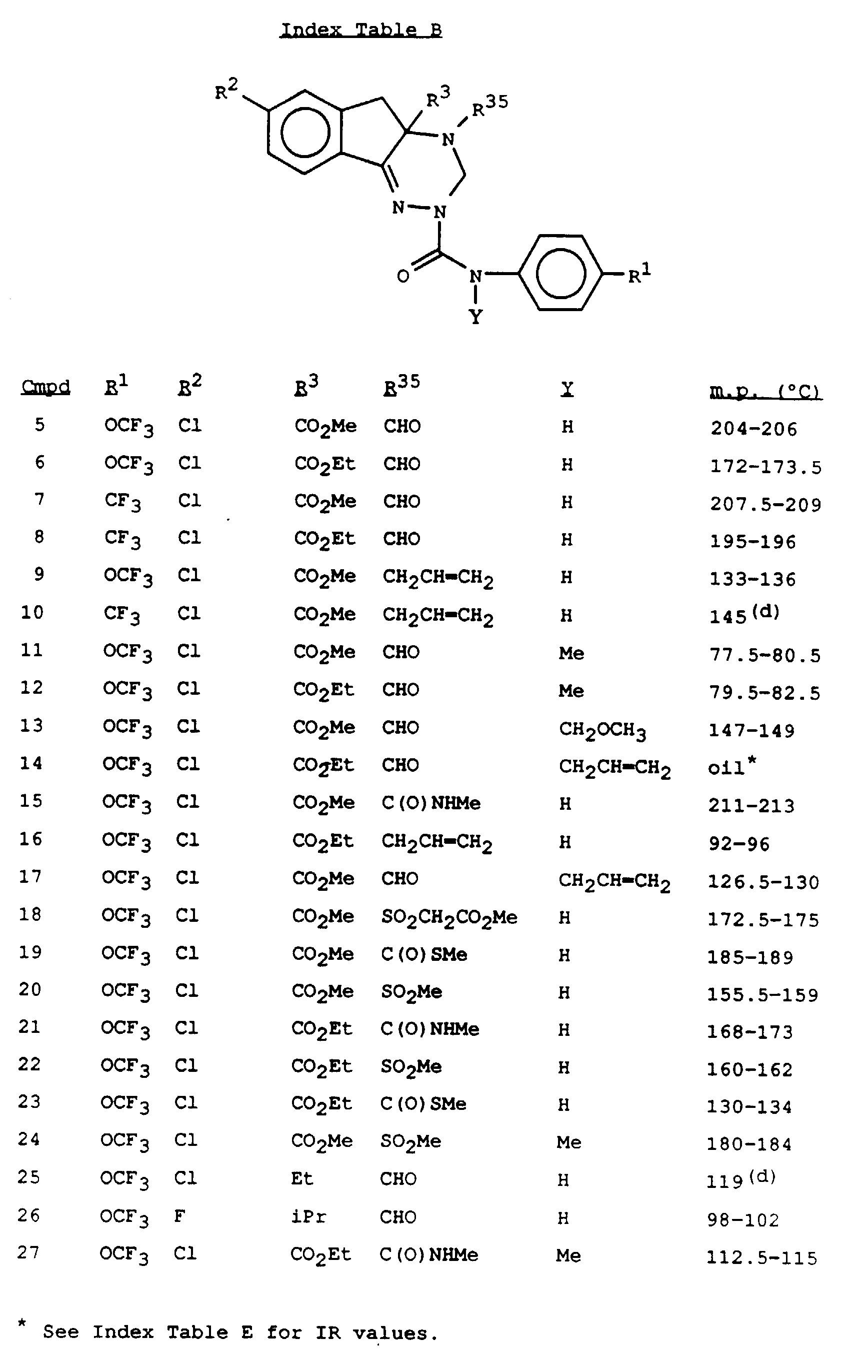

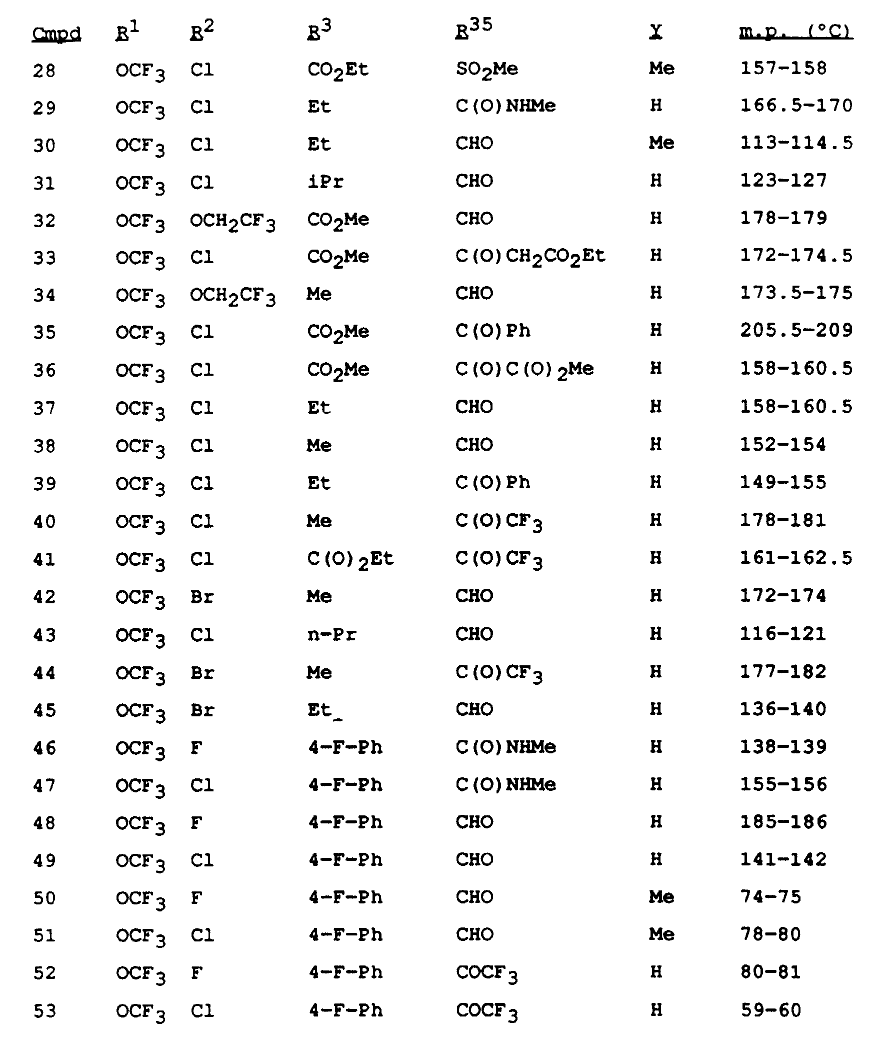

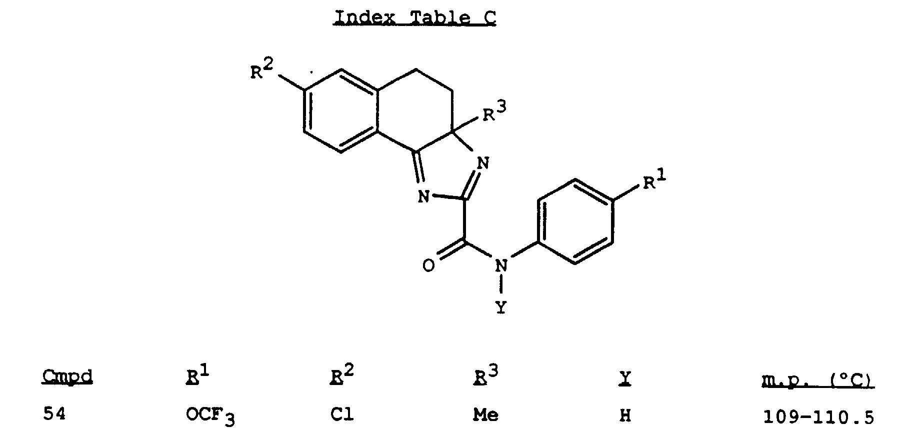

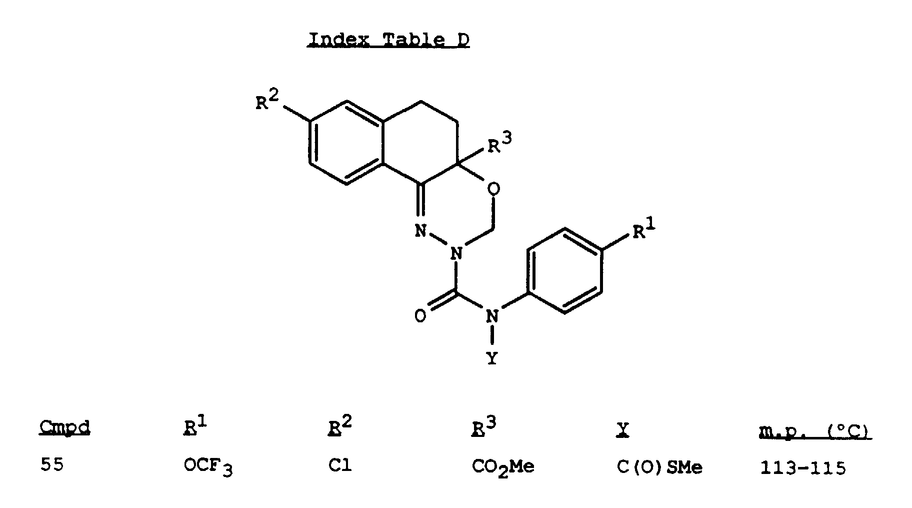

| | Index Table E |
|---|---|
| Cmpd | IR (neat) |
| 14 | 1743, 1678, 1605, 1509 cm⁻¹ |

### TEST A

### Fall Armyworm

Test units, each consisting of an 8-ounce (230 mL) plastic cup containing a layer of wheat germ diet, approximately 0.5 cm thick, were prepared. Ten third-instar larvae of fall armyworm (Spodoptera frugiperda) were placed into a cup. Solutions of each of the test compounds (acetone/distilled water 75/25 solvent) were sprayed into the cups, a single solution per set of three cups. Spraying was accomplished by passing the cups, on a conveyer belt, directly beneath a flat fan hydraulic nozzle which discharged the spray at a rate of 0.138 kg/ha of active ingredient per acre (about 0.125 pounds per hectare) at 30 p.s.i. (207 kPa). The cups were then covered and held at 27°C and 50% relative humidity for 72 hours, after which time readings were taken. Of the compounds tested, the following resulted in greater than or equal to 80% mortality: 1, 3*, 4*, 5, 6, 7, 8, 9, 10, 11, 12, 14, 15, 16, 17, 18, 19, 20, 21, 22, 23, 24, 25, 27,28, 29, 30, 31, 42 and 43.

### TEST B

### Tobacco Budworm

The test procedure of Test A was repeated for efficacy against third-instar larvae of the tobacco budworm (Heliothis virescens) except that mortality was assessed at 48 hours. Of the compounds tested, the following resulted in greater than or equal to 80% mortality: 3*, 4*, 7, 8, 9, 10, 11, 12, 15, 16, 19, 20, 21, 22, 23, 25, 27, 28, 29, 30, 31 and 42.
* Tested at 0.55 kg/ha.

### TEST C

### Southern Corn Rootworm

Test units, each consisting of an 8-ounce (230 mL) plastic cup containing 1 sprouted corn seed, were prepared. Sets of three test units were sprayed as described in Test A with individual solutions of the test compounds. After the spray on the cups had dried, five third-instar larvae of the southern corn rootworm (Diabrotica undecimpunctata howardi) were placed into each cup. A moistened dental wick was inserted into each cup to prevent drying and the cups were then covered. The cups were then held at 27°C and 50% relative humidity for 48 hours, after which time mortality readings were taken. Of the compounds tested, the following resulted in greater than or equal to 80% mortality: 5, 6, 7, 8, 9, 10, 11, 14, 15, 16, 17, 18, 19, 20, 21, 22, 23, 24, 25, 27, 28, 29, 30, 31, 42 and 43.

### TEST D

### Aster Leafhopper

Test units were prepared from a series of 12-ounce (350 mL) cups, each containing oat (Avena sativa) seedlings in a 1-inch (2.54 cm) layer of sterilized soil. The test units were sprayed as described in Test A with individual solutions of the below-listed compounds. After the oats had dried from the spraying, between 10 and 15 adult aster leafhoppers (Mascrosteles fascifrons) were aspirated into each of the covered cups. The cups were held at 27°C and 50% relative humidity for 48 hours, after which time mortality readings were taken. Of the compounds tested, the following resulted in greater than or equal to 80% mortality: 5, 7, 8, 9, 10, 11, 12, 25, 30, 31, 42 and 43.

### TEST E

### Boll Weevil

Five adult boll weevils (Anthonomus grandis) were placed into each of a series of 9 ounce (260 mL) cups. The test procedure employed was then otherwise the same as in Test A with three cups per treatment. Mortality readings were taken 48 hours after treatment. Of the compounds tested, the following resulted in greater than or equal to 80% mortality: 5, 6, 7, 8, 12, 17, 18, 19, 20, 21, 22, 23, 25, 27,28, 29, 30 and 42.

## Claims

1. A compound of the formula 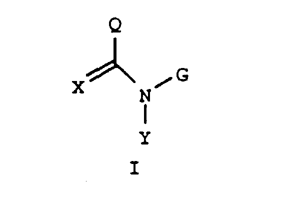 wherein
Q is selected from the group 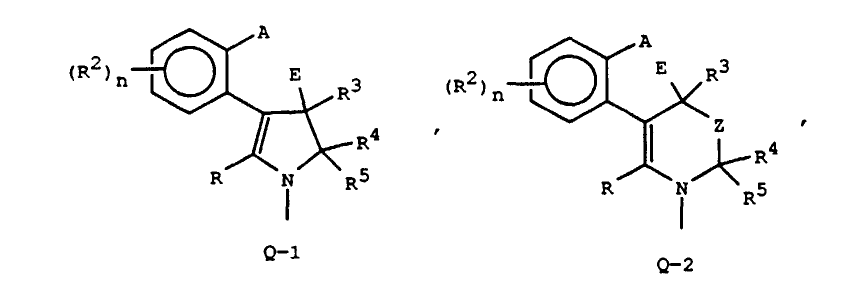 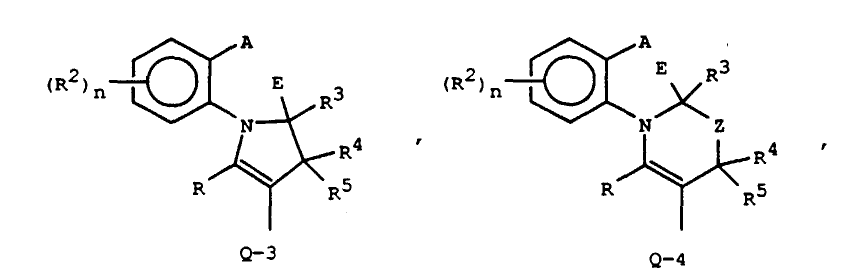 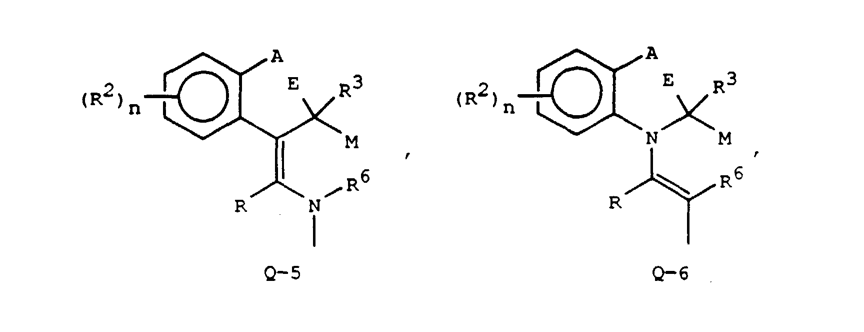 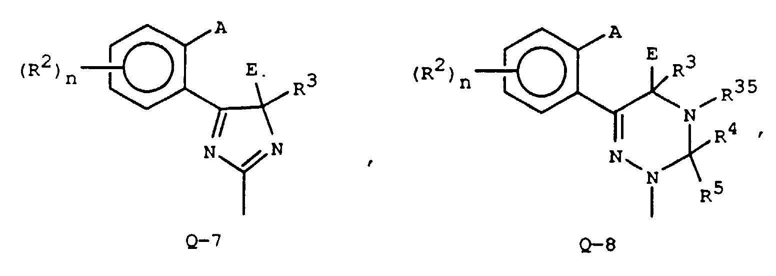 and 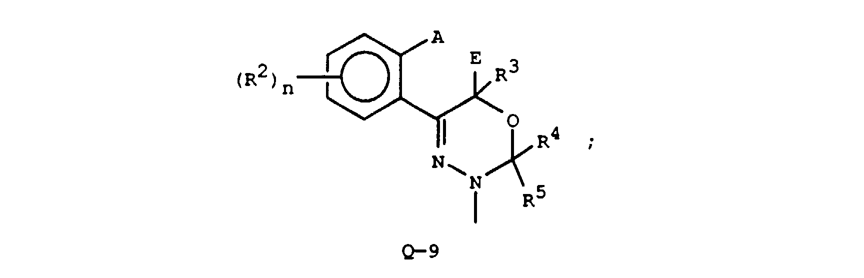
A is H;
E is selected from the group H and C₁-C₃ alkyl; E being other than H when Q is Q-7; or
A and E are taken together to form -CH₂-,-CH₂CH₂-, -O-, -S-, -S(O)-, -S(O)₂-, -NR⁷-, -OCH₂-, -SCH₂-, -N(R⁷)CH₂-, substituted -CH₂-, and substituted -CH₂CH₂-, the substituents independently selected from 1-2 halogen and 1-2 methyl;
M is selected from the group H and C₁-C₃ alkyl; or
E and M are taken together as -CH₂CH₂-, -CH₂CH₂CH₂- or -CH₂CH₂CH₂CH₂-, each group optionally substituted with one or more members independently selected from the group halogen, NO₂, CN, C₁-C₃ alkyl, C₁-C₃ haloalkyl, OH, OR⁶ and C(O)₂R¹⁹;
G is selected from the group 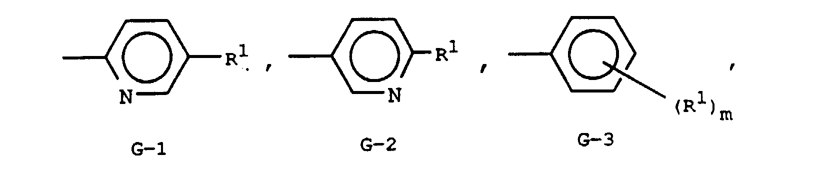 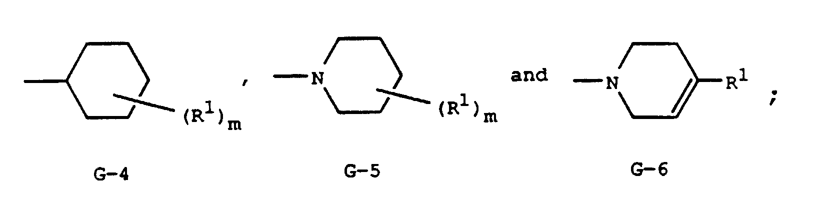
X is selected from the group O and S;
Y is selected from the group H; C₁-C₆ alkyl; benzyl; C₂-C₆ alkenyl; C₂-C₆ alkynyl; C₁-C₆ alkyl substituted by halogen, C₁-C₃ alkoxy, C₁-C₃ haloalkoxy, CN, NO₂, S(O)ᵣR³⁰, P(X)(OR²⁵)₂, C(O)R³⁰, O(O)₂R³⁰ and phenyl optionally substituted by halogen, CN, C₁-C₂ haloalkyl and C₁-C₂ haloalkoxy; C₃-C₆ cycloalkyl; C₃-C₆ halocycloalkyl; C₄-C₆ cycloalkylalkyl; CHO; C₂-C₆ alkylcarbonyl; C₂-C₆ alkoxycarbonyl; C₂-C₆ haloalkylcarbonyl; C(O)R³³; C(O)₂R³³; C(S)R²⁶; C(S)R³³; C(O)C(O)₂R²⁵; C(O)CH₂C(O)₂R²⁵; S(O)ᵣR³⁰; S(O)₂CH₂C(O)₂R²⁵; P(X)(OR²⁵)₂; phenylthio; R¹¹OC(O)N(R¹²)S-; R¹³(R¹⁴)NS-; N-CR⁹R¹⁰; OR⁸; NR⁸R⁹; and R³⁸; Y being R³⁸ when Q is Q-9; Y being other than N-CR⁹R¹⁰, OR⁸, and NR⁸R⁹ when Q is Q-8 and A and E are taken together as -CH₂-;
Z is selected from the group CH₂, O, S and NR²⁹;
R is selected from the group H, halogen, C₁-C₃ alkyl, C₁-C₃ alkoxy, CN, and NO₂;
R¹ and R² are independently selected from the group H, C₁-C₆ alkyl, C₁-C₆ haloalkyl, C₂-C₆ alkenyl, C₂-C₆ haloalkenyl, C₂-C₆ alkynyl, C₃-C₆ haloalkynyl, C₂-C₆ alkoxyalkyl, C₂-C₆ alkylthioalkyl, C₁-C₆ nitroalkyl, C₂-C₆ cyanoalkyl, C₃-C₈ alkoxycarbonylalkyl, C₃-C₆ cycloalkyl, C₃-C₆ halocycloalkyl, halogen, CN, N₃, SCN, NO₂, OR¹⁶, SR¹⁶, S(O)R¹⁶, S(O)₂R¹⁶, OC(O)R¹⁶, OS(O)₂R¹⁶, C(O)R¹⁶, C(O)₂R¹⁶, C(O)NR¹⁶R¹⁷, S(O)₂NR¹⁶R¹⁷, NR¹⁶R¹⁷, NR¹⁷C(O)R¹⁶, OC(O)NHR¹⁶, NR¹⁷C(O)NHR¹⁶, NR¹⁷S(O)₂R¹⁶, phenyl optionally substituted with 1 to 3 substituents independently selected from W, and benzyl optionally substituted with 1 to 3 substituents independently selected from W; or when m or n is 2, (R¹)₂ are taken together, or (R²)₂ are taken together as -OCH₂O-, -OCF₂O-, -OCH₂CH₂O-, -CH₂C(CH₃)₂O-, -CF₂CF₂O- or -OCF₂CF₂O- to form a cyclic bridge; provided that when R¹ or R² is S(O)R¹⁶, S(O)₂R¹⁶, OC(O)R¹⁶, NR¹⁷S(O)₂R¹⁶ or OS(O)₂R¹⁶ then R¹⁶ is other than H;
R³ is selected from the group H, J, N₃, NO₂, halogen, N(R²¹)R²², C(R³¹)=N-O-R³², C₁-C₆ alkyl, C₁-C₆ haloalkyl, C₂-C₆ alkenyl, C₂-C₆ haloalkenyl, C₂-C₆ alkynyl, C₂-C₆ alkoxyalkyl, C₃-C₈ alkoxycarbonylalkyl, C(O)R¹⁶, C(O)₂R¹⁶, OR¹⁸, C(O)NR¹⁶R¹⁷, C(S)NR¹⁶R¹⁷, C(S)R¹⁶, C(S)SR¹⁶, CN, Si(R²⁵)(R²⁶)(R²⁷), SR²⁵, S(O)R²⁵, S(O)₂R²⁵, P(O)(OR²⁵)₂, phenyl optionally substituted with (R¹⁵)ₚ, and benzyl optionally substituted with 1 to 3 substituents independently selected from W; or R³ is C₂-C₆ epoxyalkyl optionally substituted with one or more members independently selected from the group C₁-C₃ alkyl, CN, C(O)R²³, C(O)₂R²³, and phenyl optionally substituted with W; or R³ is C₁-C₆ alkyl substituted with one or more members independently selected from the group C(O)N(R²⁴)R³⁴, C(O)R²⁴, SR²⁵, S(O)R²⁵, S(O)₂R²⁵, SCN, CN, C₁-C₂ haloalkoxy, Si(R²⁵) (R²⁶) (R²⁷), N(R²¹)R²², NO₂, OC(O)R²⁴, P(O)(OR²⁵)₂, and J; R³ being other than H when Q is Q-7;
J is selected from the group saturated, partially unsaturated or aromatic 5- or 6-membered heterocyclic ring, bonded through carbon or nitrogen, containing 1-4 heteroatoms independently selected from the group consisting of 0-2 oxygen, 0-2 sulfur and 0-4 nitrogen, this substituent optionally containing one carbonyl and optionally substituted with one or more members independently selected from W;
R⁴ and R⁵ are independently selected from the group H, C₁-C₄ alkyl, C(O)R¹⁹ and C₂-C₄ alkoxycarbonyl;
R⁶ is selected from the group H, C₁-C₄ alkyl, C(O)R¹⁹ and C(O)₂R¹⁹;
R⁷ is selected from the group H, C₁-C₄ alkyl, C₁-C₄ haloalkyl, C₂-C₄ alkenyl, C₂-C₄ haloalkenyl, SR¹⁶, S(O)R¹⁶, S(O)₂R¹⁶, C(O)R¹⁶, C(O)₂R¹⁶, C(O)NR¹⁶R²⁰, C(S)NR¹⁶R²⁰, C(S)R¹⁶, C(S)OR¹⁶, P(O) (OR¹⁶)₂, P(S) (OR¹⁶)₂, P(O) (R¹⁶)OR¹⁶, P(O)(R¹⁶)SR²⁰, optionally substituted phenyl, and optionally substituted benzyl wherein the optional phenyl and benzyl substituents are independently selected from F, Cl, Br, CH₃, CF₃ or OCF₃; provided that when R⁷ is other than C(O)R¹⁶, C(O)NR¹⁶R²⁰ or C(S)NR¹⁶R²⁰ then R¹⁶ is other than H;
R⁸ is selected from the group H, C₁-C₄ alkyl, C₁-C₄ haloalkyl, C₂-C₄ alkenyl, C₂-C₄ alkynyl, S(O)₂NR¹⁷R¹⁸, S(O)₂R¹⁶, C(O)R¹⁶, C(O)₂R¹⁶, C(O)NR¹⁶R²⁰, phenyl optionally substituted with halogen or C₁-C₄ alkoxy, and benzyl optionally substituted with halogen; provided that when R⁸ is S(O)₂R¹⁶, R¹⁶ is other than H;
R⁹ is selected from the group H, C₁-C₄ alkyl and C(O)R¹⁶;
R¹⁰ is selected from the group H, C₁-C₄ alkyl, C₁-C₄ haloalkyl and phenyl optionally substituted with one or more members independently selected from the group halogen, CN, NO₂, CF₃ and OCF₃; or
R⁹ and R¹⁰ are taken together as -CH₂CH₂CH₂-, -CH₂CH₂CH₂CH₂- or -CH₂CH₂CH₂CH₂CH₂-;
R¹¹ is C₁-C₁₈ alkyl;
R¹² is C₁-C₄ alkyl;
R¹³ and R¹⁴ are independently C₁-C₄ alkyl; or
R¹³ and R¹⁴ are taken together as -CH₂CH₂CH₂CH₂CH₂- or -CH₂CH₂OCH₂CH₂-;
R¹⁵ is selected from the group C₁-C₆ alkyl, C₁-C₆ haloalkyl, C₂-C₆ alkenyl, C₂-C₆ haloalkenyl, C₂-C₆ alkynyl, C₃-C₆ haloalkynyl, C₂-C₆ alkoxyalkyl, C₂-C₆ alkylthioalkyl, C₁-C₆ nitroalkyl, C₂-C₆ cyanoalkyl, C₃-C₈ alkoxycarbonylalkyl, C₃-C₆ cycloalkyl, C₃-C₆ halocycloalkyl, halogen, CN, N₃, SCN, NO₂, O^{R16,} SR¹⁶, S(O)R¹⁶, S(O)₂R¹⁶, OC(O)R¹⁶, OS(O)₂R¹⁶, C(O)R¹⁶, C(O)₂R¹⁶, C(O)NR¹⁶R¹⁷, S(O)₂NR¹⁶R¹⁷, NR¹⁶R¹⁷, NR¹⁷C(O)R¹⁶, OC(O)NHR¹⁶, NR¹⁷C(O)NHR¹⁶, NR¹⁷S(O)₂R¹⁶, phenyl optionally substituted with 1 to 3 substituents independently selected from W, and benzyl optionally substituted with 1 to 3 substituents independently selected from W; or when p is 2, (R¹⁵)₂ are taken together as -OCH₂O-, -OCF₂O-, -OCH₂CH₂O-, -CH₂C(CH₃)₂O-, -CF₂CF₂O- or -OCF₂CF₂O- to form a cyclic bridge; provided that when R¹⁵ is S(O)R¹⁶, S(O)₂R¹⁶, OC(O)R¹⁶, NR¹⁷S(O)₂R¹⁶ or OS(O)₂R¹⁶ then R¹⁶ is other than H;
R¹⁶ is selected from the group H, C₁-C₆ alkyl, C₁-C₆ haloalkyl, C₂-C₆ alkenyl, C₂-C₆ haloalkenyl, C₂-C₆ alkynyl, C₃-C₆ haloalkynyl, C₂-C₆ alkoxyalkyl, C₂-C₆ alkylthioalkyl, C₁-C₆ nitroalkyl, C₂-C₆ cyanoalkyl, C₃-C₈ alkoxycarbonylalkyl, C₃-C₆ cycloalkyl, C₃-C₆ halocycloalkyl, and optionally substituted phenyl and optionally substituted benzyl wherein the optional phenyl and benzyl substituents are 1 to 3 substituents independently selected from W;
R¹⁷ is selected from the group H and C₁-C₄ alkyl; or
R¹⁶ and R¹⁷, when attached to the same atom, are taken together as -(CH₂)₄-, -(CH₂)₅-, or -CH₂CH₂OCH₂CH₂-;
R¹⁸ is selected from the group H, C₁-C₄ alkyl, C₂-C₄ alkenyl, C₂-C₄ alkynyl, C₂-C₄ alkylcarbonyl, C₂-C₄ alkoxycarbonyl and C₁-C₄ alkylsulfonyl;
R¹⁹ is C₁-C₃ alkyl;
R²⁰ is selected from the group H, C₁-C₄ alkyl, C₂-C₄ alkenyl and C₂-C₄ alkynyl;
R²¹ is selected from the group H, C₂-C₇ alkylcarbonyl, C₂-C₇ alkoxycarbonyl, optionally substituted C₁-C₄ alkyl, optionally substituted C₂-C₄ alkenyl, and optionally substituted C₂-C₄ alkynyl, all of these optional substituents being independently selected from C₁-C₂ alkoxy, CN, C(O)R²⁸ or C(O)₂R²⁵;
R²² is selected from the group H, C₁-C₃ alkyl, phenyl optionally substituted with at least one member independently selected from W, and benzyl optionally substituted with at least one member independently selected from W;
R²³ is selected from the group H, C₁-C₄ alkyl, C₂-C₄ alkenyl and C₂-C₄ alkynyl;
R²⁴ is selected from the group H and C₁-C₃ alkyl;
R²⁵ is selected from the group C₁-C₃ alkyl and phenyl optionally substituted with at least one member independently selected from W;
R²⁶ is selected from the group C₁-C₄ alkyl, C₁-C₄ haloalkyl, C₁-C₄ alkoxy, and C₁-C₄ haloalkoxy;
R²⁷ is C₁-C₃ alkyl;
R²⁸ is selected from the group H, C₁-C₃ alkyl and phenyl optionally substituted with at least one member independently selected from W;
R²⁹ is selected from the group H, CHO, C₁-C₄ alkyl, C₂-C₄ alkylcarbonyl and C₂-C₄ alkoxycarbonyl;
R³⁰ is selected from the group C₁-C₃ alkyl and C₁-C₃ haloalkyl;
R³¹ is selected from the group H, Cl, C₁-C₄ alkyl, C₁-C₄ alkoxy, C₁-C₂ alkylthio and CN;
R³² is selected from the group H, C₁-C₄ alkyl, C₂-C₃ alkylcarbonyl and C₂-C₃ alkoxycarbonyl;
R³³ is selected from the group C₁-C₄ alkylthio, C₁-C₄ haloalkylthio, and phenyl optionally substituted with at least one member independently selected from W;
R³⁴ is selected from the group H and C₁-C₂ alkyl;
R³⁵ is selected from the group CHO, C₁-C₄ alkyl substituted with substituents independently selected from the group halogen, C₁-C₂ alkoxy, C₁-C₂ haloalkoxy, CN, NO₂, C₂-C₄ alkylcarbonyl, C₂-C₄ alkoxycarbonyl or N(R³⁵)(R³⁶); or R³⁵ is C₂-C₆ haloalkylcarbonyl; C₃-C₆ alkenyl; C₃-C₆ haloalkenyl; C₃-C₆ alkynyl; C₃-C₆ haloalkynyl; C₃-C₆ cycloalkyl; C(S)R²⁶; C(S)R³³; C(O)C(O)₂R²⁵; C(O)CH₂C(O)₂R²⁵; S(O)ᵣR³⁰; S(O)₂CH₂C(O)₂R²⁵; P(X)(OR²⁵)₂; C(O)N(R³⁶)(R³⁷); S(O)ᵣN(R¹³)R¹⁴; S(O)ᵣN(R¹²)C(O)OR¹¹; S(O)ᵣN(R¹²)CHO; J; CH₂J; C(O)J; C(O)Ph where the phenyl group is optionally substituted by a group independently selected from W; and benzyl optionally substituted by a group independently selected from W;
R³⁶ is selected from the group H, C₁-C₄ alkyl, C₁-C₄ alkenyl, phenyl optionally substituted by a group independently selected from W and benzyl optionally substituted by a group independently selected from W;
R³⁷ is selected from the group C₁-C₄ alkyl and C₁-C₄ alkenyl;
R³⁸ is selected from the group C(S)R²⁶, C(S)R³³, C(O)C(O)₂R²⁵, C(O)CH₂C(O)₂R²⁵, S(O)R³⁰, S(O)₂R³⁰, S(O)₂CH₂C(O)₂R²⁵, P(X)(OR²⁵)₂, C₃-C₆ haloalkynyl, and C₁-C₆ alkyl substituted by P(X)(OR²⁵)₂;
W is selected from the group halogen, CN, NO₂, C₁-C₂ alkyl, C₁-C₂ haloalkyl, C₁-C₂ alkoxy, C₁-C₂ haloalkoxy, C₁-C₂ alkylthio, C₁-C₂ haloalkylthio, C₁-C₂ alkylsulfonyl, and C₁-C₂ haloalkylsulfonyl;
m is 1 to 3;
n is 1 to 3;
p is 1 to 3; and
r is 0, 1 or 2.

2. A compound according to Claim 1 wherein: J is selected from the group:

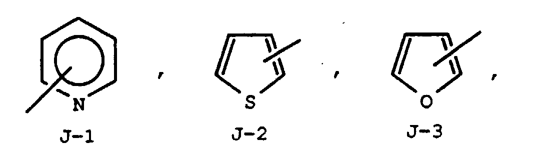

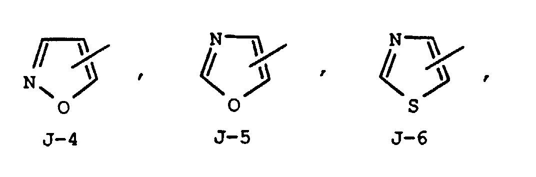

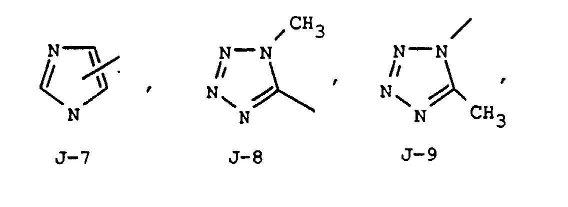

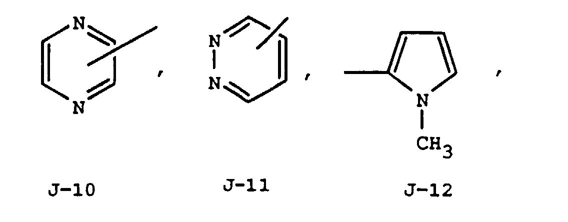

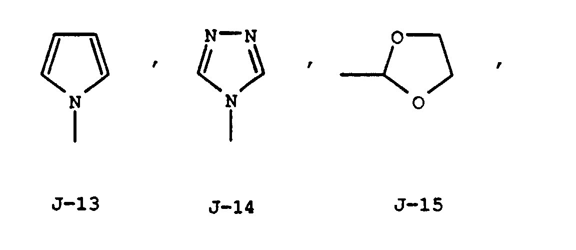

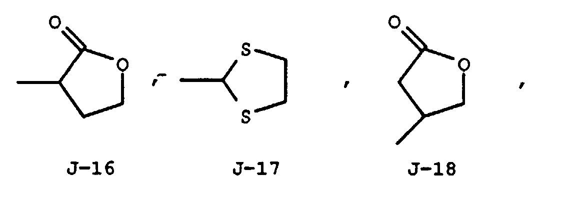

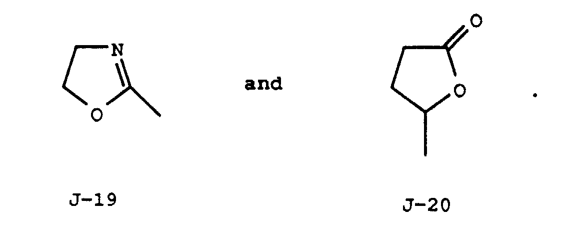

3. A compound according to Claim 1 wherein:
R¹ is selected from the group H, C₁-C₆ alkyl, C₁-C₆ haloalkyl, C₂-C₆ alkenyl, C₂-C₆ haloalkenyl, C₂-C₆ alkynyl, C₃-C₆ haloalkynyl, C₂-C₆ alkoxyalkyl, C₂-C₆ alkylthioalkyl, C₁-C₆ nitroalkyl, C₂-C₆ cyanoalkyl, C₃-C₈ alkoxycarbonylalkyl, C₃-C₆ cycloalkyl, C₃-C₆ halocycloalkyl, halogen, CN, SCN, NO₂, OR¹⁶, SR¹⁶, S(O)₂R¹⁶, C(O)R¹⁶, C(O)₂R¹⁶, phenyl optionally substituted with 1 to 3 substituents independently selected from W, and benzyl optionally substituted with 1 to 3 substituents independently selected from W; with one R¹ substituent in the 4-position, or when m is 2 then (R¹)₂ are taken together as -CH₂C(CH₃)₂O-, -OCH₂CH₂O-, -OCF₂CF₂O-, or -CF₂CF₂O- to form a 5- or 6-membered fused ring;
R² is selected from the group H, C₁-C₆ alkyl, C₁-C₆ haloalkyl, C₂-C₆ alkenyl, C₂-C₆ haloalkenyl, halogen, CN, SCN, NO₂, OR^{16,} SR¹⁶, S(O)₂R¹⁶, OC(O)R¹⁶, OS(O)₂R¹⁶, C(O)R¹⁶, C(O)₂R¹⁶, C(O)NR¹⁶R¹⁷, S(O)₂NR¹⁶R¹⁷, NR¹⁶R¹⁷, phenyl optionally substituted with 1 to 3 substituents independently selected from W, and benzyl optionally substituted with 1 to 3 substituents independently selected from W;
R³ is selected from the group H, C₁-C₄ alkyl, C₃-C₄ alkoxycarbonylalkyl, C(O)R¹⁶, C(O)₂R¹⁶, and phenyl independently substituted by one or more substituents selected from (R¹⁵)ₚ;
R¹⁵ is selected from the group C₁-C₆ alkyl, C₁-C₆ haloalkyl, C₂-C₆ alkenyl, C₂-C₆ haloalkenyl, halogen, CN, SCN, NO₂, OR¹⁶, SR¹⁶, S(O)₂R¹⁶, OC(O)R¹⁶, OS(O)₂R¹⁶, C(O)R¹⁶, C(O)₂R¹⁶, C(O)NR¹⁶R¹⁷, SO₂NR¹⁶R¹⁷, NR¹⁶R¹⁷, phenyl optionally substituted with 1 to 3 substituents independently selected from W, and benzyl optionally substituted with 1 to 3 substituents independently selected from W;
R¹⁶ is selected from the group C₁-C₄ alkyl, C₁-C₂ haloalkyl, C₃-C₄ alkenyl and propargyl;
R¹⁷ is selected from the group H and CH₃;
R³⁵ is selected from the group CHO, C₁-C₄ alkyl substituted with substituents independently selected from the group halogen, C₁-C₂ alkoxy, C₁-C₂ haloalkoxy, CN, NO₂, C₂-C₄ alkylcarbonyl, C₂-C₄ alkoxycarbonyl and N(R³⁵) (R³⁶); or R³⁵ is C₂-C₆ haloalkylcarbonyl; C₃-C₆ alkenyl; C₃-C₆ haloalkenyl; C₃-C₆ alkynyl; C(O)N(R³⁵)(R³⁶); R¹¹OC(O)N(R)¹²S-; R¹³(R¹⁴)NS-; C(O)Ph where the phenyl group is optionally substituted by a group independently selected from W; and benzyl optionally substituted by a group independently selected from W;
R³⁸ is selected from the group C(S)R²⁶, C(S)R³³, C(O)C(O)₂R²⁵, C(O)CH₂C(O)₂R²⁵, S(O)R³⁰, S(O)₂R³⁰, S(O)₂CH₂C(O)₂R²⁵, P(X)(OR²⁵)₂, C₃-C₆ haloalkynyl, and C₁-C₆ alkyl substituted by P(X)(OR²⁵)₂; and
m is 1 or 2.

4. A compound according to Claim 3 wherein G is G-3, R⁴ is H and R⁵ is H.

5. A compound according to Claim 4 wherein Q is selected from the group Q-1, Q-2, Q-7, Q-8 and Q-9.

6. A compound according to Claim 5 selected from the group:
3,4-bis(4-chlorophenyl)-4,5-dihydro-N-[4-(trifluoromethyl)phenyl]-1H-pyrrole-1-carboxamide,
methyl 2,3-dihydro-7-(trifluoromethyl)-2-[[[4-(trifluoromethyl)phenyl]amino]carbonyl]-[1] -benzopyrano[3,4-c]pyrrole-3a(4H)-carboxylate,
methyl 7-chloro-3,9-dihydro-3-[[[4-(trifluoromethoxy)phenyl]amino]carbonyl]-indeno[1,2-e]-1,3-oxazine-9a(2H)-carboxylate,
methyl 7-chloro-4-formyl-2,3,4,5-tetrahydro-2-[[4-(trifluoromethoxy)phenylamino]-carbonyl]-4aH-indeno[2,1-e]-1,2,4-triazine-4a-carboxylate, and
methyl 4-formyl-2,3,4,5-tetrahydro-7-(2,2,2-trifluoroethoxy)-2-[[4-(trifluoromethoxy)-phenylamino]carbonyl]-4aH-indeno[2,1-e]-1,2,4-triazine-4a-carboxylate.

7. An arthropodical composition comprising an arthropodically effective amount of a compound according to any one of Claims 1 to 6 and a carrier therefor.

8. A method for controlling arthropods comprising contacting the arthropods or their environment with an arthropodically effective amount of a compound according to any one of Claims 1 to 6.

## Patentansprüche

1. Verbindung der Formel 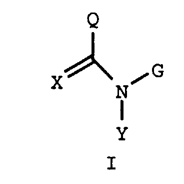 wobei
Q ausgewählt ist aus der Gruppe 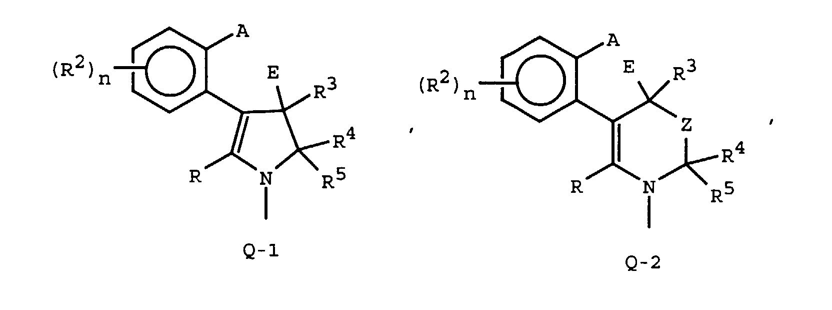 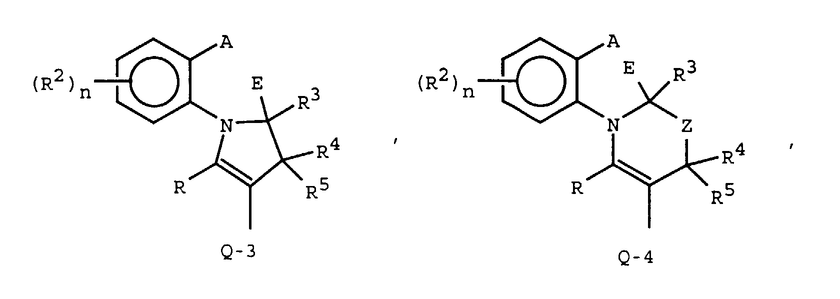 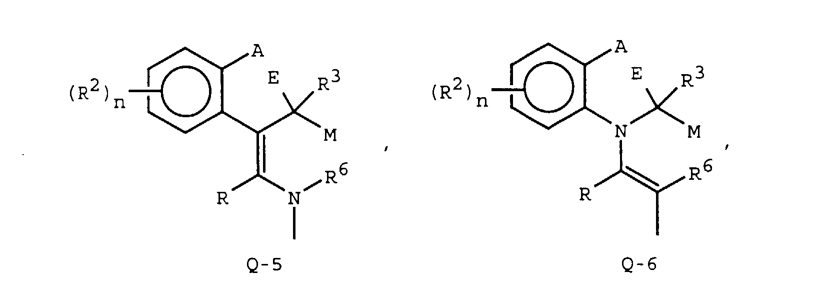 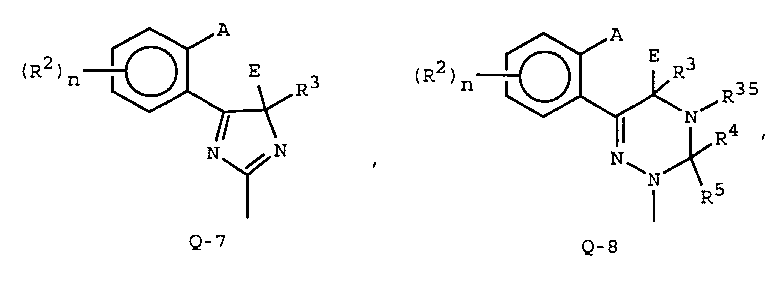 und 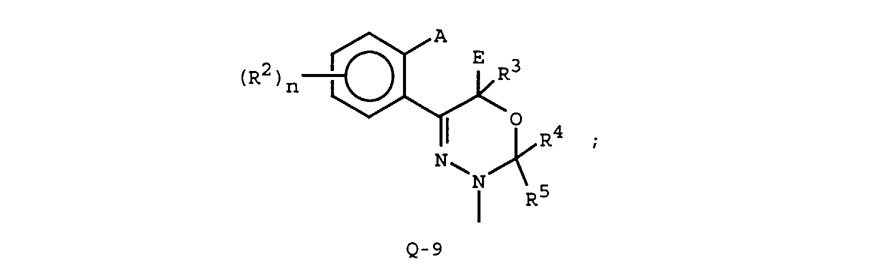
A H ist;
E ausgewählt ist aus der Gruppe H und C₁-C₃-Alkyl; wobei E anders als H ist, wenn Q Q-7 ist; oder
A und E zusammengenommen sind zur Bildung von -CH₂-, -CH₂CH₂-, -O-, -S-,-S(O)-, -S(O)₂-, -NR⁷-, -OCH₂-, -SCH₂-, -N(R⁷)CH₂-, substituiertem -CH₂- und substituiertem -CH₂CH₂-, wobei die Substituenten unabhängig aus 1-2 Halogen und 1-2 Methyl ausgewählt sind;
M aus der Gruppe H und C₁-C₃-Alkyl ausgewählt ist; oder
E und M zusammengenommen sind als -CH₂CH₂-, -CH₂CH₂CH₂- oder -CH₂CH₂CH₂CH₂-, wobei jede Gruppe gegebenenfalls substituiert ist mit einem oder mehreren Gliedern, unabhängig ausgewählt aus der Gruppe Halogen, NO₂, CN, C₁-C₃-Alkyl, C₁-C₃-Halogenalkyl, OH, OR⁶ und C(O)₂R¹⁹;
G ausgewählt ist aus der Gruppe 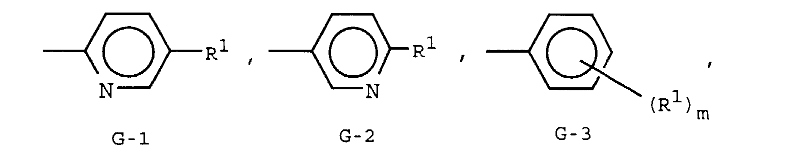 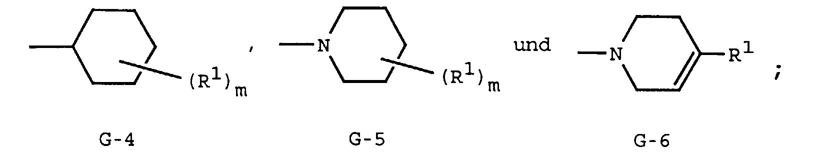
X aus der Gruppe O und S ausgewählt ist;
Y ausgewählt ist aus der Gruppe H; C₁-C₆-Alkyl; Benzyl; C₂-C₆-Alkenyl; C₂-C₆-Alkinyl; C₁-C₆-Alkyl, substituiert mit Halogen, C₁-C₃-Alkoxy, C₁-C₃-Halogenalkoxy, CN, NO₂, S(O)ᵣR³⁰, P(X)(OR²⁵)₂, C(O)R³⁰, C(O)₂R³⁰ und Phenyl, gegebenenfalls substituiert mit Halogen, CN, C₁-C₂-Halogenalkyl und C₁-C₂-Halogenalkoxy; C₃-C₆-Cycloalkyl; C₃-C₆-Halogencycloalkyl; C₄-C₆-Cycloalkylalkyl; CHO; C₂-C₆-Alkylcarbonyl; C₂-C₆-Alkoxycarbonyl; C₂-C₆-Halogenalkylcarbonyl; C(O)R³³; C(O)₂R³³; C(S)R²⁶; C(S)R³³; C(O)C(O)₂R²⁵; C(O)CH₂C(O)₂R²⁵; S(O)ᵣR³⁰; S(O)₂CH₂C(O)₂R²⁵; P(X)(OR²⁵)₂; Phenylthio; R¹¹OC(O)N(R¹²)S-; R¹³(R¹⁴)NS-; N=CR⁹R¹⁰; OR⁸; NR⁸R⁹; und R³⁸; wobei Y R³⁸ ist, wenn Q Q-9 ist; wobei Y anders ist als N=CR⁹R¹⁰, OR⁸ und NR⁸R⁹, wenn Q Q-8 ist und A und E als -CH₂- zusammengenommen sind;
Z aus der Gruppe CH₂, O, S und NR²⁹ ausgewählt ist;
R aus der Gruppe H, Halogen, C₁-C₃-Alkyl, C₁-C₃-Alkoxy, CN und NO₂ ausgewählt ist;
R¹ und R² unabhängig ausgewählt sind aus der Gruppe H, C₁-C₆-Alkyl, C₁-C₆-Halogenalkyl, C₂-C₆-Alkenyl, C₂-C₆-Halogenalkenyl, C₂-C₆-Alkinyl, C₃-C₆-Halogenalkinyl, C₂-C₆-Alkoxyalkyl, C₂-C₆-Alkylthioalkyl, C₁-C₆-Nitroalkyl, C₂-C₆-Cyanalkyl, C₃-C₈-Alkoxycarbonylalkyl, C₃-C₆-Cycloalkyl, C₃-C₆-Halogencycloalkyl, Halogen, CN, N₃, SCN, NO₂, OR¹⁶, SR¹⁶, S(O)R¹⁶, S(O)₂R¹⁶, OC(O)R¹⁶, OS(O)₂R¹⁶, C(O)R¹⁶, C(O)₂R¹⁶, C(O)NR¹⁶R¹⁷, S(O)₂NR¹⁶R¹⁷, NR¹⁶R¹⁷, NR¹⁷C(O)R¹⁶, OC(O)NHR¹⁶, NR¹⁷C(O)NHR¹⁶, NR¹⁷S(O)₂R¹⁶, Phenyl, gegebenenfalls substituiert mit 1 bis 3 Substituenten, unabhängig ausgewählt aus W, und Benzyl, gegebenenfalls substituiert mit 1 bis 3 Substituenten, unabhängig ausgewählt aus W; oder, wenn m oder n 2 ist, (R¹)₂ zusammengenommen sind oder (R²)₂ zusammengenommen sind als -OCH₂O-, -OCF₂O-, -OCH₂CH₂O-, -CH₂C(CH₃)₂O-, -CF₂CF₂O- oder -OCF₂CF₂O- zur Bildung einer cyclischen Brücke; mit der Maßgabe, daß, wenn R¹ oder R² S(O)R¹⁶, S(O)₂R¹⁶, OC(O)R¹⁶, NR¹⁷S(O)₂R¹⁶ oder OS(O)₂R¹⁶ ist, dann R¹⁶ anders als H ist;
R³ ausgewählt ist aus der Gruppe H, J, N₃, NO₂, Halogen, N(R²¹)R²², C(R³¹)=N-O-R³², C₁-C₆-Alkyl, C₁-C₆-Halogenalkyl, C₂-C₆-Alkenyl, C₂-C₆-Halogenalkenyl, C₂-C₆-Alkinyl, C₂-C₆-Alkoxyalkyl, C₃-C₈-Alkoxycarbonylalkyl, C(O)R¹⁶, C(O)₂R¹⁶, OR¹⁸, C(O)NR¹⁶R¹⁷, C(S)NR¹⁶R¹⁷, C(S)R¹⁶, C(S)SR¹⁶, CN, Si(R²⁵)(R²⁶)(R²⁷), SR²⁵, S(O)R²⁵, S(O)₂R²⁵, P(O)(OR²⁵)₂, Phenyl, gegebenenfalls substituiert mit (R¹⁵)ₚ, und Benzyl, gegebenenfalls substituiert mit 1 bis 3 Substituenten, unabhängig ausgewählt aus W; oder R³ ist C₂-C₆-Epoxyalkyl, gegebenenfalls substituiert mit einem oder mehreren Gliedern, unabhängig ausgewählt aus der Gruppe C₁-C₃-Alkyl, CN, C(O)R²³, C(O)₂R²³ und Phenyl, gegebenenfalls substituiert mit W; oder R³ ist C₁-C₆-Alkyl, substituiert mit einem oder mehreren Gliedern, unabhängig ausgewählt aus der Gruppe C(O)N(R²⁴)R³⁴, C(O)R²⁴, SR²⁵, S(O)R²⁵, S(O)₂R²⁵, SCN, CN, C₁-C₂-Halogenalkoxy, Si(R²⁵)(R²⁶)(R²⁷), N(R²¹)R²², NO₂, OC(O)R²⁴, P(O)(OR²⁵)₂ und J; wobei R³ anders als H ist, wenn Q Q-7 ist;
J ausgewählt ist aus der Gruppe gesättigter, teilweise ungesättigter oder aromatischer 5- oder 6-gliedriger heterocyclischer Ring, gebunden durch Kohlenstoff oder Stickstoff, enthaltend 1-4 Heteroatome, unabhängig ausgewählt aus der Gruppe, bestehend aus 0-2 Sauerstoff, 0-2 Schwefel und 0-4 Stickstoff, wobei dieser Substituent gegebenenfalls ein Carbonyl enthält und gegebenenfalls mit einem oder mehreren Gliedern, unabhängig ausgewählt aus W, substituiert ist;
R⁴ und R⁵ unabhängig aus der Gruppe H, C₁-C₄-Alkyl, C(O)R¹⁹ und C₂-C₄-Alkoxycarbonyl ausgewählt sind;
R⁶ aus der Gruppe H, C₁-C₄-Alkyl, C(O)R¹⁹ und C(O)₂R¹⁹ ausgewählt ist;
R⁷ ausgewählt ist aus der Gruppe H, C₁-C₄-Alkyl, C₁-C₄-Halogenalkyl, C₂-C₄-Alkenyl, C₂-C₄-Halogenalkenyl, SR¹⁶, S(O)R¹⁶, S(O)₂R¹⁶, C(O)R¹⁶, C(O)₂R¹⁶, C(O)NR¹⁶R²⁰, C(S)NR¹⁶R²⁰, C(S)R¹⁶, C(S)OR¹⁶, P(O)(OR¹⁶)₂, P(S)(OR¹⁶)₂, P(O)(R¹⁶)OR¹⁶, P(O)(R¹⁶)SR²⁰, gegebenenfalls substituiertes Phenyl und gegebenenfalls substituiertes Benzyl, wobei die wahlfreien Phenyl- und Benzylsubstituenten unabhängig ausgewählt sind aus F, Cl, Br, CH₃, CF₃ oder OCF₃; mit der Maßgabe, daß, wenn R⁷ anders als C(O)R¹⁶, C(O)NR¹⁶R²⁰ oder C(S)NR¹⁶R²⁰ ist, dann R¹⁶ anders als H ist;
R⁸ ausgewählt ist aus der Gruppe H, C₁-C₄-Alkyl, C₁-C₄-Halogenalkyl, C₂-C₄-Alkenyl, C₂-C₄-Alkinyl, S(O)₂NR¹⁷R¹⁸, S(O)₂R¹⁶, C(O)R¹⁶, C(O)₂R¹⁶, C(O)NR¹⁶R²⁰, Phenyl, gegebenenfalls substituiert mit Halogen oder C₁-C₄-Alkoxy, und Benzyl, gegebenenfalls substituiert mit Halogen; mit der Maßgabe, daß, wenn R⁸ S(O)₂R¹⁶ ist, R¹⁶ anders als H ist;
R⁹ aus der Gruppe H, C₁-C₄-Alkyl und C(O)R¹⁶ ausgewählt ist;
R¹⁰ ausgewählt ist aus der Gruppe H, C₁-C₄-Alkyl, C₁-C₄-Halogenalkyl und Phenyl, gegebenenfalls substituiert mit einem oder mehreren Gliedern, unabhängig ausgewählt aus der Gruppe Halogen, CN, NO₂, CF₃ und OCF₃; oder
R⁹ und R¹⁰ als -CH₂CH₂CH₂-, -CH₂CH₂CH₂CH₂- oder -CH₂CH₂CH₂CH₂CH₂- zusammengenommen sind;
R¹¹ C₁-C₁₈-Alkyl ist;
R¹² C₁-C₄-Alkyl ist;
R¹³ und R¹⁴ unabhängig C₁-C₄-Alkyl sind; oder
R¹³ und R¹⁴ als -CH₂CH₂CH₂CH₂CH₂- oder -CH₂CH₂OCH₂CH₂- zusammengenommen sind;
R¹⁵ ausgewählt ist aus der Gruppe C₁-C₆-Alkyl, C₁-C₆-Halogenalkyl, C₂-C₆-Alkenyl, C₂-C₆-Halogenalkenyl, C₂-C₆-Alkinyl, C₃-C₆-Halogenalkinyl, C₂-C₆-Alkoxyalkyl, C₂-C₆-Alkylthioalkyl, C₁-C₆-Nitroalkyl, C₂-C₆-Cyanalkyl, C₃-C₈-Alkoxycarbonylalkyl, C₃-C₆-Cycloalkyl, C₃-C₆-Halogencycloalkyl, Halogen, CN, N₃, SCN, NO₂, OR¹⁶, SR¹⁶, S(O)R¹⁶, S(O)₂R¹⁶, OC(O)R¹⁶, OS(O)₂R¹⁶, C(O)R¹⁶, C(O)₂R¹⁶, C(O)NR¹⁶R¹⁷, S(O)₂NR¹⁶R¹⁷, NR¹⁶R¹⁷, NR¹⁷C(O)R¹⁶, OC(O)NHR¹⁶, NR¹⁷C(O)NHR¹⁶, NR¹⁷S(O)₂R¹⁶, Phenyl, gegebenenfalls substituiert mit 1 bis 3 Substituenten, unabhängig ausgewählt aus W, und Benzyl, gegebenenfalls substituiert mit 1 bis 3 Substituenten, unabhängig ausgewählt aus W; oder, wenn p 2 ist, (R¹⁵)₂ als -OCH₂O-, -OCF₂O-, -OCH₂CH₂O-, -CH₂C(CH₃)₂O-, -CF₂CF₂O- oder -OCF₂CF₂O- zur Bildung einer cyclischen Brücke zusammengenommen sind; mit der Maßgabe, daß, wenn R¹⁵ S(O)R¹⁶, S(O)₂R¹⁶, OC(O)R¹⁶, NR¹⁷S(O)₂R¹⁶ oder OS(O)₂R¹⁶ ist, dann R¹⁶ anders als H ist;
R¹⁶ ausgewählt ist aus der Gruppe H, C₁-C₆-Alkyl, C₁-C₆-Halogenalkyl, C₂-C₆-Alkenyl, C₂-C₆-Halogenalkenyl, C₂-C₆-Alkinyl, C₃-C₆-Halogenalkinyl, C₂-C₆-Alkoxyalkyl, C₂-C₆-Alkylthioalkyl, C₁-C₆-Nitroalkyl, C₂-C₆-Cyanalkyl, C₃-C₈-Alkoxycarbonylalkyl, C₃-C₆-Cycloalkyl, C₃-C₆-Halogencycloalkyl und gegebenenfalls substituiertem Phenyl und gegebenenfalls substituiertem Benzyl, wobei die wahlfreien Phenyl- und Benzylsubstituenten 1 bis 3 Substituenten sind, unabhängig ausgewählt aus W;
R¹⁷ aus der Gruppe H und C₁-C₄-Alkyl ausgewählt ist; oder
R¹⁶ und R¹⁷, wenn an das gleiche Atom gebunden, als -(CH₂)₄-, -(CH₂)₅- oder -CH₂CH₂OCH₂CH₂- zusammengenommen sind;
R¹⁸ aus der Gruppe H, C₁-C₄-Alkyl, C₂-C₄-Alkenyl, C₂-C₄-Alkinyl, C₂-C₄-Alkylcarbonyl, C₂-C₄-Alkoxycarbonyl und C₁-C₄-Alkylsulfonyl ausgewählt ist;
R¹⁹ C₁-C₃-Alkyl ist;
R²⁰ aus der Gruppe H, C₁-C₄-Alkyl, C₂-C₄-Alkenyl und C₂-C₄-Alkinyl ausgewählt ist;
R²¹ ausgewählt ist aus der Gruppe H, C₂-C₇-Alkylcarbonyl, C₂-C₇-Alkoxycarbonyl, gegebenenfalls substituiertem C₁-C₄-Alkyl, gegebenenfalls substituiertem C₂-C₄-Alkenyl und gegebenenfalls substituiertem C₂-C₄-Alkinyl, wobei alle diese wahlfreien Substituenten unabhängig aus C₁-C₂-Alkoxy, CN, C(O)R²⁸ oder C(O)₂R²⁵ ausgewählt sind;
R²² ausgewählt ist aus der Gruppe H, C₁-C₃-Alkyl, Phenyl, gegebenenfalls substituiert mit mindestens einem Glied, unabhängig ausgewählt aus W, und Benzyl, gegebenenfalls substituiert mit mindestens einem Glied, unabhängig ausgewählt aus W;
R²³ aus der Gruppe H, C₁-C₄-Alkyl, C₂-C₄-Alkenyl und C₂-C₄-Alkinyl ausgewählt ist;
R²⁴ aus der Gruppe H und C₁-C₃-Alkyl ausgewählt ist;
R²⁵ ausgewählt ist aus der Gruppe C₁-C₃-Alkyl und Phenyl, gegebenenfalls substituiert mit mindestens einem Glied, unabhängig ausgewählt aus W;
R²⁶ aus der Gruppe C₁-C₄-Alkyl, C₁-C₄-Halogenalkyl, C₁-C₄-Alkoxy, und C₁-C₄-Halogenalkoxy ausgewählt ist;
R²⁷ C₁-C₃-Alkyl ist;
R²⁸ ausgewählt ist aus der Gruppe H, C₁-C₃-Alkyl und Phenyl, gegebenenfalls substituiert mit mindestens einem Glied, unabhängig ausgewählt aus W;
R²⁹ aus der Gruppe H, CHO, C₁-C₄-Alkyl, C₂-C₄-Alkylcarbonyl und C₂-C₄-Alkoxycarbonyl ausgewählt ist;
R³⁰ aus der Gruppe C₁-C₃-Alkyl und C₁-C₃-Halogenalkyl ausgewählt ist;
R³¹ aus der Gruppe H, Cl, C₁-C₄-Alkyl, C₁-C₄-Alkoxy, C₁-C₂-Alkylthio und CN ausgewählt ist;
R³² aus der Gruppe H, C₁-C₄-Alkyl, C₂-C₃-Alkylcarbonyl und C₂-C₃-Alkoxycarbonyl ausgewählt ist;
R³³ ausgewählt ist aus der Gruppe C₁-C₄-Alkylthio, C₁-C₄-Halogenalkylthio und Phenyl, gegebenenfalls substituiert mit mindestens einem Glied, unabhängig ausgewählt aus W;
R³⁴ aus der Gruppe H und C₁-C₂-Alkyl ausgewählt ist;
R³⁵ ausgewählt ist aus der Gruppe CHO, C₁-C₄-Alkyl, substituiert mit Substituenten, unabhängig ausgewählt aus der Gruppe Halogen, C₁-C₂-Alkoxy, C₁-C₂-Halogenalkoxy, CN, NO₂, C₂-C₄-Alkylcarbonyl, C₂-C₄-Alkoxycarbonyl oder N(R³⁵)(R³⁶); oder R³⁵ ist C₂-C₆-Halogenalkylcarbonyl; C₃-C₆-Alkenyl; C₃-C₆-Halogenalkenyl; C₃-C₆-Alkinyl; C₃-C₆-Halogenalkinyl; C₃-C₆-Cycloalkyl; C(S)R²⁶; C(S)R³³; C(O)C(O)₂R²⁵; C(O)CH₂C(O)₂R²⁵; S(O)ᵣR³⁰; S(O)₂CH₂C(O)₂R²⁵; P(X)OR²⁵)₂; C(O)N(R³⁶)(R³⁷); S(O)ᵣN(R¹³)R¹⁴; S(O)ᵣN(R¹²)C(O)OR¹¹; S(O)ᵣN(R¹²)CHO; J; CH₂J; C(O)J; C(O)Ph, wobei die Phenylgruppe gegebenenfalls substituiert ist mit einer Gruppe, unabhängig ausgewählt aus W; und Benzyl, gegebenenfalls substituiert mit einer Gruppe, unabhängig ausgewählt aus W;
R³⁶ ausgewählt ist aus der Gruppe H, C₁-C₄-Alkyl, C₁-C₄-Alkenyl, Phenyl, gegebenenfalls substituiert mit einer Gruppe, unabhängig ausgewählt aus W, und Benzyl, gegebenenfalls substituiert mit einer Gruppe, unabhängig ausgewählt aus W;
R³⁷ aus der Gruppe C₁-C₄-Alkyl und C₁-C₄-Alkenyl ausgewählt ist;
R³⁸ ausgewählt ist aus der Gruppe C(S)R²⁶, C(S)R³³, C(O)C(O)₂R²⁵, C(O)CH₂C(O)₂R²⁵, S(O)R³⁰, S(O)₂R³⁰, S(O)₂CH₂C(O)₂R²⁵, P(X)(OR²⁵)₂, C₃-C₆-Halogenalkinyl und C₁-C₆-Alkyl, substituiert mit P(X)(OR²⁵)₂;
W aus der Gruppe Halogen, CN, NO₂, C₁-C₂-Alkyl, C₁-C₂-Halogenalkyl, C₁-C₂-Alkoxy, C₁-C₂-Halogenalkoxy, C₁-C₂-Alkylthio, C₁-C₂-Halogenalkylthio, C₁-C₂-Alkylsulfonyl und C₁-C₂-Halogenalkylsulfonyl ausgewählt ist;
m 1 bis 3 ist;
n 1 bis 3 ist;
p 1 bis 3 ist; und
r 0, 1 oder 2 ist.

2. Verbindung nach Anspruch 1, wobei:
J ausgewählt ist aus der Gruppe:

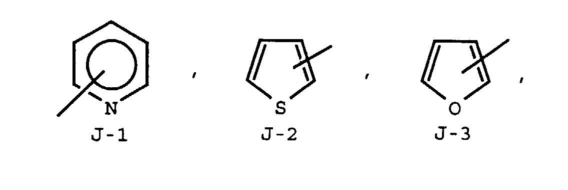

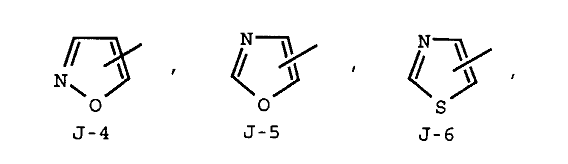

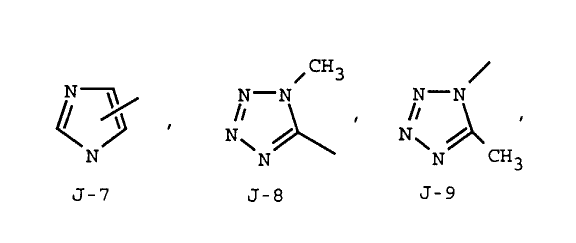

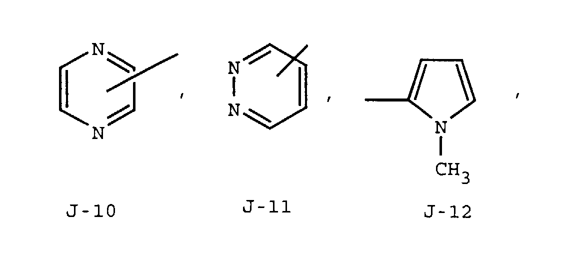

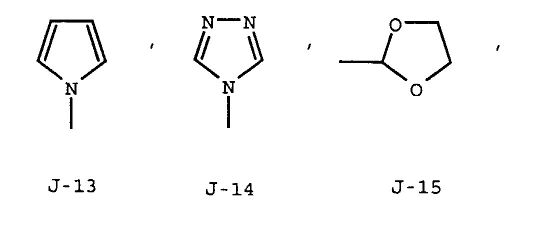

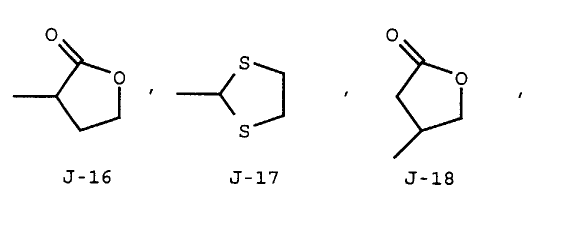

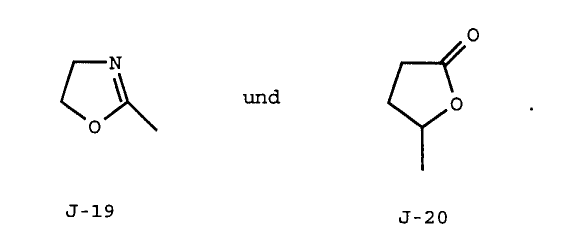

3. Verbindung nach Anspruch 1, wobei:
R¹ ausgewählt ist aus der Gruppe H, C₁-C₆-Alkyl, C₁-C₆-Halogenalkyl, C₂-C₆-Alkenyl, C₂-C₆-Halogenalkenyl, C₂-C₆-Alkinyl, C₃-C₆-Halogenalkinyl, C₂-C₆-Alkoxyalkyl, C₂-C₆-Alkylthioalkyl, C₁-C₆-Nitroalkyl, C₂-C₆-Cyanalkyl, C₃-C₈-Alkoxycarbonylalkyl, C₃-C₆-Cycloalkyl, C₃-C₆-Halogencycloalkyl, Halogen, CN, SCN, NO₂, OR¹⁶, SR¹⁶, S(O)₂R¹⁶, C(O)R¹⁶, C(O)₂R¹⁶, Phenyl, gegebenenfalls substituiert mit 1 bis 3 Substituenten, unabhängig ausgewählt aus W, und Benzyl, gegebenenfalls substituiert mit 1 bis 3 Substituenten, unabhängig ausgewählt aus W; mit einem R¹-Substituenten in der 4-Position, oder, wenn m 2 ist, dann (R¹)₂ zusammengenommen sind als -CH₂C(CH₃)₂O-, -OCH₂CH₂O-, -OCF₂CF₂O- oder -CF₂CF₂O- zur Bildung eines 5- oder 6-gliedrigen kondensierten Ringes;
R² ausgewählt ist aus der Gruppe H, C₁-C₆-Alkyl, C₁-C₆-Halogenalkyl, C₁-C₆-Alkenyl, C₂-C₆-Halogenalkenyl, Halogen, CN, SCN, NO₂, OR¹⁶, SR¹⁶, S(O)₂R¹⁶, OC(O)R¹⁶, OS(O)₂R¹⁶, C(O)R¹⁶, C(O)₂R¹⁶, C(O)NR¹⁶R¹⁷, S(O)₂NR¹⁶R¹⁷, NR¹⁶R¹⁷, Phenyl, gegebenenfalls substituiert mit 1 bis 3 Substituenten, unabhängig ausgewählt aus W, und Benzyl, gegebenenfalls substituiert mit 1 bis 3 Substituenten, unabhängig ausgewählt aus W;
R³ ausgewählt ist aus der Gruppe H, C₁-C₄-Alkyl, C₃-C₄-Alkoxycarbonylalkyl, C(O)R¹⁶, C(O)₂R¹⁶ und Phenyl, unabhängig substituiert mit einem oder mehreren Substituenten, ausgewählt aus (R¹⁵)ₚ;
R¹⁵ ausgewählt ist aus der Gruppe C₁-C₆-Alkyl, C₁-C₆-Halogenalkyl, C₂-C₆-Alkenyl, C₂-C₆-Halogenalkenyl, Halogen, CN, SCN, NO₂, OR¹⁶, SR¹⁶, S(O)₂R¹⁶, OC(O)R¹⁶, OS(O)₂R¹⁶, C(O)R¹⁶, C(O)₂R¹⁶, C(O)NR¹⁶R¹⁷, SO₂NR¹⁶R¹⁷, NR¹⁶R¹⁷, Phenyl, gegebenenfalls substituiert mit 1 bis 3 Substituenten, unabhängig ausgewählt aus W, und Benzyl, gegebenenfalls substituiert mit 1 bis 3 Substituenten, unabhängig ausgewählt aus W;
R¹⁶ aus der Gruppe C₁-C₄-Alkyl, C₁-C₂-Halogenalkyl, C₃-C₄-Alkenyl und Propargyl ausgewählt ist;
R¹⁷ aus der Gruppe H und CH₃ ausgewählt ist;
R³⁵ ausgewählt ist aus der Gruppe CHO, C₁-C₄-Alkyl, substituiert mit Substituenten, unabhängig ausgewählt aus der Gruppe Halogen, C₁-C₂-Alkoxy, C₁-C₂-Halogenalkoxy, CN, NO₂, C₂-C₄-Alkylcarbonyl, C₂-C₄-Alkoxycarbonyl und N(R³⁵)(R³⁶); oder R³⁵ ist C₂-C₆-Halogenalkylcarbonyl; C₃-C₆-Alkenyl; C₃-C₆-Halogenalkenyl; C₃-C₆-Alkinyl; C(O)N(R³⁵)(R³⁶); R¹¹OC(O)N(R¹²)S-; R¹³(R¹⁴)NS-; C(O)Ph, wobei die Phenylgruppe gegebenenfalls substituiert ist mit einer Gruppe, unabhängig ausgewählt aus W; und Benzyl, gegebenenfalls substituiert mit einer Gruppe, unabhängig ausgewählt aus W;
R³⁸ ausgewählt ist aus der Gruppe C(S)R²⁶, C(S)R³³, C(O)C(O)₂R²⁵, C(O)CH₂C(O)₂R²⁵, S(O)R³⁰, S(O)₂R³⁰, S(O)₂CH₂C(O)₂R²⁵, P(X)(OR²⁵)₂, C₃-C₆-Halogenalkinyl und C₁-C₆-Alkyl, substituiert mit P(X)(OR²⁵)₂; und
m 1 oder 2 ist.

4. Verbindung nach Anspruch 3, wobei G G-3 ist, R⁴ H ist und R⁵ H ist.

5. Verbindung nach Anspruch 4, wobei Q aus der Gruppe Q-1, Q-2, Q-7, Q-8 und Q-9 ausgewählt ist.

6. Verbindung nach Anspruch 5, ausgewählt aus der Gruppe:
3,4-Bis(4-chlorphenyl)4,5-dihydro-N-[4-(trifluormethyl)phenyl]-1H-pyrrol-1-carboxamid,
Methyl-2,3-dihydro-7-(trifluormethyl)-2-[[[4-(trifluormethyl)phenyl]amino]carbonyl]-[1]-benzopyrano[3,4-c]pyrrol-3a(4H)-carboxylat,
Methyl-7-chlor-3,9-dihydro-3-[[[4-(trifluormethoxy)phenyl]amino]carbonyl]-indeno[1,2-e]-1,3-oxazin-9a(2H)-carboxylat,
Methyl-7-chlor-4-formyl-2,3,4,5-tetrahydro-2-[[4-(trifluormethoxy)-phenylamino]carbonyl]-4aH-indeno[2,1-e]-1,2,4-triazin-4a-carboxylat und
Methyl-4-formyl-2,3,4,5-tetrahydro-7-(2,2,2-trifluorethoxy)-2-[[4-(trifluor-methoxy)phenylarnino]carbonyl]-4aH-indeno[2,1-e]-1,2,4-triazin-4a-carboxylat.

7. Arthropodische Zusammensetzung, umfassend eine arthropodisch wirksame Menge einer Verbindung nach einem der Ansprüche 1 bis 6 und einen Träger dafür.

8. Verfahren zur Bekämpfung von Arthropoden, umfassend das Inkontaktbringen der Arthropoden oder ihres Umfeldes mit einer arthropodisch wirksamen Menge einer Verbindung nach einem der Ansprüche 1 bis 6.

## Revendications

1. Un composé de formule : 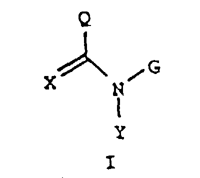 dans lequel :
Q est choisi dans le groupe : 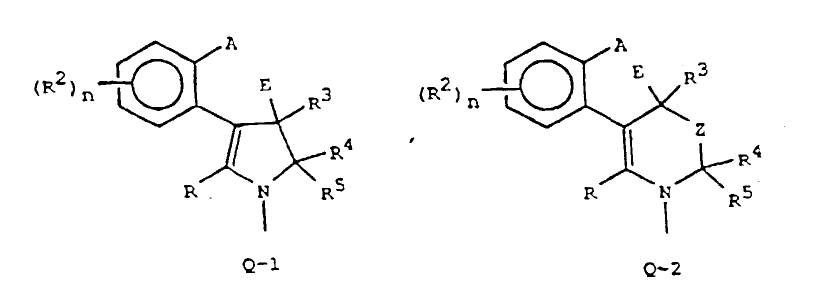 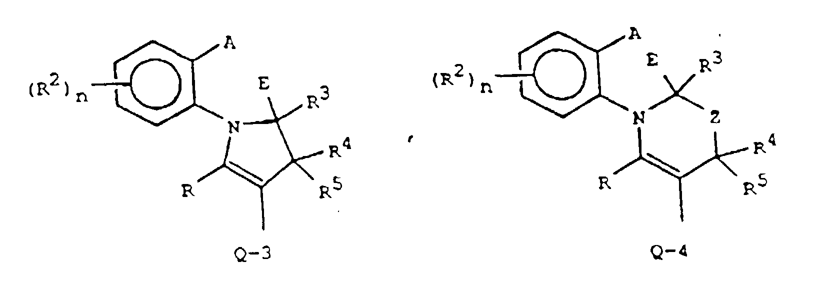 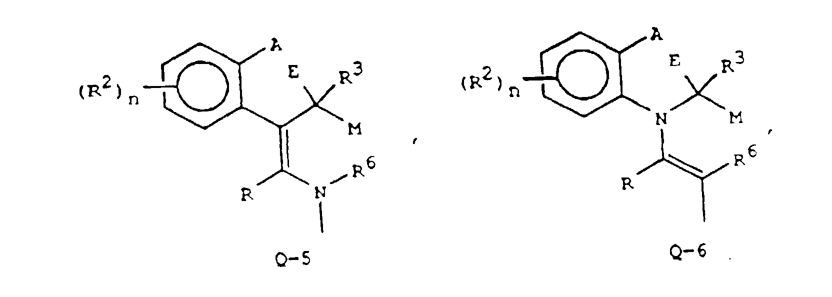 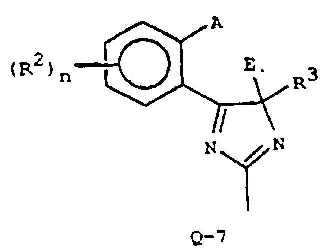 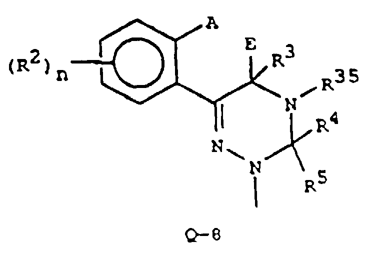 et 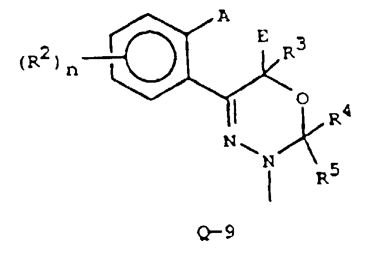
A est un atome d'hydrogène;
E est choisi dans le groupe formé par atome d'hydrogène et groupe alkyle en C₁₋₃; E étant autre qu'un atome d'hydrogène lorsque Q est Q-7; ou
A et E sont pris ensemble pour former -CH₂-, -CH₂CH₂-, -O-, -S-, -S(O)-, -S(O)₂-, -NR⁷-, -OCH₂-, -SCH₂-,-N(R⁷)CH₂-, -CH₂- substitué et -CH₂CH₂- substitué, les substituants étant indépendamment choisis parmi 1 à 2 atomes d'halogène et 1 à 2 groupes méthyle;
M est choisi dans le groupe formé par atome d'hydrogène et groupe alkyle en C₁₋₃; ou
E et M sont pris ensemble pour former -CH₂CH₂-, -CH₂CH₂CH₂- ou -CH₂CH₂CH₂CH₂-, chaque groupe étant éventuellement substitué par un ou plusieurs substituants indépendamment choisis dans le groupe formé par atome d'halogène, groupe NO₂; CN, alkyle en C₁₋₃, haloalkyle en C₁₋₃, OH, OR⁶ et C(O)₂R¹⁹;
G est choisi dans le groupe consistant en : 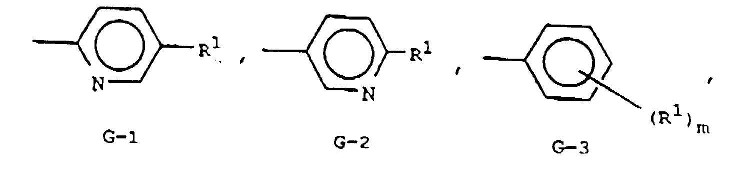 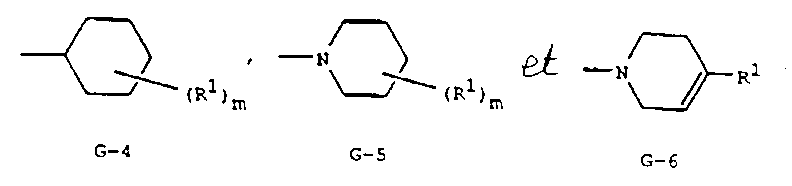
X est choisi dans le groupe formé par O et S;
Y est choisi dans le groupe formé par atome d'hydrogène, groupes alkyle en C₁₋₆; benzyle; alcényle en C₂₋₆; alcynyle en C₂₋₆; alkyle en C₁₋₆ substitué par un atome d'halogène, un groupe alcoxy en C₁₋₃, haloalcoxy en C₁₋₃, CN, NO₂, S(O)ᵣR³⁰, P(X)(OR²⁵)₂, C(O)R³⁰, C(O)₂R³⁰ et phényle éventuellement substitué par un atome d'halogène, un groupe CN, haloalkyle en C₁₋₂ et haloalcoxy en C₁₋₂; cycloalkyle en C₃₋₆, halocycloalkyle en C₃₋₆, cycloalkylalkyle en C₄₋₆; CHO; alkylcarbonyle en C₂₋₆; alcoxycarbonyle en C₂₋₆; haloalkylcarbonyle en C₂₋₆; C(O)R³³; C(O)₂R³³; C(S)R²⁶; C(S)R³³; C(O)C(O)₂R²⁵; C(O)CH₂C(O)₂R²⁵; S(O)ᵣR³⁰; S(O)₂CH₂C(O)₂R²⁵; P(X)(OR²⁵)₂; phénylthio; R¹¹OC(O)N(R¹²)S-; R¹³(R¹⁴)NS-; N=CR⁹R¹⁰; OR⁸; NR⁸R⁹; et R³⁸; Y étant R³⁸ lorsque Q est Q-9; Y étant autre que N=CR⁹R¹⁰, OR⁸ et NR⁸R⁹ lorsque Q est Q-8 et A et E sont pris ensemble pour former -CH₂-;
Z est choisi dans le groupe formé par CH₂, O, S et NR²⁹;
R est choisi dans le groupe formé par atome d'hydrogène, d'halogène, groupe alkyle en C₁₋₃, alcoxy en C₁₋₃, CN et NO₂;
R¹ et R² sont choisis indépendamment dans le groupe formé par atome d'hydrogène, groupe alkyle en C₁₋₆, haloalkyle en C₁₋₆, alcényle en C₂₋₆, haloalcényle en C₂₋₆, alcynyle en C₂₋₆, haloalcynyle en C₃₋₆, alcoxyalkyle en C₂₋₆, alkylthioalkyle en C₂₋₆, nitroalkyle en C₁₋₆, cyanoalkyle en C₂₋₆, alcoxycarbonylalkyle en C₃₋₈, cycloalkyle en C₃₋₆, halocycloalkyle en C₃₋₆, atome d'halogène groupe CN, N₃, SCN, NO₂, OR¹⁶, SR¹⁶, S(O)R¹⁶, S(O)₂R¹⁶, OC(O)R¹⁶, OS(O)₂R¹⁶, C(O)R¹⁶, C(O)₂R¹⁶, C(O)NR¹⁶R¹⁷, S(O)₂NR¹⁶R¹⁷, NR¹⁶R¹⁷, NR¹⁷C(O)R¹⁶, OC(O)NHR¹⁶, NR¹⁷C(O)NHR¹⁶, NR¹⁷S(O)₂R¹⁶, phényle éventuellement substitué par 1 à 3 substituants choisis indépendamment parmi W, et benzyle éventuellement substitué par 1 à 3 substituants choisis indépendamment parmi W; ou lorsque m ou n est 2, (R¹)₂ sont pris ensemble, ou (R²)₂ sont pris ensemble pour former -OCH₂O-, -OCF₂O-, -OCH₂CH₂O-, -CH₂C(CH₃)₂O-. -CF₂CF₂O- ou -OCF₂CF₂O- pour former un pont cyclique; à condition que lorsque R¹ ou R² est S(O)R¹⁶, S(O)₂R¹⁶, OC(O)R¹⁶, NR¹⁷S(O)₂R¹⁶ ou OS(O)₂R¹⁶, R¹⁶ soit alors autre qu'un atome d'hydrogène;
R³ est choisi dans le groupe formé par : atome d'hydrogène, groupe J, N₃. NO₂, atome d'halogène, groupe N(R²¹)R²², C(R³¹)=N-O-R³², alkyle en C₁₋₆, haloalkyle en C₁₋₆, alcényle en C₂₋₆, haloalcényle en C₂₋₆, alcynyle en C₂₋₆, alcoxyalkyle en C₂₋₆, alcoxycarbonylalkyle en C₃₋₈, C(O)R¹⁶, C(O)₂R¹⁶, OR¹⁸, C(O)NR¹⁶R¹⁷, C(S)NR¹⁶R¹⁷, C(S)R¹⁶, C(S)SR¹⁶, CN, Si(R²⁵)(R²⁶)(R²⁷), SR²⁵, S(O)R²⁵, S(O)₂R²⁵, P(O)(OR²⁵)₂, phényle éventuellement substitué par (R¹⁵)ₚ et benzyle éventuellement substitué par 1 à 3 substituants choisis indépendamment parmi W; ou R³ est un groupe époxyalkyle en C₂₋₆ éventuellement substitué par un ou plusieurs substituants choisis indépendamment dans le groupe consistant en groupe alkyle en C₁₋₃, CN, C(O)R²³, C(O)₂R²³ et phényle éventuellement substitué par W; ou R³ est groupe alkyle en C₁₋₆ substitué par un ou plusieurs substituants choisis indépendamment dans le groupe consistant en C(O)N(R²⁴)R³⁴, C(O)R²⁴, SR²⁵, S(O)R²⁵, S(O)₂R²⁵, SCN, CN, haloalcoxy en C₁₋₂, Si(R²⁵)(R²⁶)(R²⁷), N(R²¹)R²², NO₂, OC(O)R²⁴, P(O)(OR²⁵)₂, et J; R³ étant autre qu'un atome d'hydrogène lorsque Q est Q-7;
J est choisi dans le groupe formé par : noyau hétérocyclique saturé, partiellement insaturé ou aromatique de 5 à 6 chaînons, lié par un atome de carbone ou d'azote, contenant 1 à 4 hétéroatomes choisis indépendamment dans le groupe consistant en 0 à 2 atomes d'oxygène, 0 à 2 atomes de soufre et 0 à 4 atomes d'azotc. ce substituant contenant éventuellement un groupe carbonyle et étant éventuellement substitué par un ou plusieurs substituants choisis indépendamment parmi W;
R⁴ et R⁵ sont choisis indépendamment dans le groupe formé par : atome d'hydrogène, alkyle en C₁₋₄, C(O)R¹⁹ et alcoxycarbonyle en C₂₋₄;
R⁶ est choisi dans le groupe formé par : atome d'hydrogène, alkyle en C₁₋₄, C(O)R¹⁹ et C(O)₂R¹⁹;
R⁷ est choisi dans le groupe formé par atome d'hydrogène, groupe alkyle en C₁₋₄, haloalkyle en C₁₋₄, alcényle en C₂₋₄, haloalcényle en C₂₋₄, SR¹⁶, S(O)R¹⁶, S(O)₂R¹⁶, C(O)R¹⁶, C(O)₂R¹⁶, C(O)NR¹⁶R²⁰, C(S)NR¹⁶R²⁰, C(S)R¹⁶, C(S)OR¹⁶, P(O)(OR¹⁶)₂, P(S)((OR¹⁶)₂, P(O)(R¹⁶)OR¹⁶, P(O)(R¹⁶)SR²⁰, phényle éventuellement substitué et benzyle éventuellement substitué dans lesquels les substituants éventuels des groupes phényle et benzyle sont choisis indépendamment parmi F, Cl, Br, CH₃, CF₃ ou OCF₃; à condition que lorsque R⁷ est autre que C(O)R¹⁶, C(O)NR¹⁶R²⁰ ou C(S)NR¹⁶R²⁰, R¹⁶ est alors autre qu'un atome d'hydrogène;
R⁸ est choisi dans le groupe formé par : atome d'hydrogène, alkyle en C_{1-4,} haloalkyle en C₁₋₄, alcényle en C₂₋₄, alcynyle en C₂₋₄, S(O)₂NR¹⁷R¹⁸, S(O)₂R¹⁶, C(O)R¹⁶, C(O)₂R¹⁶, C(O)NR¹⁶R²⁰, phényle éventuellement substitué par un atome d'halogène ou un groupe alcoxy en C₁₋₄, et benzyle éventuellement substitué par un atome d'halogène; à condition que lorsque R⁸ est S(O)₂R¹⁶, R¹⁶ soit alors autre qu'un atome d'hydrogène;
R⁹ est choisi dans le groupe formé par : atome d'hydrogène, groupe alkyle en C₁₋₄ et C(O)R¹⁶;
R¹⁰ est choisi dans le groupe formé par : atome d'hydrogène, groupe alkyle en C₁₋₄, haloalkyle en C₁₋₄ et phényle éventuellement substitué par un ou plusieurs substituants choisis indépendamment parmi un atome d'halogène, un groupe CN, NO₂, CF₃ et OCF₃; ou
R⁹ et R¹⁰ sont pris ensemble pour former -CH₂CH₂CH₂-, -CH₂CH₂CH₂CH₂- ou -CH₂CH₂CH₂CH₂CH₂-;
R¹¹ est un groupe alkyle en C₁₋₁₈;
R¹² est un groupe alkyle en C₁₋₄;
R¹³ et R14 sont indépendamment un groupe alkyle en C₁₋₄; ou
R¹³ et R¹⁴ sont pris ensemble pour former -CH₂CH₂CH₂CH₂CH₂- ou -CH₂CH₂OCH₂CH₂-;
R¹⁵ est choisi dans le groupe formé par les groupes : alkyle en C₁₋₆, haloalkyle en C₁₋₆, alcényle en C₂₋₆, haloalcényle en C₂₋₆, alcynyle en C₂₋₆, haloalcynyle en C₃₋₆, alcoxyalkyle en C₂₋₆, alkylthioalkyle en C₂₋₆, nitroalkyle en C₁₋₆, cyanoalkyle en C₂₋₆, alcoxycarbonylalkyle en C₃₋₈, cycloalkyle en C₃₋₆, halocycloalkyle en C₃₋₆, atome d'halogène, groupe CN, N₃, SCN, NO₂, OR¹⁶, SR¹⁶, S(O)R¹⁶, S(O)₂R¹⁶, OC(O)R¹⁶, OS(O)₂R¹⁶, C(O)R¹⁶, C(O)₂R¹⁶, C(O)NR¹⁶R¹⁷, S(O)₂NR¹⁶R¹⁷, NR¹⁶R¹⁷, NR¹⁷C(O)R¹⁶, OC(O)NHR¹⁶, NR¹⁷C(O)NHR¹⁶, NR¹⁷S(O)₂R¹⁶, phényle éventuellement substitué par 1 à 3 substituants choisis indépendamment parmi W et benzyle éventuellement substitué par 1 à 3 substituants choisis indépendamment parmi W; ou lorsque p est 2, (R¹⁵)₂ sont pris ensemble pour former -OCH₂O-, -OCF₂O-, -OCH₂CH₂O-, -CH₂C(CH₃)₂O-, -CF₂CF₂O- ou -OCF₂CF₂O-pour former un pont cyclique; à condition que lorsque R¹⁵ est S(O)R¹⁶, S(O)₂R¹⁶, OC(O)R¹⁶, NR¹⁷S(O)₂R¹⁶ ou OS(O)₂R¹⁶, R¹⁶ soit alors autre qu'un atome d'hydrogène;
R¹⁶ est choisi dans le groupe formé par : atome d'hydrogène, groupe alkyle en C₁₋₆, haloalkyle en C₁₋₆, alcényle en C₂₋₆, haloalcényle en C₂₋₆, alcynyle en C₂₋₆, haloalcynyle en C₃₋₆, alcoxyalkyle en C₂₋₆, alkylthioalkyle en C₂₋₆, nitroalkyle en C₁₋₆, cyanoalkyle en C₂₋₆, alcoxycarbonylalkyle en C₃₋₈, cycloalkyle en C₃₋₆, halocycloalkyle en C₃₋₆, et phényle éventuellement substitué et benzylc éventuellement substitué dans lesquels les substituants éventuels des groupes phényle et henzyle sont 1 à 3 susbtituants choisis indépendamment parmi W;
R¹⁷ est choisi dans le groupe formé par : atome d'hydrogène et groupe alkyle en C₁₋₄; ou
R¹⁶ et R¹⁷, lorsqu'ils sont fixés au même atome, sont pris ensemble pour former -CH₂CH₂CH₂CH₂-, -CH₂CH₂CH₂CH₂CH₂ ou -CH₂CH₂OCH₂CH₂-;
R¹⁸ est choisi dans le groupe formé par : atome d'hydrogène, groupe alkyle en C₁₋₄, alcényle en C₂₋₄, alcynyle en C₂₋₄, alkylcarbonyle en C₂₋₄, alcoxycarbonyle en C₂₋₄ et alkylsulfonyle en C₁₋₄;
R¹⁹ est un groupe alkyle en C₁₋₃,
R²⁰ est choisi dans le groupe formé par atome d'hydrogène, groupe alkyle en C₁₋₄, alcényle en C₂₋₄ et alcynyle en C₂₋₄;
R²¹ est choisi dans le groupe formé par atome d'hydrogène, groupe alkylcarbonyle en C₂₋₇, alcoxycarbonyle en C₂₋₇, alkyle en C₁₋₄ éventuellement substitué, alcényle en C₂₋₄ éventuellement substitué et alcynyle en C₂₋₄ éventuellement substitué, tous ces substituants éventuels étant choisis indépendamment parmi alcoxy en C₁₋₂, CN, C(O)R²⁸ ou C(O)₂R²⁵;
R²² est choisi dans le groupe formé par atome d'hydrogène, groupe alkyle en C₁₋₃, phényle éventuellement substitué par au moins un substituant choisi indépendamment dans W et benzyle éventuellement substitué par au moins un substituant choisi indépendamment dans W;
R²³ est choisi dans le groupe formé par : atome d'hydrogène, groupe alkyle en C₁₋₄, alcényle en C₂₋₄ et alcynyle en C₂₋₄;
R²⁴ est choisi dans le groupe formé par : atome d'hydrogène et groupe alkyle en C₁₋₃;
R²⁵ est choisi dans le groupe formé par : groupe alkyle en C₁₋₃ et phényle éventuellement substitué par au moins un substituant choisi indépendamment dans W;
R²⁶ est choisi dans le groupe formé par : groupe alkyle en C₁₋₄, haloalkyle en C₁₋₄, alcoxy en C₁₋₄ et haloalcoxy en C₁₋₄;
R²⁷ est un groupe alkyle en C₁₋₃;
R²⁸ est choisi dans le groupe formé par atome d'hydrogène, groupe alkylc cn C₁₋₃ et phényle éventuellement substitué par au moins un substituant choisi indépendamment dans W;
R²⁹ est choisi dans lc groupe formé par : atome d'hydrogène, groupe CHO, alkyle en C₁₋₄, alkylcarbonyle en C₂₋₄ et alcoxycarbonyle en C₂₋₄;
R³⁰ est choisi dans le groupe formé par : groupe alkyle en C₁₋₃ et haloalkyle en C₁₋₃;
R³¹ est choisi dans le groupe formé par : atome d'hydrogène, de chlore, groupe alkyle en C₁₋₄, alcoxy en C₁₋₄, alkylthio en C₁₋₂ et CN;
R³² est choisi dans le groupe consistant en atome d'hydrogène, groupe alkyle en C₁₋₄, alkylcarbonyle en C₂₋₃ et alcoxycarbonyle en C₂₋₃;
R³³ est choisi dans le groupe formé par : groupe alkylthio en C₁₋₄, haloalkylthio en C₁₋₄ et phényle éventuellement substitué par au moins un substituant choisi indépendamment dans W;
R³⁴ est choisi dans le groupe formé par atome d'hydrogène et groupe alkyle en C₁₋₂;
R³⁵ est choisi dans le groupe formé par les groupes CHO, alkyle en C₁₋₄ substitué par des substituants choisis indépendamment dans le groupe consistant en atome d'halogène, groupe alcoxy en C₁₋₂, haloalcoxy en C₁₋₂, CN, NO₂, alkylcarbonyle en C₂₋₄, alcoxycarbonyle en C₂₋₄ ou N(R³⁵)(R³⁶); ou R³⁵ est un groupe haloalkylcarbonyle en C₂₋₆; alcényle en C₃₋₆; haloalcényle en C₃₋₆; alcynyle en C₃₋₆; haloalcynyle en C₃₋₆; cycloalkyle en C₃₋₆, C(S)R²⁶; C(S)R³³; C(O)C(O)₂R²⁵; C(O)CH₂C(O)₂R²⁵; S(O)ᵣR³⁰; S(O)₂CH₂C(O)₂R²⁵; P(X)(OR²⁵)₂; C(O)N(R³⁶)(R³⁷); S(O)ᵣN(R¹³)R¹⁴; S(O)ᵣN(R¹²)C(O)OR¹¹; S(O)ᵣN(R¹²)CHO; J; CH₂J; C(O)J; C(O)Ph dans lequel le groupe phényle est éventuellement substitué par un substituant choisi indépendamment dans W et benzyle éventuellement substitué par un substituant choisi indépendamment dans W;
R³⁶ est choisi dans le groupe formé par atome d'hydrogène, groupe alkyle en C₁₋₄, alcényle en C₂₋₄, phényle éventuellement substitué par un substituant choisi indépendamment dans W et benzyle éventuellement substitué par un substituant choisi indépendamment dans W;
R³⁷ est choisi dans le groupe formé par : groupe alkyle en C₁₋₄ et alcényle en C₂₋₄;
R³⁸ est choisi dans le groupe formé par groupe C(S)R²⁶; C(S)R³³; C(O)C(O)₂R²⁵; C(O)CH₂C(O)₂R²⁵; S(O)R³⁰; S(O)₂R³⁰; S(O)₂CH₂C(O)₂R²⁵; P(X)(OR²⁵)₂; haloalcynyle en C₃₋₆; et alkyle en C₁₋₆ substitué par un groupe P(X)(OR²⁵)₂;
W est choisi dans le groupe formé d'atome d'halogène, groupe CN, NO₂, alkyle en C₁₋₂, haloalkyle en C₁₋₂, alcoxy en C₁₋₂, haloalcoxy en C₁₋₂, alkylthio en C₁₋₂, haloalkylthio en C₁₋₂, alkylsulfonyle en C₁₋₂ et haloalkylsulfonyle en C₁₋₂;
m est 1 à 3;
n est 1 à 3;
p est 1 à 3; et
r est 0, 1 ou 2.

2. Composé suivant la revendication 1, dans lequel :
J est choisi dans le groupe :

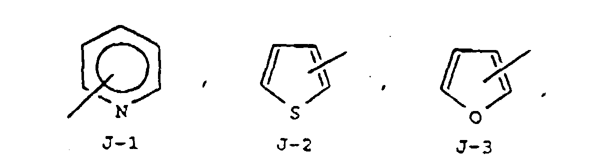

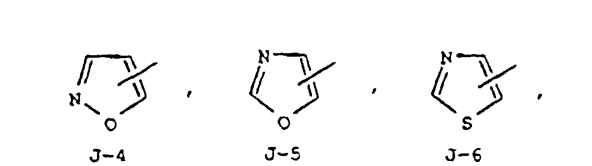

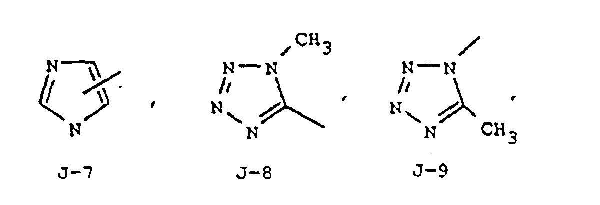

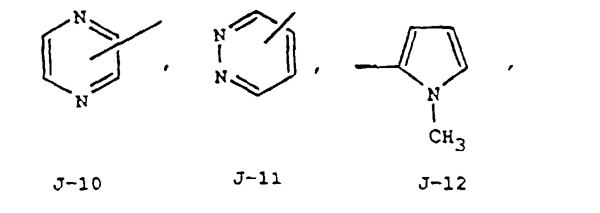

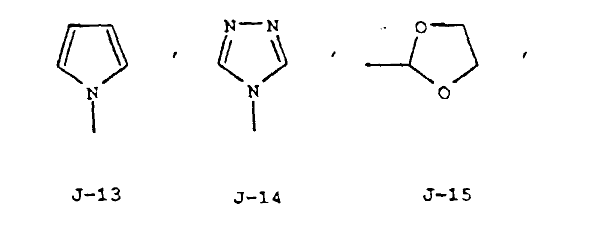

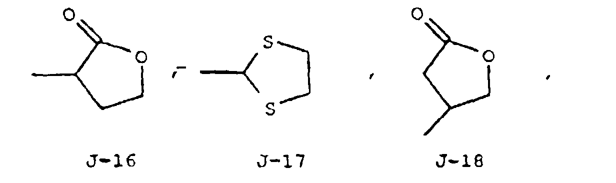

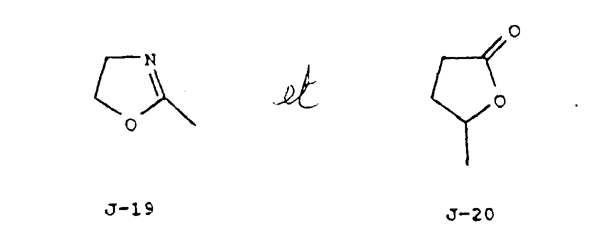

3. Composé suivant la revendication 1, dans lequel :
R¹ est choisi dans le groupe formé par : atome d'hydrogène, groupe alkyle en C₁₋₆, haloalkyle en C₁₋₆, alcényle en C₂₋₆, haloalcényle en C₂₋₆, alcynyle en C₂₋₆, haloalcynyle en C₃₋₆, alcoxyalkyle en C₂₋₆, alkylthioalkyle en C₂₋₆, nitroalkyle en C₁₋₆, cyanoalkyle en C₂₋₆, alcoxycarbonylalkyle en C₃₋₈, cycloalkyle en C₃₋₆, halocycloalkyle en C₃₋₆, atome d'halogène, groupe CN, SCN, NO₂, OR¹⁶, SR¹⁶, S(O)₂R¹⁶, C(O)R¹⁶, C(O)₂R¹⁶, phényle éventuellement substitué par 1 à 3 substituants choisis indépendamment parmi W et benzyle éventuellement substitué par 1 à 3 substituants choisis indépendamment parmi W; avec l'un des substituants R¹ en position 4, ou lorsque m est 2, les (R¹)₂ sont alors pris ensemble en donnant -CH₂C(CH₃)₂O-, -OCH₂CH₂O-, -OCF₂CF₂O- ou -CF₂CF₂O- pour former un noyau condensé de 5 à 6 chaînons;
R² est choisi dans le groupe formé par atome d'hydrogène, groupe alkyle en C₁₋₆, haloalkyle en C₁₋₆, alcényle en C₂₋₆, haloalcényle en C₂₋₆, atome d'halogène, groupe CN, SCN, NO₂, OR¹⁶, SR¹⁶, S(O)₂R¹⁶, OC(O)R¹⁶, OS(O)₂R¹⁶, C(O)R¹⁶, C(O)₂R¹⁶, C(O)NR¹⁶R¹⁷, S(O)₂NR¹⁶R¹⁷, NR¹⁶R¹⁷, phényle éventuellement substitué par 1 à 3 substituants choisis indépendamment parmi W et benzyle éventuellement substitué par 1 à 3 substituants choisis indépendamment parmi W;
R³ est choisi dans le groupe formé par : atome d'hydrogène, groupe alkyle en C₁₋₄, alcoxycarbonylalkyle en C₃₋₄, C(O)R¹⁶, C(O)₂R¹⁶, et phényle indépendamment substitué par un ou plusieurs substituants choisis parmi (R¹⁵)ₚ;
R¹⁵ est choisi dans le groupe consistant en groupe alkyle en C₁₋₆, haloalkyle en C₁₋₆, alcényle en C₂₋₆, haloalcényle en C₂₋₆, atome d'halogène, groupe CN, SCN, NO₂, OR¹⁶, SR¹⁶, S(O)₂R¹⁶, OC(O)R¹⁶, OS(O)₂R¹⁶, C(O)R¹⁶, C(O)₂R¹⁶, C(O)NR¹⁶R¹⁷, S(O)₂NR¹⁶R¹⁷, NR¹⁶R¹⁷, phényle éventuellement substitué par 1 à 3 substituants choisis indépendamment parmi W et benzyle éventuellement substitué par 1 à 3 substituants choisis indépendamment parmi W;
R¹⁶ est choisi dans le groupe formé par : groupe alkyle en C₁₋₄, haloalkyle en C₁₋₂, alcényle en C₃₋₄ et propargyle;
R¹⁷ est choisi dans le groupe consistant en atome d'hydrogène et groupe CH₃;
R³⁵ est choisi dans le groupe consistant en groupe CHO, alkyle en C₁₋₄ substitué par des substituants choisis indépendamment dans le groupe formé par atome d'halogène, groupe alcoxy en C₁₋₂, haloalcoxy en C₁₋₂, CN, NO₂, alkylcarbonyle en C₂₋₄, alcoxycarbonyle en C₂₋₄ et N(R³⁵)(R³⁶); ou R³⁵ est un groupe haloalkylcarbonyle en C₂₋₆; alcényle en C₃₋₆; haloalcényle en C₃₋₆; alcynyle en C₃₋₆; C(O)N(R³⁵)(R³⁶); R¹¹OC(O)N(R¹²)S-; (R¹³)(R¹⁴)NS-; C(O)Ph dans lequel le groupe phényle est éventuellement substitué par un groupe choisi indépendamment dans W et benzyle est éventuellement substitué par un groupe choisi indépendamment dans W;
R³⁸ est choisi dans le groupe formé par C(S)R²⁶; C(S)R³³; C(O)C(O)₂R²⁵; C(O)CH₂C(O)₂R²⁵; S(O)R³⁰; S(O)₂R³⁰; S(O)₂CH₂C(O)₂R²⁵; P(X)(OR²⁵)₂; haloalcynyle en C₃₋₆; et alkyle en C₁₋₆ substituré avec un groupe P(X)(OR²⁵)₂; et
m est 1 ou 2.

4. Composé suivant la revendication 3, dans lequel G est G-3, R⁴ est un atome d'hydrogène et R⁵ est un atome d'hydrogène.

5. Composé suivant la revendication 4, dans lequel Q est choisi dans le groupe formé par Q-1, Q-2, Q-7, Q-8 et Q-9.

6. Composé suivant la revendication 5, choisi dans le groupe :
3,4-bis-(4-chlorophényl)-4,5-dihydro-N-[4-(trifluorométhyl)-phényl]-1H-pyrrole-1-carboxamide,
2,3-dihydro-7-(trifluorométhyl)-2-[[[4-(trifluorométhyl)-phényl]-amino]-carbonyl]-[1]-benzopyrano-[3,4-c]-pyrrole-3a-(4H)-carboxylate de méthyle,
7-chloro-3,9-dihydro-3-[[[4-(trifluorométhoxy)-phényl]-amino]-carbonyl]-indéno-[1,2-e]-1,3-oxazine-9a-(2H)-carboxylate de méthyle,
7-chloro-4-formyl-2,3,4,5-tétrahydro-2-[[4-(trifluorométhoxy)-phénylaminocarbonyl]-4aH-indéno-[2,1-e]-1,2,4-triazine-4a-carboxylate de méthyle, et
4-formyl-2,3,4,5-tétrahydro-7-(2,2,2-trifluoroéthoxy)-2-[[4-(tri-fluorométhoxy)-phénylamino]-carbonyl]-4aH-indéno-[2,1-e]-1,2,4-triazine-4a-carboxylate de méthyle

7. Composition arthropodicide comprenant une quantité efficace du point de vue arthropodicide d'un composé suivant l'une quelconque des revendications 1 à 6 et un support pour celui-ci.

8. Procédé pour lutter contre les arthropodes comprenant la mise en contact des arthropodcs ou de leur environnement avec une quantité efficace du point de vue arthropodicide d'un composé suivant l'une quelconque des revendications 1 à 6.
